(19) **Europäisches Patentamt / European Patent Office / Office européen des brevets**

(11) **EP 4 624 493 A1**

(12) **EUROPEAN PATENT APPLICATION**
published in accordance with Art. 153(4) EPC

(43) Date of publication:
**01.10.2025 Bulletin 2025/40**

(21) Application number: **23894003.5**

(22) Date of filing: **24.11.2023**

(51) International Patent Classification (IPC):
**C07K 16/28** $^{(2006.01)}$   **A61K 39/395** $^{(2006.01)}$
**C12N 15/13** $^{(2006.01)}$

(52) Cooperative Patent Classification (CPC):
**A61K 39/395; C07K 16/00; C07K 16/28**

(86) International application number:
**PCT/CN2023/133895**

(87) International publication number:
**WO 2024/109914 (30.05.2024 Gazette 2024/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.11.2022  CN 202211485154**

(71) Applicants:
• **Jiangsu Hengrui Pharmaceuticals Co., Ltd.**
  **Lianyungang, Jiangsu 222047 (CN)**
• **Shanghai Hengrui Pharmaceutical Co., Ltd.**
  **Shanghai 200245 (CN)**

(72) Inventors:
• **LI, Xiaofei**
  **Shanghai 200245 (CN)**
• **TIAN, Chenmin**
  **Shanghai 200245 (CN)**

(74) Representative: **Regimbeau**
  **20, rue de Chazelles**
  **75847 Paris Cedex 17 (FR)**

Remarks:
The complete document including Reference Table(s) and the Sequence Listing(s) can be downloaded from the EPO website

(54) **PHARMACEUTICAL COMPOSITION COMPRISING BISPECIFIC ANTIBODY SPECIFICALLY BINDING TO HGFR AND EGFR**

(57)  The present disclosure relates to a pharmaceutical composition comprising a bispecific antibody specifically binding to HGFR and EGFR. Specifically, the present disclosure relates to a pharmaceutical composition comprising a bispecific antibody specifically binding to HGFR and EGFR, and a use thereof as a drug.

**EP 4 624 493 A1**

**Description**

**TECHNICAL FIELD**

The present disclosure pertains to the field of pharmaceutical formulations and particularly relates to a pharmaceutical composition comprising a bispecific antibody specifically binding to HGFR and EGFR and use thereof as a medicament.

**BACKGROUND**

The statements herein merely provide background information related to the present disclosure and may not necessarily constitute the prior art.

Epidermal growth factor receptor (EGFR) and hepatocyte growth factor receptor (HGFR) are two receptor tyrosine kinases and are both highly expressed on a variety of tumor cells, such as non-small cell lung cancer (NSCLC), colorectal cancer (CRC), and head and neck cancer (HNC).

The EGFR signaling pathway plays an important role in tumor biology by regulating cell proliferation, angiogenesis, and metastasis and survival of cancer cells. Dysregulation of this pathway can lead to tumorigenesis. HGFR is also associated with the development of many tumors due to abnormal signal activation caused by HGFR overexpression, activating mutations, autocrine or paracrine signal transduction, or gene amplification. Investigations into cancer treatment efficacy have revealed a significant connection between the EGFR and HGFR signaling pathways.

NSCLC accounts for 83% of all lung cancers, and EGFR-activating mutations are a common type (10-15% in Caucasians and 50% in Asians). EGFR tyrosine kinase inhibitors (TKIs) have long been the first-line therapy for NSCLC. Although the initial response rate is high (70-80%), resistance usually develops within a year. There are two major mechanisms of drug resistance. One is the occurrence of another EGFR mutation, such as EGFR T790M, and the other is the compensatory activation of the HGFR signaling pathway. These factors affect the effect of tumor treatment.

**SUMMARY**

The present disclosure provides a pharmaceutical composition comprising a bispecific antibody specifically binding to HGFR and EGFR, and the composition has therapeutic activity. In addition, the composition also has the advantages of good stability and the like.

In some embodiments, the present disclosure provides a pharmaceutical composition comprising a bispecific antibody specifically binding to HGFR and EGFR and a buffer, wherein:

the bispecific antibody specifically binding to HGFR and EGFR comprises at least one antigen-binding moiety 1 specifically binding to HGFR and at least one antigen-binding moiety 2 specifically binding to EGFR;
the buffer is a histidine buffer, an acetate buffer, a citrate buffer, a succinate buffer, or a phosphate buffer.

In some embodiments, the present disclosure provides a pharmaceutical composition comprising a bispecific antibody specifically binding to HGFR and EGFR and a buffer, wherein:

the bispecific antibody specifically binding to HGFR and EGFR comprises at least one antigen-binding moiety 1 specifically binding to HGFR and at least one antigen-binding moiety 2 specifically binding to EGFR;
the antigen-binding moiety 1 comprises a heavy chain variable region M-VH and a light chain variable region M-VL, wherein the M-VH comprises an M-HCDR1, an M-HCDR2, and an M-HCDR3, and the M-VL comprises an M-LCDR1, an M-LCDR2, and an M-LCDR3; and
the antigen-binding moiety 2 comprises a heavy chain variable region E-VH and a light chain variable region E-VL, wherein the E-VH comprises an E-HCDR1, an E-HCDR2, and an E-HCDR3, and the E-VL comprises an E-LCDR1, an E-LCDR2, and an E-LCDR3;
the E-HCDR1, the E-HCDR2, the E-HCDR3, the E-LCDR1, the E-LCDR2, and the E-LCDR3 are defined according to the Kabat numbering scheme, wherein the E-HCDR1 is set forth in SEQ ID NO: 6, the E-HCDR2 is set forth in SEQ ID NO: 7, the E-HCDR3 is set forth in SEQ ID NO: 8, the E-LCDR1 is set forth in SEQ ID NO: 9, the E-LCDR2 is set forth in SEQ ID NO: 10, and the E-LCDR3 is set forth in SEQ ID NO: 11; and
the M-HCDR1, the M-HCDR2, the M-HCDR3, the M-LCDR1, the M-LCDR2, and the M-LCDR3 are defined according to the Kabat numbering scheme, wherein

(i) the M-HCDR1 is set forth in SEQ ID NO: 30, the M-HCDR2 is set forth in SEQ ID NO: 31, the M-HCDR3 is set forth in SEQ ID NO: 32, the M-LCDR1 is set forth in SEQ ID NO: 27, the M-LCDR2 is set forth in SEQ ID NO: 33, and the M-LCDR3 is set forth in SEQ ID NO: 29; or

(ii) the M-HCDR1 is set forth in SEQ ID NO: 18, the M-HCDR2 is set forth in SEQ ID NO: 19, the M-HCDR3 is set forth in SEQ ID NO: 20, the M-LCDR1 is set forth in SEQ ID NO: 21, the M-LCDR2 is set forth in SEQ ID NO: 22, and the M-LCDR3 is set forth in SEQ ID NO: 23; or

(iii) the M-HCDR1 is set forth in SEQ ID NO: 24, the M-HCDR2 is set forth in SEQ ID NO: 25, the M-HCDR3 is set forth in SEQ ID NO: 26, the M-LCDR1 is set forth in SEQ ID NO: 27, the M-LCDR2 is set forth in SEQ ID NO: 28, and the M-LCDR3 is set forth in SEQ ID NO: 29;

the buffer is a histidine buffer, an acetate buffer, a citrate buffer, a succinate buffer, or a phosphate buffer.

In some embodiments, the buffer is a histidine buffer.

In some embodiments, the buffer is a histidine-histidine hydrochloride buffer or a histidine-histidine acetate buffer.

In some specific embodiments, the buffer is a histidine-histidine hydrochloride buffer.

In some embodiments, provided is the pharmaceutical composition according to any one of the above, wherein the pharmaceutical composition has a pH of 4.5 to 6.5. In some embodiments, provided is the pharmaceutical composition according to any one of the above, wherein the pharmaceutical composition has a pH of 4.8 to 5.2. In some embodiments, the pharmaceutical composition has a pH of 5.0 to 6.0.

In some embodiments, the pharmaceutical composition has a pH of 5.0 to 6.5. In some embodiments, the pharmaceutical composition has a pH of 5.5 to 6.5. In some embodiments, the pharmaceutical composition has a pH of 5.5 to 6.0. In some embodiments, the pharmaceutical composition has a pH of 6.0 to 6.5. In some embodiments, provided is the pharmaceutical composition according to any one of the above, wherein the pharmaceutical composition has a pH of 5.8 to 6.2. In some embodiments, the pharmaceutical composition has a pH of about 5.8. In some embodiments, the pharmaceutical composition has a pH of about 6.0. In some embodiments, the pharmaceutical composition has a pH of about 6.2.

In some embodiments, provided is the pharmaceutical composition according to any one of the above, wherein the pharmaceutical composition has a pH of 4.5 to 6.0. In some embodiments, provided is the pharmaceutical composition according to any one of the above, wherein the pharmaceutical composition has a pH of 4.5 to 5.5. In some embodiments, provided is the pharmaceutical composition according to any one of the above, wherein the pharmaceutical composition has a pH of 4.8 to 5.2. In some embodiments, the pharmaceutical composition has a pH of about 4.8. In some embodiments, the pharmaceutical composition has a pH of about 5.0. In some embodiments, the pharmaceutical composition has a pH of about 5.2.

When a point value is referred to in the present disclosure, it should be understood that the point value includes a margin of error. This margin of error is due to factors such as laboratory environment, personnel operations, instruments, methodology, and measurement errors. Taking the pH as an example, when the measurement is about 6.0, it should be understood that it includes a margin of error. As an example, when a formulation is measured using an industrial pH meter, "about 6.0" represents $6.0 \pm 0.2$ (i.e., a pH of 5.8 to 6.2).

In some embodiments, the pharmaceutical composition has a pH of 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, or 6.5, or any range between these point values. In some embodiments, the pharmaceutical composition has a pH of 4.8. In some embodiments, the pharmaceutical composition has a pH of 5.0. In some embodiments, the pharmaceutical composition has a pH of 5.2. In some embodiments, the pharmaceutical composition has a pH of 5.8. In some embodiments, the pharmaceutical composition has a pH of 6.0. In some embodiments, the pharmaceutical composition has a pH of 6.2.

Generally, the pH of a pharmaceutical composition obtained by buffer exchange is almost consistent with the pH of the buffer. In addition, it is well known to those skilled in the art that in the process of pharmaceutical formulation, there may sometimes be a pH drift, but the pH drift of the pharmaceutical formulation is generally small (e.g., within a range of $\pm 0.3$). In some embodiments, the pH drift of the pharmaceutical formulation is within a range of $\pm 0.3$. In some embodiments, the pH drift of the pharmaceutical formulation is within a range of $\pm 0.2$. In some embodiments, the pH drift of the pharmaceutical formulation is within a range of $\pm 0.1$.

In some embodiments, provided is the pharmaceutical composition according to any one of the above, wherein the bispecific antibody specifically binding to HGFR and EGFR is at a concentration of 1 mg/mL to 250 mg/mL. In some embodiments, the bispecific antibody specifically binding to HGFR and EGFR is at a concentration of 1 mg/mL to 200 mg/mL. In some embodiments, the bispecific antibody specifically binding to HGFR and EGFR is at a concentration of 50 mg/mL to 200 mg/mL. In some embodiments, the bispecific antibody specifically binding to HGFR and EGFR is at a concentration of 80 mg/mL to 180 mg/mL.

In some embodiments, the bispecific antibody specifically binding to HGFR and EGFR is at a concentration of 1 mg/mL to 150 mg/mL. In some embodiments, the bispecific antibody specifically binding to HGFR and EGFR is at a concentration of 1 mg/mL to 120 mg/mL. In some embodiments, the bispecific antibody specifically binding to HGFR and EGFR is at a concentration of 1 mg/mL to 100 mg/mL. In some embodiments, the bispecific antibody specifically binding to HGFR and EGFR is at a concentration of 50 mg/mL to 150 mg/mL. In some embodiments, the bispecific antibody specifically binding to HGFR and EGFR is at a concentration of 80 mg/mL to 120 mg/mL. In some embodiments, the bispecific antibody specifically binding to HGFR and EGFR is at a concentration of 90 mg/mL to 110 mg/mL. In some embodiments, the bispecific antibody specifically binding to HGFR and EGFR is at a concentration of about 100 mg/mL.

In some embodiments, the bispecific antibody specifically binding to HGFR and EGFR is at a concentration of 80 mg/mL to 200 mg/mL. In some embodiments, the bispecific antibody specifically binding to HGFR and EGFR is at a concentration of 90 mg/mL to 200 mg/mL. In some embodiments, the bispecific antibody specifically binding to HGFR and EGFR is at a concentration of 100 mg/mL to 200 mg/mL. In some embodiments, the bispecific antibody specifically binding to HGFR and EGFR is at a concentration of 120 mg/mL to 200 mg/mL. In some embodiments, the bispecific antibody specifically binding to HGFR and EGFR is at a concentration of 120 mg/mL to 180 mg/mL. In some embodiments, the bispecific antibody specifically binding to HGFR and EGFR is at a concentration of 135 mg/mL to 165 mg/mL. In some embodiments, the bispecific antibody specifically binding to HGFR and EGFR is at a concentration of about 150 mg/mL.

In some embodiments, the bispecific antibody specifically binding to HGFR and EGFR is at a concentration of 50 mg/mL to 100 mg/mL. In some embodiments, the bispecific antibody specifically binding to HGFR and EGFR is at a concentration of 50 mg/mL to 75 mg/mL. In some embodiments, the bispecific antibody specifically binding to HGFR and EGFR is at a concentration of 48 mg/mL to 72 mg/mL. In some embodiments, the bispecific antibody specifically binding to HGFR and EGFR is at a concentration of 54 mg/mL to 66 mg/mL. In some embodiments, the bispecific antibody specifically binding to HGFR and EGFR is at a concentration of about 60 mg/mL. In some embodiments, the bispecific antibody specifically binding to HGFR and EGFR is at a concentration of about 70 mg/mL.

In some embodiments, the bispecific antibody specifically binding to HGFR and EGFR is at a concentration of 50 mg/mL to 250 mg/mL. In some embodiments, the bispecific antibody specifically binding to HGFR and EGFR is at a concentration of 100 mg/mL to 250 mg/mL. In some embodiments, the bispecific antibody specifically binding to HGFR and EGFR is at a concentration of 160 mg/mL to 240 mg/mL. In some embodiments, the bispecific antibody specifically binding to HGFR and EGFR is at a concentration of 180 mg/mL to 220 mg/mL. In some embodiments, the bispecific antibody specifically binding to HGFR and EGFR is at a concentration of about 200 mg/mL.

In some embodiments, the bispecific antibody specifically binding to HGFR and EGFR is at a concentration of 1 mg/mL, 5 mg/mL, 10 mg/mL, 20 mg/mL, 30 mg/mL, 40 mg/mL, 45 mg/mL, 48 mg/mL, 50 mg/mL, 54 mg/mL, 55 mg/mL, 60 mg/mL, 65 mg/mL, 66 mg/mL, 70 mg/mL, 72 mg/mL, 75 mg/mL, 80 mg/mL, 85 mg/mL, 90 mg/mL, 95 mg/mL, 100 mg/mL, 105 mg/mL, 110 mg/mL, 120 mg/mL, 130 mg/mL, 135 mg/mL, 140 mg/mL, 150 mg/mL, 160 mg/mL, 165 mg/mL, 180 mg/mL, 200 mg/mL, 220 mg/mL, 240 mg/mL, or 250 mg/mL, or any range between these point values. In some embodiments, the bispecific antibody specifically binding to HGFR and EGFR is at a concentration of 60 mg/mL. In some embodiments, the bispecific antibody specifically binding to HGFR and EGFR is at a concentration of 70 mg/mL. In some embodiments, the bispecific antibody specifically binding to HGFR and EGFR is at a concentration of 100 mg/mL. In some embodiments, the bispecific antibody specifically binding to HGFR and EGFR is at a concentration of 150 mg/mL. In some embodiments, the bispecific antibody specifically binding to HGFR and EGFR is at a concentration of 200 mg/mL.

In some embodiments, provided is the pharmaceutical composition according to any one of the above, wherein the pharmaceutical composition comprises a surfactant. In some embodiments, the surfactant is a nonionic surfactant. In some embodiments, the surfactant is selected from the group consisting of a poloxamer (e.g., poloxamer 188, abbreviated as P188), a polysorbate (e.g., polysorbate 20 or polysorbate 80), poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramidopropyl-betaine, cocamidopropyl-betaine, linoleamidopropyl-betaine, myristamidopropyl-betaine, palmitamidopropyl-betaine, isostearamidopropyl-betaine, myristamidopropyl-dimethylamine, palmitamidopropyl-dimethylamine, isostearamidopropyl-dimethylamine, sodium methyl cocoyl, sodium methyl oleyl taurate, polyethylene glycol, polypropylene glycol, copolymers of ethylene and propylene glycol, etc. In some embodiments, the surfactant is a polysorbate or poloxamer. In some embodiments, the surfactant is polysorbate 80, polysorbate 20, or poloxamer 188. In some embodiments, the surfactant is polysorbate 80.

In some embodiments, provided is the pharmaceutical composition according to any one of the above, wherein the surfactant is at a concentration of 0.01 mg/mL to 1.0 mg/mL. In some embodiments, the surfactant is at a concentration of 0.1 mg/mL to 0.8 mg/mL. In some embodiments, the surfactant is at a concentration of 0.2 mg/mL to 0.8 mg/mL. In some embodiments, the surfactant is at a concentration of 0.3 mg/mL to 0.8 mg/mL. In some embodiments, the surfactant is at a concentration of 0.4 mg/mL to 0.8 mg/mL. In some embodiments, the surfactant is at a concentration of 0.48 mg/mL to 0.72 mg/mL. In some embodiments, the surfactant is at a concentration of 0.54 mg/mL to 0.66 mg/mL. In some embodiments, the surfactant is at a concentration of about 0.6 mg/mL.

In some embodiments, the surfactant is at a concentration of 0.3 mg/mL to 0.6 mg/mL. In some embodiments, the surfactant is at a concentration of 0.4 mg/mL to 0.6 mg/mL. In some embodiments, the surfactant is at a concentration of 0.6 mg/mL to 0.8 mg/mL. In some embodiments, the surfactant is at a concentration of 0.2 mg/mL to 0.4 mg/mL. In some embodiments, the surfactant is at a concentration of 0.2 mg/mL to 0.3 mg/mL. In some embodiments, the surfactant is at a concentration of 0.24 mg/mL to 0.36 mg/mL. In some embodiments, the surfactant is at a concentration of about 0.24 mg/mL. In some embodiments, the surfactant is at a concentration of about 0.3 mg/mL.

In some embodiments, the surfactant is at a concentration of 0.01 mg/mL, 0.05 mg/mL, 0.1 mg/mL, 0.15 mg/mL, 0.2 mg/mL, 0.24 mg/mL, 0.3 mg/mL, 0.36 mg/mL, 0.4 mg/mL, 0.5 mg/mL, 0.6 mg/mL, 0.7 mg/mL, 0.8 mg/mL, 0.9 mg/mL, or 1.0 mg/mL, or any range between these point values. In some embodiments, the surfactant is at a concentration of 0.6 mg/mL. In some embodiments, the surfactant is at a concentration of 0.24 mg/mL. In some embodiments, the surfactant is at a concentration of 0.3 mg/mL.

In some embodiments, the surfactant is 0.6 mg/mL polysorbate 80. In some embodiments, the surfactant is polysorbate 80 at a concentration of 0.24 mg/mL. In some embodiments, the surfactant is polysorbate 80 at a concentration of 0.3 mg/mL.

In some embodiments, provided is the pharmaceutical composition according to any one of the above, which comprises a sugar. In some embodiments, the sugar is selected from the group consisting of conventional compositions $(CH_2O)_n$ and derivatives thereof, including monosaccharides, disaccharides, trisaccharides, polysaccharides, sugar alcohols, reducing sugars, non-reducing sugars, and the like. The sugar may be selected from the group consisting of sucrose, trehalose, glucose, lactose, fructose, maltose, dextran, glycerin, erythritol, glycerol, arabitol, sylitol, sorbitol, mannitol, melibiose, melezitose, raffinose, mannotriose, stachyose, maltose, lactulose, maltulose, glucitol, maltitol, lactitol, iso-maltulose, and the like.

In some embodiments, the sugar is sucrose, trehalose, mannitol, or sorbitol. In some embodiments, the sugar is sucrose or trehalose. In some embodiments, the sugar is sucrose.

In some embodiments, the sugar is at a concentration of 10 mg/mL to 100 mg/mL. In some embodiments, the sugar is at a concentration of 20 mg/mL to 90 mg/mL. In some embodiments, the sugar is at a concentration of 20 mg/mL to 80 mg/mL. In some embodiments, the sugar is at a concentration of 30 mg/mL to 80 mg/mL. In some embodiments, the sugar is at a concentration of 50 mg/mL to 80 mg/mL. In some embodiments, the sugar is at a concentration of 60 mg/mL to 90 mg/mL. In some embodiments, the sugar is at a concentration of 60 mg/mL to 80 mg/mL. In some embodiments, the sugar is at a concentration of 67.5 mg/mL to 82.5 mg/mL. In some embodiments, the sugar is at a concentration of 70 mg/mL to 80 mg/mL. In some embodiments, the sugar is at a concentration of about 75 mg/mL.

In some embodiments, the sugar is at a concentration of 56 mg/mL to 84 mg/mL. In some embodiments, the sugar is at a concentration of 63 mg/mL to 77 mg/mL. In some embodiments, the sugar is at a concentration of about 70 mg/mL.

In some embodiments, the sugar is at a concentration of 20 mg/mL to 50 mg/mL. In some embodiments, the sugar is at a concentration of 20 mg/mL to 40 mg/mL. In some embodiments, the sugar is at a concentration of 20 mg/mL to 35 mg/mL. In some embodiments, the sugar is at a concentration of 22.4 mg/mL to 33.6 mg/mL. In some embodiments, the sugar is at a concentration of 25.2 mg/mL to 30.8 mg/mL. In some embodiments, the sugar is at a concentration of about 28 mg/mL.

In some embodiments, the sugar is at a concentration of 30 mg/mL to 50 mg/mL. In some embodiments, the sugar is at a concentration of 30 mg/mL to 40 mg/mL. In some embodiments, the sugar is at a concentration of about 37.5 mg/mL.

In some embodiments, non-limiting examples of the concentration of the sugar include 10 mg/mL, 20 mg/mL, 22.4 mg/mL, 25.2 mg/mL, 28 mg/mL, 30 mg/mL, 30.8 mg/mL, 33.6 mg/mL, 35 mg/mL, 37.5 mg/mL, 40 mg/mL, 45 mg/mL, 50 mg/mL, 56 mg/mL, 55 mg/mL, 60 mg/mL, 63 mg/mL, 65 mg/mL, 67.5 mg/mL, 70 mg/mL, 75 mg/mL, 77 mg/mL, 80 mg/mL, 82.5 mg/mL, 84 mg/mL, 85 mg/mL, 90 mg/mL, 95 mg/mL, and 100 mg/mL, and any range between these point values. In some embodiments, the sugar is at a concentration of 28 mg/mL. In some embodiments, the sugar is at a concentration of 37.5 mg/mL. In some embodiments, the sugar is at a concentration of 70 mg/mL. In some embodiments, the sugar is at a concentration of 75 mg/mL.

In some embodiments, the sugar is 70 mg/mL to 80 mg/mL sucrose; preferably, the sugar is 75 mg/mL sucrose.

In some embodiments, the sugar is 63 mg/mL to 77 mg/mL sucrose; preferably, the sugar is 70 mg/mL sucrose.

In some embodiments, the sugar is 25.2 mg/mL to 30.8 mg/mL sucrose; preferably, the sugar is 28 mg/mL sucrose.

In some embodiments, provided is the pharmaceutical composition according to any one of the above, wherein the buffer is at a concentration of 5 mM to 100 mM. In some embodiments, the buffer is at a concentration of 5 mM to 50 mM. In some embodiments, the buffer is at a concentration of 10 mM to 50 mM. In some embodiments, the buffer is at a concentration of 5 mM to 30 mM. In some embodiments, the buffer is at a concentration of 10 mM to 30 mM. In some embodiments, the buffer is at a concentration of 15 mM to 25 mM. In some embodiments, the buffer is at a concentration of 18 mM to 22 mM. In some embodiments, the buffer is at a concentration of 10 mM to 20 mM. In some embodiments, the buffer is at a concentration of 10 mM or 20 mM. In some embodiments, the buffer is at a concentration of about 20 mM.

In some embodiments, the buffer is at a concentration of 20 mM to 30 mM. In some embodiments, the buffer is at a concentration of 22.5 mM to 27.5 mM. In some embodiments, the buffer is at a concentration of about 25 mM.

In some embodiments, the buffer is at a concentration of 5 mM to 15 mM. In some embodiments, the buffer is at a concentration of 8 mM to 12 mM. In some embodiments, the buffer is at a concentration of 9 mM to 11 mM. In some embodiments, the buffer is at a concentration of about 10 mM.

In some embodiments, the buffer is at a concentration of 5 mM, 8 mM, 9 mM, 10 mM, 11 mM, 12 mM, 15 mM, 18 mM, 20 mM, 22 mM, 22.5 mM, 25 mM, 27.5 mM, 30 mM, 40 mM, 50 mM, 60 mM, 70 mM, 80 mM, 90 mM, or 100 mM, and any range between these point values. In some embodiments, the buffer is at a concentration of 10 mM. In some embodiments, the buffer is at a concentration of 20 mM. In some embodiments, the buffer is at a concentration of 25 mM.

In some embodiments, the buffer is 10 mM or 20 mM histidine-histidine hydrochloride buffer. In some embodiments, the buffer is 10 mM or 20 mM histidine-histidine acetate buffer. In some embodiments, the buffer is 25 mM histidine-histidine hydrochloride buffer.

In some embodiments, provided is the pharmaceutical composition according to any one of the above, wherein the pharmaceutical composition further comprises an auxiliary material. In some embodiments, the auxiliary material is methionine, arginine hydrochloride, glycine, proline, histidine, phenylalanine, glutamic acid, aspartic acid, sodium chloride, calcium chloride, or disodium edetate. In some embodiments, the auxiliary material is sodium chloride or calcium chloride. In some embodiments, the auxiliary material is methionine or arginine hydrochloride. In some embodiments, the auxiliary material is arginine hydrochloride. In some embodiments, the auxiliary material is methionine.

In some embodiments, provided is the pharmaceutical composition according to any one of the above, wherein the auxiliary material is at a concentration of 1 mM to 300 mM; preferably, the auxiliary material is at a concentration of 25 mM to 200 mM. In some embodiments, the auxiliary material is at a concentration of 1 mM to 200 mM. In some embodiments, the auxiliary material is at a concentration of 1 mM to 20 mM. In some embodiments, the auxiliary material is at a concentration of 4 mM to 20 mM. In some embodiments, the auxiliary material is at a concentration of 8 mM to 12 mM. In some embodiments, the auxiliary material is at a concentration of 9 mM to 11 mM. In some embodiments, the auxiliary material is at a concentration of about 10 mM.

In some embodiments, the auxiliary material is at a concentration of 1 mM to 10 mM. In some embodiments, the auxiliary material is at a concentration of 3 mM to 5 mM. In some embodiments, the auxiliary material is at a concentration of 3.6 mM to 4.4 mM. In some embodiments, the auxiliary material is at a concentration of about 4 mM.

In some embodiments, the auxiliary material is at a concentration of 1 mM to 240 mM. In some embodiments, the auxiliary material is at a concentration of 80 mM to 240 mM. In some embodiments, the auxiliary material is at a concentration of 160 mM to 240 mM. In some embodiments, the auxiliary material is at a concentration of 180 mM to 220 mM. In some embodiments, the auxiliary material is at a concentration of about 200 mM.

In some embodiments, the auxiliary material is at a concentration of 80 mM to 120 mM. In some embodiments, the auxiliary material is at a concentration of 90 mM to 110 mM. In some embodiments, the auxiliary material is at a concentration of about 100 mM.

In some embodiments, the auxiliary material is at a concentration of 1 mM, 3 mM, 3.6 mM, 4.4 mM, 5 mM, 8 mM, 9 mM, 10 mM, 11 mM, 12 mM, 20 mM, 25 mM, 30 mM, 50 mM, 75 mM, 80 mM, 90 mM, 100 mM, 110 mM, 120 mM, 150 mM, 160 mM, 180 mM, 200 mM, 240 mM, 250 mM, 280 mM, or 300 mM, and any range between these point values. In some embodiments, the auxiliary material is at a concentration of 4 mM. In some embodiments, the auxiliary material is at a concentration of 10 mM. In some embodiments, provided is the pharmaceutical composition according to any one of the above, wherein the auxiliary material is at a concentration of 100 mM. In some embodiments, provided is the pharmaceutical composition according to any one of the above, wherein the auxiliary material is at a concentration of 200 mM.

In some embodiments, provided is the pharmaceutical composition according to any one of the above, wherein the auxiliary material is methionine at a concentration of 4 mM or 10 mM.

In some embodiments, provided is the pharmaceutical composition according to any one of the above, wherein the auxiliary material is arginine hydrochloride at a concentration of 100 mM or 200 mM.

In some embodiments, provided is the pharmaceutical composition according to any one of the above, wherein the pharmaceutical composition further comprises an enzyme. In some embodiments, the enzyme is hyaluronidase (PH20).

In some embodiments, provided is the pharmaceutical composition according to any one of the above, wherein the enzyme is at a concentration of 10 U/mL to 3000 U/mL. In some embodiments, provided is the pharmaceutical composition according to any one of the above, wherein the enzyme is at a concentration of 150 U/mL to 2400 U/mL. In some embodiments, provided is the pharmaceutical composition according to any one of the above, wherein the enzyme is at a concentration of 150 U/mL to 2200 U/mL. In some embodiments, provided is the pharmaceutical composition according to any one of the above, wherein the enzyme is at a concentration of 600 U/mL to 2200 U/mL. In some embodiments, provided is the pharmaceutical composition according to any one of the above, wherein the enzyme is at a concentration of 800 U/mL to 2000 U/mL. In some embodiments, provided is the pharmaceutical composition according to any one of the above, wherein the enzyme is at a concentration of 700 U/mL to 2400 U/mL. In some embodiments, provided is the pharmaceutical composition according to any one of the above, wherein the enzyme is at a concentration of 800 U/mL to 2400 U/mL. In some embodiments, provided is the pharmaceutical composition according to any one of the above, wherein the enzyme is at a concentration of 1600 U/mL to 2400 U/mL. In some embodiments, provided is the pharmaceutical composition according to any one of the above, wherein the enzyme is at a concentration of 1800 U/mL to 2200 U/mL. In some embodiments, provided is the pharmaceutical composition according to any one of the above, wherein the enzyme is at a concentration of about 2000 U/mL.

In some embodiments, provided is the pharmaceutical composition according to any one of the above, wherein the enzyme is at a concentration of 150 U/mL to 2000 U/mL. In some embodiments, provided is the pharmaceutical composition according to any one of the above, wherein the enzyme is at a concentration of 500 U/mL to 1100 U/mL. In some embodiments, provided is the pharmaceutical composition according to any one of the above, wherein the enzyme is at a concentration of 600 U/mL to 1000 U/mL. In some embodiments, provided is the pharmaceutical composition according to any one of the above, wherein the enzyme is at a concentration of 640 U/mL to 960 U/mL. In some embodiments, provided is the pharmaceutical composition according to any one of the above, wherein the enzyme is at a concentration of 700 U/mL to 900 U/mL. In some embodiments, provided is the pharmaceutical composition according to any one of the above, wherein the enzyme is at a concentration of 720 U/mL to 880 U/mL. In some embodiments, provided is the pharmaceutical composition according to any one of the above, wherein the enzyme is at a concentration of about 800 U/mL.

In some embodiments, the enzyme is at a concentration of 10 U/mL, 50 U/mL, 100 U/mL, 150 U/mL, 300 U/mL, 500 U/mL, 600 U/mL, 640 U/mL, 700 U/mL, 720 U/mL, 800 U/mL, 880 U/mL, 900 U/mL, 960 U/mL, 1000 U/mL, 1100 U/mL, 1200 U/mL, 1500 U/mL, 1600 U/mL, 1800 U/mL, 2000 U/mL, 2200 U/mL, 2400 U/mL, 2500 U/mL, 2800 U/mL, or 3000 U/mL, and any range between these point values. In some embodiments, provided is the pharmaceutical composition according to any one of the above, wherein the enzyme is at a concentration of 800 U/mL. In some embodiments, provided is the pharmaceutical composition according to any one of the above, wherein the enzyme is at a concentration of 1000 U/mL. In some embodiments, provided is the pharmaceutical composition according to any one of the above, wherein the enzyme is at a concentration of 2000 U/mL.

In some embodiments, provided is the pharmaceutical composition according to any one of the above, wherein the enzyme is 1000 U/mL or 2000 U/mL PH20. In some embodiments, provided is the pharmaceutical composition according to any one of the above, wherein the enzyme is 800 U/mL PH20.

In some embodiments, provided is the pharmaceutical composition according to any one of the above, wherein in the bispecific antibody specifically binding to HGFR and EGFR,
the antigen-binding moiety 1 comprises a heavy chain variable region M-VH and a light chain variable region M-VL, wherein the M-VH comprises an M-HCDR1, an M-HCDR2, and an M-HCDR3, and the M-VL comprises an M-LCDR1, an M-LCDR2, and an M-LCDR3, wherein:

the E-HCDR1, the E-HCDR2, the E-HCDR3, the E-LCDR1, the E-LCDR2, and the E-LCDR3 are defined according to the Kabat numbering scheme, wherein the E-HCDR1 is set forth in SEQ ID NO: 6, the E-HCDR2 is set forth in SEQ ID NO: 7, the E-HCDR3 is set forth in SEQ ID NO: 8, the E-LCDR1 is set forth in SEQ ID NO: 9, the E-LCDR2 is set forth in SEQ ID NO: 10, and the E-LCDR3 is set forth in SEQ ID NO: 11; and
the M-HCDR1, the M-HCDR2, the M-HCDR3, the M-LCDR1, the M-LCDR2, and the M-LCDR3 are defined according to the Kabat numbering scheme, wherein

(i) the M-HCDR1 is set forth in SEQ ID NO: 30, the M-HCDR2 is set forth in SEQ ID NO: 31, the M-HCDR3 is set forth in SEQ ID NO: 32, the M-LCDR1 is set forth in SEQ ID NO: 27, the M-LCDR2 is set forth in SEQ ID NO: 33, and the M-LCDR3 is set forth in SEQ ID NO: 29; or
(ii) the M-HCDR1 is set forth in SEQ ID NO: 18, the M-HCDR2 is set forth in SEQ ID NO: 19, the M-HCDR3 is set forth in SEQ ID NO: 20, the M-LCDR1 is set forth in SEQ ID NO: 21, the M-LCDR2 is set forth in SEQ ID NO: 22, and the M-LCDR3 is set forth in SEQ ID NO: 23; or
(iii) the M-HCDR1 is set forth in SEQ ID NO: 24, the M-HCDR2 is set forth in SEQ ID NO: 25, the M-HCDR3 is set forth in SEQ ID NO: 26, the M-LCDR1 is set forth in SEQ ID NO: 27, the M-LCDR2 is set forth in SEQ ID NO: 28, and the M-LCDR3 is set forth in SEQ ID NO: 29;

preferably,

(i) the bispecific antibody specifically binding to HGFR and EGFR comprises two antigen-binding moieties 1 specifically binding to HGFR and one antigen-binding moiety 2 specifically binding to EGFR, wherein

one of the antigen-binding moieties 1 comprises an M-VH and an M-VL, wherein the M-VH comprises: an M-HCDR1 set forth in SEQ ID NO: 30, an M-HCDR2 set forth in SEQ ID NO: 31, and an M-HCDR3 set forth in SEQ ID NO: 32, and the M-VL comprises: an M-LCDR1 set forth in SEQ ID NO: 27, an M-LCDR2 set forth in SEQ ID NO: 33, and an M-LCDR3 set forth in SEQ ID NO: 29;
the other antigen-binding moiety 1 comprises an M-VH and an M-VL, wherein the M-VH comprises: an M-HCDR1 set forth in SEQ ID NO: 18, an M-HCDR2 set forth in SEQ ID NO: 19, and an M-HCDR3 set forth in SEQ ID NO: 20, and the M-VL comprises: an M-LCDR1 set forth in SEQ ID NO: 21, an M-LCDR2 set forth in SEQ ID NO: 22, and an M-LCDR3 set forth in SEQ ID NO: 23; and
the antigen-binding moiety 2 comprises an E-VH and an E-VL, wherein the E-VH comprises: an E-HCDR1 set forth in SEQ ID NO: 6, an E-HCDR2 set forth in SEQ ID NO: 7, and an E-HCDR3 set forth in SEQ ID NO: 8, and the E-VL comprises: an E-LCDR1 set forth in SEQ ID NO: 9, an E-LCDR2 set forth in SEQ ID NO: 10, and an E-LCDR3 set forth in SEQ ID NO: 11; or

(ii) the bispecific antibody specifically binding to HGFR and EGFR comprises one antigen-binding moiety 1 specifically binding to HGFR and one antigen-binding moiety 2 specifically binding to EGFR, wherein

the antigen-binding moiety 1 comprises an M-VH and an M-VL, wherein the M-VH comprises: an M-HCDR1 set forth in SEQ ID NO: 18, an M-HCDR2 set forth in SEQ ID NO: 19, and an M-HCDR3 set forth in SEQ ID NO: 20, and the M-VL comprises: an M-LCDR1 set forth in SEQ ID NO: 21, an M-LCDR2 set forth in SEQ ID NO: 22, and an M-LCDR3 set forth in SEQ ID NO: 23; and
the antigen-binding moiety 2 comprises an E-VH and an E-VL, wherein the E-VH comprises: an E-HCDR1 set forth in SEQ ID NO: 6, an E-HCDR2 set forth in SEQ ID NO: 7, and an E-HCDR3 set forth in SEQ ID NO: 8, and the E-VL comprises: an E-LCDR1 set forth in SEQ ID NO: 9, an E-LCDR2 set forth in SEQ ID NO: 10, and an E-LCDR3 set forth in SEQ ID NO: 11.

In some embodiments, provided is the pharmaceutical composition according to any one of the above, wherein in the bispecific antibody specifically binding to HGFR and EGFR,

(i) the M-VH comprises an amino acid sequence having at least 90% sequence identity to SEQ ID NO: 16, and the M-VL comprises an amino acid sequence having at least 90% sequence identity to SEQ ID NO: 17; or

the M-VH comprises an amino acid sequence having at least 90% sequence identity to SEQ ID NO: 12, and the M-VL comprises an amino acid sequence having at least 90% sequence identity to SEQ ID NO: 13; or
the M-VH comprises an amino acid sequence having at least 90% sequence identity to SEQ ID NO: 14, and the M-VL comprises an amino acid sequence having at least 90% sequence identity to SEQ ID NO: 15; and/or

(ii) the E-VH comprises an amino acid sequence having at least 90% sequence identity to SEQ ID NO: 3, and the E-VL comprises an amino acid sequence having at least 90% sequence identity to SEQ ID NO: 5 or SEQ ID NO: 4;

preferably,

(i) the bispecific antibody specifically binding to HGFR and EGFR comprises two antigen-binding moieties 1 specifically binding to HGFR and one antigen-binding moiety 2 specifically binding to EGFR, wherein

one of the antigen-binding moieties 1 comprises an M-VH set forth in SEQ ID NO: 16 and an M-VL set forth in SEQ ID NO: 17;
the other antigen-binding moiety 1 comprises an M-VH set forth in SEQ ID NO: 12 and an M-VL set forth in SEQ ID NO: 13; and
the antigen-binding moiety 2 comprises an E-VH set forth in SEQ ID NO: 3 and an E-VL set forth in SEQ ID NO: 5; or

(ii) the bispecific antibody specifically binding to HGFR and EGFR comprises one antigen-binding moiety 1 specifically binding to HGFR and one antigen-binding moiety 2 specifically binding to EGFR, wherein
the antigen-binding moiety 1 comprises an M-VH set forth in SEQ ID NO: 12 and an M-VL set forth in SEQ ID NO: 13, and the antigen-binding moiety 2 comprises an E-VH set forth in SEQ ID NO: 3 and an E-VL set forth in SEQ ID NO: 5.

In some embodiments, provided is the pharmaceutical composition according to any one of the above, wherein:

the bispecific antibody specifically binding to HGFR and EGFR comprises one first chain set forth in SEQ ID NO: 34, one second chain set forth in SEQ ID NO: 35, one third chain set forth in SEQ ID NO: 36, and one fourth chain set forth in SEQ ID NO: 37; or
comprises one first chain set forth in SEQ ID NO: 38, one second chain set forth in SEQ ID NO: 39, one third chain set forth in SEQ ID NO: 40, and one fourth chain set forth in SEQ ID NO: 41.

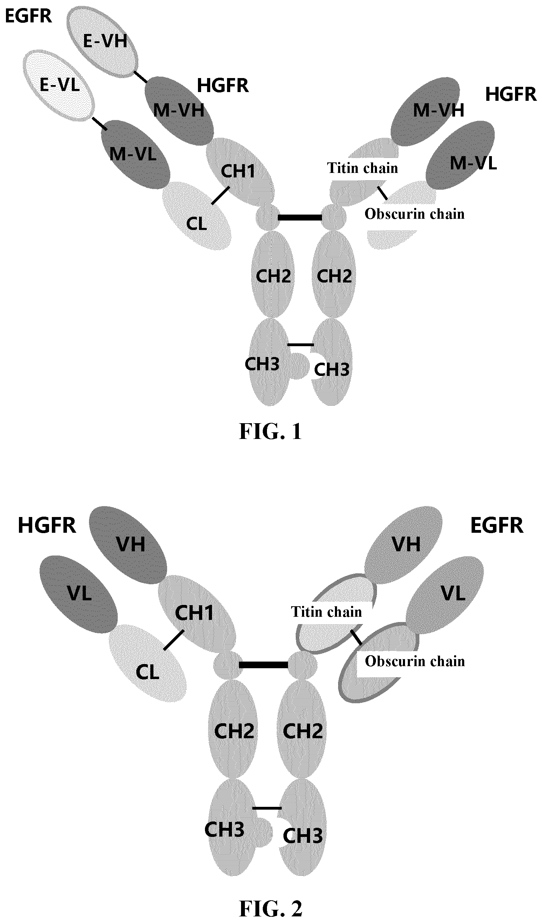

In some embodiments, provided is the pharmaceutical composition according to any one of the above, wherein the bispecific antibody specifically binding to HGFR and EGFR has a Format1 or Format2 structure. The structural schematic diagram of Format1 is shown in FIG. 1, and the structural schematic diagram of Format2 is shown in FIG. 2.

In some embodiments, provided is the pharmaceutical composition according to any one of the above, wherein the bispecific antibody specifically binding to HGFR and EGFR is a low-fucosylated bispecific antibody; preferably, the low-fucosylated bispecific antibody is an antibody that is at least 80%, 85%, 90%, 95%, or 100% unmodified by fucosylation; more preferably, no fucosylation modification is detected in the bispecific antibody.

In some embodiments, provided is the pharmaceutical composition according to any one of the above, which comprises the following components:

(a) 1 mg/mL to 250 mg/mL said bispecific antibody specifically binding to HGFR and EGFR,
(b) 0.01 mg/mL to 1.0 mg/mL surfactant,
(c) 10 mg/mL to 100 mg/mL sugar, and
(d) 5 mM to 100 mM buffer; the pharmaceutical composition has a pH of 5.0 to 6.5.

In some embodiments, provided is the pharmaceutical composition according to any one of the above, which comprises the following components:

(a) 1 mg/mL to 200 mg/mL said bispecific antibody specifically binding to HGFR and EGFR,
(b) 0.1 mg/mL to 0.8 mg/mL polysorbate 80,
(c) 30 mg/mL to 80 mg/mL sucrose, and
(d) 10 mM to 50 mM histidine buffer; the pharmaceutical composition has a pH of 5.5 to 6.5.

In some embodiments, provided is the pharmaceutical composition according to any one of the above, which comprises the following components:

(a) 90 mg/mL to 110 mg/mL bispecific antibody specifically binding to HGFR and EGFR,
(b) 0.4 mg/mL to 0.8 mg/mL polysorbate 80,
(c) 70 mg/mL to 80 mg/mL sucrose, and
(d) 10 mM to 20 mM histidine-histidine hydrochloride buffer or histidine-histidine acetate buffer; the pharmaceutical composition has a pH of about 6.0.

In some embodiments, provided is the pharmaceutical composition according to any one of the above, which comprises the following components:

(a) 90 mg/mL to 110 mg/mL bispecific antibody specifically binding to HGFR and EGFR,
(b) 0.6 mg/mL polysorbate 80,
(c) 70 mg/mL to 80 mg/mL sucrose, and
(d) 20 mM histidine-histidine hydrochloride buffer; the pharmaceutical composition has a pH of about 6.0.

In some embodiments, provided is the pharmaceutical composition according to any one of the above, which comprises the following components:

(a) 120 mg/mL bispecific antibody specifically binding to HGFR and EGFR,
(b) 0.6 mg/mL polysorbate 80,
(c) 70 mg/mL to 80 mg/mL sucrose,
(d) 2000 U/mL PH20, and
(e) 20 mM histidine-histidine hydrochloride buffer; the pharmaceutical composition has a pH of about 6.0.

In some embodiments, provided is the pharmaceutical composition according to any one of the above, which comprises the following components:

(a) 150 mg/mL bispecific antibody specifically binding to HGFR and EGFR,
(b) 0.6 mg/mL polysorbate 80,
(c) 70 mg/mL to 80 mg/mL sucrose,
(d) 2000 U/mL PH20, and
(e) 20 mM histidine-histidine hydrochloride buffer; the pharmaceutical composition has a pH of about 5.0.

In some embodiments, provided is the pharmaceutical composition according to any one of the above, which comprises the following components:

(a) 200 mg/mL bispecific antibody specifically binding to HGFR and EGFR,
(b) 0.6 mg/mL polysorbate 80,
(c) 70 mg/mL to 80 mg/mL sucrose,
(d) 200 mM arginine hydrochloride,
(e) 2000 U/mL PH20, and
(f) 20 mM histidine-histidine hydrochloride buffer; the pharmaceutical composition has a pH of about 5.0.

In some embodiments, provided is the pharmaceutical composition according to any one of the above, which comprises the following components:

(a) 200 mg/mL bispecific antibody specifically binding to HGFR and EGFR,
(b) 0.6 mg/mL polysorbate 80,
(c) 70 mg/mL to 80 mg/mL sucrose,
(d) 100 mM arginine hydrochloride,
(e) 2000 U/mL PH20, and
(f) 20 mM histidine-histidine hydrochloride buffer; the pharmaceutical composition has a pH of about 5.0.

In some embodiments, provided is the pharmaceutical composition according to any one of the above, which comprises the following components:

(a) 1 mg/mL to 250 mg/mL said bispecific antibody specifically binding to HGFR and EGFR,
(b) 0.01 mg/mL to 1.0 mg/mL surfactant,
(c) 10 mg/mL to 100 mg/mL sugar, and
(d) 5 mM to 100 mM buffer; the pharmaceutical composition has a pH of 4.5 to 6.5.

In some embodiments, provided is the pharmaceutical composition according to any one of the above, which comprises the following components:

(a) 1 mg/mL to 200 mg/mL said bispecific antibody specifically binding to HGFR and EGFR,
(b) 0.1 mg/mL to 0.8 mg/mL polysorbate 80,
(c) 20 mg/mL to 80 mg/mL sucrose, and
(d) 5 mM to 50 mM histidine buffer; the pharmaceutical composition has a pH of 4.8 to 6.2.

In some embodiments, provided is the pharmaceutical composition according to any one of the above, which comprises the following components:

(a) 80 mg/mL to 180 mg/mL bispecific antibody specifically binding to HGFR and EGFR,
(b) 0.4 mg/mL to 0.8 mg/mL polysorbate 80,
(c) 60 mg/mL to 80 mg/mL sucrose, and
(d) 5 mM to 30 mM histidine-histidine hydrochloride buffer or histidine-histidine acetate buffer; the pharmaceutical composition has a pH of 5.0 to 6.0.

In some embodiments, provided is the pharmaceutical composition according to any one of the above, which comprises the following components:

(a) 1 mg/mL to 200 mg/mL said bispecific antibody specifically binding to HGFR and EGFR,
(b) 0.1 mg/mL to 0.8 mg/mL polysorbate 80,
(c) 20 mg/mL to 80 mg/mL sucrose, and
(d) 5 mM to 50 mM histidine buffer; the pharmaceutical composition has a pH of 5.0 to 6.5.

In some embodiments, provided is the pharmaceutical composition according to any one of the above, which comprises the following components:

(a) 50 mg/mL to 200 mg/mL bispecific antibody specifically binding to HGFR and EGFR,
(b) 0.4 mg/mL to 0.8 mg/mL polysorbate 80,

(c) 60 mg/mL to 80 mg/mL sucrose, and
(d) 10 mM to 30 mM histidine-histidine hydrochloride buffer or histidine-histidine acetate buffer; the pharmaceutical composition has a pH of 5.5 to 6.5.

In some embodiments, provided is the pharmaceutical composition according to any one of the above, which comprises the following components:

(a) 90 mg/mL to 110 mg/mL bispecific antibody specifically binding to HGFR and EGFR,
(b) 0.4 mg/mL to 0.8 mg/mL polysorbate 80,
(c) 70 mg/mL to 80 mg/mL sucrose, and
(d) 15 mM to 25 mM histidine-histidine hydrochloride buffer; the pharmaceutical composition has a pH of 5.8 to 6.2.

In some embodiments, provided is the pharmaceutical composition according to any one of the above, which comprises the following components:

(a) about 100 mg/mL bispecific antibody specifically binding to HGFR and EGFR,
(b) about 0.6 mg/mL polysorbate 80,
(c) about 75 mg/mL sucrose, and
(d) about 20 mM histidine-histidine hydrochloride buffer; the pharmaceutical composition has a pH of about 6.0.

In some embodiments, provided is the pharmaceutical composition according to any one of the above, which comprises the following components:

(a) 100 mg/mL bispecific antibody specifically binding to HGFR and EGFR,
(b) 0.6 mg/mL polysorbate 80,
(c) 75 mg/mL sucrose, and
(d) 20 mM histidine-histidine hydrochloride buffer; the pharmaceutical composition has a pH of 6.0.

In some embodiments, provided is the pharmaceutical composition according to any one of the above, which comprises the following components:

(a) 1 mg/mL to 250 mg/mL said bispecific antibody specifically binding to HGFR and EGFR,
(b) 0.01 mg/mL to 1.0 mg/mL surfactant,
(c) 10 mg/mL to 100 mg/mL sugar,
(d) 1 mM to 300 mM auxiliary material,
(e) 10 U/mL to 3000 U/mL enzyme, and
(f) 5 mM to 100 mM buffer; the pharmaceutical composition has a pH of 4.5 to 6.5.

In some embodiments, provided is the pharmaceutical composition according to any one of the above, which comprises the following components:

(a) 1 mg/mL to 200 mg/mL said bispecific antibody specifically binding to HGFR and EGFR,
(b) 0.1 mg/mL to 0.8 mg/mL polysorbate 80,
(c) 20 mg/mL to 80 mg/mL sucrose,
(d) 1 mM to 200 mM methionine,
(e) 700 U/mL to 2400 U/mL hyaluronidase, and
(f) 5 mM to 50 mM histidine buffer; the pharmaceutical composition has a pH of 4.8 to 6.2.

In some embodiments, provided is the pharmaceutical composition according to any one of the above, which comprises the following components:

(a) 80 mg/mL to 180 mg/mL bispecific antibody specifically binding to HGFR and EGFR,
(b) 0.4 mg/mL to 0.8 mg/mL polysorbate 80,
(c) 60 mg/mL to 80 mg/mL sucrose,
(d) 1 mM to 20 mM methionine,
(e) 1600 U/mL to 2400 U/mL hyaluronidase, and
(f) 5 mM to 30 mM histidine-histidine hydrochloride buffer or histidine-histidine acetate buffer; the pharmaceutical composition has a pH of 5.0 to 6.0.

In some embodiments, provided is the pharmaceutical composition according to any one of the above, which comprises the following components:

(a) 50 mg/mL to 200 mg/mL said bispecific antibody specifically binding to HGFR and EGFR,
(b) 0.1 mg/mL to 0.8 mg/mL polysorbate 80,
(c) 20 mg/mL to 80 mg/mL sucrose,
(d) 1 mM to 200 mM methionine,
(e) 150 U/mL to 2200 U/mL hyaluronidase, and
(f) 5 mM to 50 mM histidine buffer; the pharmaceutical composition has a pH of 4.5 to 6.0.

In some embodiments, provided is the pharmaceutical composition according to any one of the above, which comprises the following components:

(a) 100 mg/mL to 200 mg/mL bispecific antibody specifically binding to HGFR and EGFR,
(b) 0.4 mg/mL to 0.8 mg/mL polysorbate 80,
(c) 60 mg/mL to 80 mg/mL sucrose,
(d) 4 mM to 20 mM methionine,
(e) 800 U/mL to 2200 U/mL hyaluronidase, and
(f) 10 mM to 30 mM histidine-histidine hydrochloride buffer or histidine-histidine acetate buffer; the pharmaceutical composition has a pH of 4.5 to 5.5.

In some embodiments, provided is the pharmaceutical composition according to any one of the above, which comprises the following components:

(a) 120 mg/mL to 180 mg/mL bispecific antibody specifically binding to HGFR and EGFR,
(b) 0.4 mg/mL to 0.8 mg/mL polysorbate 80,
(c) 60 mg/mL to 80 mg/mL sucrose,
(d) 1 mM to 20 mM methionine,
(e) 1600 U/mL to 2400 U/mL hyaluronidase, and
(f) 20 mM to 30 mM histidine-histidine hydrochloride buffer; the pharmaceutical composition has a pH of 4.8 to 5.2.

In some embodiments, provided is the pharmaceutical composition according to any one of the above, which comprises the following components:

(a) about 150 mg/mL bispecific antibody specifically binding to HGFR and EGFR,
(b) about 0.6 mg/mL polysorbate 80,
(c) about 70 mg/mL sucrose,
(d) about 10 mM methionine,
(e) about 2000 U/mL hyaluronidase, and
(f) about 25 mM histidine-histidine hydrochloride buffer; the pharmaceutical composition has a pH of about 5.0.

In some embodiments, provided is the pharmaceutical composition according to any one of the above, which comprises the following components:

(a) 150 mg/mL bispecific antibody specifically binding to HGFR and EGFR,
(b) 0.6 mg/mL polysorbate 80,
(c) 70 mg/mL sucrose,
(d) 10 mM methionine,
(e) 2000 U/mL hyaluronidase, and
(f) 25 mM histidine-histidine hydrochloride buffer; the pharmaceutical composition has a pH of 5.0.

In some embodiments, provided is the pharmaceutical composition according to any one of the above, which comprises the following components:

(a) 50 mg/mL to 100 mg/mL bispecific antibody specifically binding to HGFR and EGFR,
(b) 0.2 mg/mL to 0.4 mg/mL polysorbate 80,
(c) 20 mg/mL to 50 mg/mL sucrose,
(d) 1 mM to 10 mM methionine,

(e) 600 U/mL to 1000 U/mL hyaluronidase, and
(f) 5 mM to 15 mM histidine-histidine hydrochloride buffer or histidine-histidine acetate buffer; the pharmaceutical composition has a pH of 4.5 to 5.5.

In some embodiments, provided is the pharmaceutical composition according to any one of the above, which comprises the following components:

(a) 50 mg/mL to 75 mg/mL bispecific antibody specifically binding to HGFR and EGFR,
(b) 0.2 mg/mL to 0.3 mg/mL polysorbate 80,
(c) 20 mg/mL to 35 mg/mL sucrose,
(d) 1 mM to 10 mM methionine,
(e) 720 U/mL to 880 U/mL hyaluronidase, and
(f) 8 mM to 12 mM histidine-histidine hydrochloride buffer; the pharmaceutical composition has a pH of 4.8 to 5.2.

In some embodiments, provided is the pharmaceutical composition according to any one of the above, which comprises the following components:

(a) about 60 mg/mL bispecific antibody specifically binding to HGFR and EGFR,
(b) about 0.24 mg/mL polysorbate 80,
(c) about 28 mg/mL sucrose,
(d) about 4 mM methionine,
(e) about 800 U/mL hyaluronidase, and
(f) about 10 mM histidine-histidine hydrochloride buffer; the pharmaceutical composition has a pH of about 5.0.

In some embodiments, provided is the pharmaceutical composition according to any one of the above, which comprises the following components:

(a) 60 mg/mL bispecific antibody specifically binding to HGFR and EGFR,
(b) 0.24 mg/mL polysorbate 80,
(c) 28 mg/mL sucrose,
(d) 4 mM methionine,
(e) 800 U/mL hyaluronidase, and
(f) 10 mM histidine-histidine hydrochloride buffer; the pharmaceutical composition has a pH of 5.0.

In some embodiments, provided is the pharmaceutical composition according to any one of the above, which comprises the following components:

(a) 50 mg/mL to 250 mg/mL said bispecific antibody specifically binding to HGFR and EGFR,
(b) 0.1 mg/mL to 0.8 mg/mL polysorbate 80,
(c) 20 mg/mL to 80 mg/mL sucrose,
(d) 80 mM to 240 mM arginine hydrochloride, and
(e) 5 mM to 50 mM histidine buffer; the pharmaceutical composition has a pH of 4.5 to 6.0.

In some embodiments, provided is the pharmaceutical composition according to any one of the above, which comprises the following components:

(a) 160 mg/mL to 240 mg/mL bispecific antibody specifically binding to HGFR and EGFR,
(b) 0.4 mg/mL to 0.8 mg/mL polysorbate 80,
(c) 60 mg/mL to 80 mg/mL sucrose,
(d) 160 mM to 240 mM arginine hydrochloride, and
(e) 10 mM to 30 mM histidine-histidine hydrochloride buffer or histidine-histidine acetate buffer; the pharmaceutical composition has a pH of 4.5 to 5.5.

In some embodiments, provided is the pharmaceutical composition according to any one of the above, which comprises the following components:

(a) 180 mg/mL to 220 mg/mL bispecific antibody specifically binding to HGFR and EGFR,
(b) 0.4 mg/mL to 0.8 mg/mL polysorbate 80,

(c) 70 mg/mL to 80 mg/mL sucrose,
(d) 180 mM to 220 mM arginine hydrochloride, and
(e) 15 mM to 25 mM histidine-histidine hydrochloride buffer; the pharmaceutical composition has a pH of 4.8 to 5.2.

In some embodiments, provided is the pharmaceutical composition according to any one of the above, which comprises the following components:

(a) about 200 mg/mL bispecific antibody specifically binding to HGFR and EGFR,
(b) about 0.6 mg/mL polysorbate 80,
(c) about 75 mg/mL sucrose,
(d) about 200 mM arginine hydrochloride, and
(e) about 20 mM histidine-histidine hydrochloride buffer; the pharmaceutical composition has a pH of about 5.0.

In some embodiments, provided is the pharmaceutical composition according to any one of the above, which comprises the following components:

(a) 200 mg/mL bispecific antibody specifically binding to HGFR and EGFR,
(b) 0.6 mg/mL polysorbate 80,
(c) 75 mg/mL sucrose,
(d) 200 mM arginine hydrochloride, and
(e) 20 mM histidine-histidine hydrochloride buffer; the pharmaceutical composition has a pH of 5.0.

In some embodiments, provided is the pharmaceutical composition according to any one of the above, which comprises the following components:

(a) 50 mg/mL to 150 mg/mL bispecific antibody specifically binding to HGFR and EGFR,
(b) 0.2 mg/mL to 0.4 mg/mL polysorbate 80,
(c) 20 mg/mL to 50 mg/mL sucrose,
(d) 80 mM to 120 mM arginine hydrochloride, and
(e) 5 mM to 15 mM histidine-histidine hydrochloride buffer or histidine-histidine acetate buffer; the pharmaceutical composition has a pH of 4.5 to 5.5.

In some embodiments, provided is the pharmaceutical composition according to any one of the above, which comprises the following components:

(a) 80 mg/mL to 120 mg/mL bispecific antibody specifically binding to HGFR and EGFR,
(b) 0.2 mg/mL to 0.4 mg/mL polysorbate 80,
(c) 30 mg/mL to 50 mg/mL sucrose,
(d) 90 mM to 110 mM arginine hydrochloride, and
(e) 15 mM to 25 mM histidine-histidine hydrochloride buffer; the pharmaceutical composition has a pH of 4.8 to 5.2.

In some embodiments, provided is the pharmaceutical composition according to any one of the above, which comprises the following components:

(a) about 100 mg/mL bispecific antibody specifically binding to HGFR and EGFR,
(b) about 0.3 mg/mL polysorbate 80,
(c) about 37.5 mg/mL sucrose,
(d) about 100 mM arginine hydrochloride, and
(e) about 10 mM histidine-histidine hydrochloride buffer; the pharmaceutical composition has a pH of about 5.0.

In some embodiments, provided is the pharmaceutical composition according to any one of the above, which comprises the following components:

(a) 100 mg/mL bispecific antibody specifically binding to HGFR and EGFR,
(b) 0.3 mg/mL polysorbate 80,
(c) 37.5 mg/mL sucrose,
(d) 100 mM arginine hydrochloride, and
(e) 10 mM histidine-histidine hydrochloride buffer; the pharmaceutical composition has a pH of 5.0.

In some embodiments, provided is the pharmaceutical composition according to any one of the above, which comprises the following components:

(a) 120 mg/mL bispecific antibody specifically binding to HGFR and EGFR,
(b) 0.6 mg/mL polysorbate 80,
(c) 75 mg/mL sucrose,
(d) 10 mM methionine,
(e) 2000 U/mL PH20, and
(f) 20 mM histidine-histidine hydrochloride buffer; the pharmaceutical composition has a pH of 5.0 or 6.0.

In some embodiments, provided is the pharmaceutical composition according to any one of the above, which comprises the following components:

(a) 120 mg/mL bispecific antibody specifically binding to HGFR and EGFR,
(b) 0.6 mg/mL polysorbate 80,
(c) 75 mg/mL sucrose,
(d) 2000 U/mL PH20, and
(e) 20 mM histidine-histidine hydrochloride buffer; the pharmaceutical composition has a pH of 6.0.

In some embodiments, provided is the pharmaceutical composition according to any one of the above, which comprises the following components:

(a) 150 mg/mL bispecific antibody specifically binding to HGFR and EGFR,
(b) 0.6 mg/mL polysorbate 80,
(c) 75 mg/mL sucrose,
(d) 10 mM methionine,
(e) 2000 U/mL PH20, and
(f) 20 mM histidine-histidine hydrochloride buffer; the pharmaceutical composition has a pH of 5.0.

In some embodiments, provided is the pharmaceutical composition according to any one of the above, which comprises the following components:

(a) 150 mg/mL bispecific antibody specifically binding to HGFR and EGFR,
(b) 0.6 mg/mL polysorbate 80,
(c) 75 mg/mL sucrose, and
(d) 20 mM histidine-histidine hydrochloride buffer; the pharmaceutical composition has a pH of 5.0.

In some embodiments, provided is the pharmaceutical composition according to any one of the above, which comprises the following components:

(a) 120 mg/mL bispecific antibody specifically binding to HGFR and EGFR,
(b) 0.6 mg/mL polysorbate 80,
(c) 75 mg/mL sucrose, and
(d) 20 mM histidine-histidine hydrochloride buffer; the pharmaceutical composition has a pH of 6.0.

In some embodiments, provided is the pharmaceutical composition according to any one of the above, which comprises the following components:

(a) 60 mg/mL bispecific antibody specifically binding to HGFR and EGFR,
(b) 0.3 mg/mL polysorbate 80,
(c) 37.5 mg/mL sucrose,
(d) 5 mM methionine,
(e) 1000 U/mL PH20, and
(f) 10 mM histidine-histidine hydrochloride buffer; the pharmaceutical composition has a pH of 5.0 or 6.0.

In some embodiments, provided is the pharmaceutical composition according to any one of the above, which comprises the following components:

(a) 60 mg/mL bispecific antibody specifically binding to HGFR and EGFR,
(b) 0.3 mg/mL polysorbate 80,
(c) 37.5 mg/mL sucrose,
(d) 1000 U/mL PH20, and
(e) 10 mM histidine-histidine hydrochloride buffer; the pharmaceutical composition has a pH of 6.0.

In some embodiments, provided is the pharmaceutical composition according to any one of the above, which comprises the following components:

(a) 75 mg/mL bispecific antibody specifically binding to HGFR and EGFR,
(b) 0.3 mg/mL polysorbate 80,
(c) 37.5 mg/mL sucrose,
(d) 5 mM methionine,
(e) 1000 U/mL PH20, and
(f) 10 mM histidine-histidine hydrochloride buffer; the pharmaceutical composition has a pH of 5.0.

In some embodiments, provided is the pharmaceutical composition according to any one of the above, which comprises the following components:

(a) 75 mg/mL bispecific antibody specifically binding to HGFR and EGFR,
(b) 0.3 mg/mL polysorbate 80,
(c) 37.5 mg/mL sucrose, and
(d) 10 mM histidine-histidine hydrochloride buffer; the pharmaceutical composition has a pH of 5.0.

In some embodiments, provided is the pharmaceutical composition according to any one of the above, which comprises the following components:

(a) 60 mg/mL bispecific antibody specifically binding to HGFR and EGFR,
(b) 0.3 mg/mL polysorbate 80,
(c) 37.5 mg/mL sucrose, and
(d) 10 mM histidine-histidine hydrochloride buffer; the pharmaceutical composition has a pH of 6.0.

In some embodiments, provided is the pharmaceutical composition according to the above, wherein the pharmaceutical composition is a liquid formulation. In some embodiments, the solvent of the liquid formulation is water.

The present disclosure further provides a freeze-dried formulation, wherein the freeze-dried formulation is capable of being reconstituted to form the pharmaceutical composition according to any one of the above.

The present disclosure further provides a freeze-dried formulation, which is a formulation in a freeze-dried form of the pharmaceutical composition according to any one of the above.

The present disclosure further provides a method for preparing a freeze-dried formulation, the method comprising a step of lyophilizing the pharmaceutical composition according to any one of the above. In some embodiments, the lyophilization according to any one of the above comprises steps of pre-freezing, primary drying, and secondary drying in sequence.

The present disclosure further provides a freeze-dried formulation, wherein the formulation is obtained by lyophilizing the pharmaceutical composition according to any one of the above.

The present disclosure further provides a reconstituted solution, wherein the reconstituted solution is prepared by reconstituting the freeze-dried formulation according to any one of the above.

The present disclosure further provides a reconstituted solution, which is a formulation in a reconstituted form of the freeze-dried formulation according to any one of the above.

In some embodiments, provided is the reconstituted solution according to any one of the above, the components and contents of which are identical to those of the aforementioned pharmaceutical composition.

In some embodiments, provided is the reconstituted solution according to any one of the above, which comprises the following components:

(a) 1 mg/mL to 250 mg/mL said bispecific antibody specifically binding to HGFR and EGFR,
(b) 0.01 mg/mL to 1.0 mg/mL surfactant,
(c) 10 mg/mL to 100 mg/mL sugar, and
(d) 5 mM to 100 mM buffer; the pharmaceutical composition has a pH of 5.0 to 6.5.

In some embodiments, provided is the reconstituted solution according to any one of the above, which comprises the following components:

(a) 1 mg/mL to 200 mg/mL said bispecific antibody specifically binding to HGFR and EGFR,
(b) 0.1 mg/mL to 0.8 mg/mL polysorbate 80,
(c) 30 mg/mL to 80 mg/mL sucrose, and
(d) 10 mM to 50 mM histidine buffer; the pharmaceutical composition has a pH of 5.5 to 6.5.

In some embodiments, provided is the reconstituted solution according to any one of the above, which comprises the following components:

(a) 90 mg/mL to 110 mg/mL bispecific antibody specifically binding to HGFR and EGFR,
(b) 0.4 mg/mL to 0.8 mg/mL polysorbate 80,
(c) 70 mg/mL to 80 mg/mL sucrose, and
(d) 10 mM to 20 mM histidine-histidine hydrochloride buffer or histidine-histidine acetate buffer; the pharmaceutical composition has a pH of about 6.0.

In some embodiments, provided is the reconstituted solution according to any one of the above, which comprises the following components:

(a) 90 mg/mL to 110 mg/mL bispecific antibody specifically binding to HGFR and EGFR,
(b) 0.6 mg/mL polysorbate 80,
(c) 70 mg/mL to 80 mg/mL sucrose, and
(d) 20 mM histidine-histidine hydrochloride buffer; the pharmaceutical composition has a pH of about 6.0.

In some embodiments, provided is the reconstituted solution according to any one of the above, which comprises the following components:

(a) 120 mg/mL bispecific antibody specifically binding to HGFR and EGFR,
(b) 0.6 mg/mL polysorbate 80,
(c) 70 mg/mL to 80 mg/mL sucrose,
(d) 2000 U/mL PH20, and
(e) 20 mM histidine-histidine hydrochloride buffer; the pharmaceutical composition has a pH of about 6.0.

In some embodiments, provided is the reconstituted solution according to any one of the above, which comprises the following components:

(a) 150 mg/mL bispecific antibody specifically binding to HGFR and EGFR,
(b) 0.6 mg/mL polysorbate 80,
(c) 70 mg/mL to 80 mg/mL sucrose,
(d) 2000 U/mL PH20, and
(e) 20 mM histidine-histidine hydrochloride buffer; the pharmaceutical composition has a pH of about 5.0.

In some embodiments, provided is the reconstituted solution according to any one of the above, which comprises the following components:

(a) 200 mg/mL bispecific antibody specifically binding to HGFR and EGFR,
(b) 0.6 mg/mL polysorbate 80,
(c) 70 mg/mL to 80 mg/mL sucrose,
(d) 200 mM arginine hydrochloride,
(e) 2000 U/mL PH20, and
(f) 20 mM histidine-histidine hydrochloride buffer; the pharmaceutical composition has a pH of about 5.0.

In some embodiments, provided is the reconstituted solution according to any one of the above, which comprises the following components:

(a) 200 mg/mL bispecific antibody specifically binding to HGFR and EGFR,
(b) 0.6 mg/mL polysorbate 80,

(c) 70 mg/mL to 80 mg/mL sucrose,
(d) 100 mM arginine hydrochloride,
(e) 2000 U/mL PH20, and
(f) 20 mM histidine-histidine hydrochloride buffer; the pharmaceutical composition has a pH of about 5.0.

In some embodiments, provided is the reconstituted solution according to any one of the above, which comprises the following components:

(a) 1 mg/mL to 250 mg/mL said bispecific antibody specifically binding to HGFR and EGFR,
(b) 0.01 mg/mL to 1.0 mg/mL surfactant,
(c) 10 mg/mL to 100 mg/mL sugar, and
(d) 5 mM to 100 mM buffer; the reconstituted solution has a pH of 4.5 to 6.5.

In some embodiments, provided is the reconstituted solution according to any one of the above, which comprises the following components:

(a) 1 mg/mL to 200 mg/mL said bispecific antibody specifically binding to HGFR and EGFR,
(b) 0.1 mg/mL to 0.8 mg/mL polysorbate 80,
(c) 20 mg/mL to 80 mg/mL sucrose, and
(d) 5 mM to 50 mM histidine buffer; the pharmaceutical composition has a pH of 4.8 to 6.2.

In some embodiments, provided is the reconstituted solution according to any one of the above, which comprises the following components:

(a) 80 mg/mL to 180 mg/mL bispecific antibody specifically binding to HGFR and EGFR,
(b) 0.4 mg/mL to 0.8 mg/mL polysorbate 80,
(c) 60 mg/mL to 80 mg/mL sucrose, and
(d) 5 mM to 30 mM histidine-histidine hydrochloride buffer or histidine-histidine acetate buffer; the pharmaceutical composition has a pH of 5.0 to 6.0.

In some embodiments, provided is the reconstituted solution according to any one of the above, which comprises the following components:

(a) 1 mg/mL to 200 mg/mL said bispecific antibody specifically binding to HGFR and EGFR,
(b) 0.1 mg/mL to 0.8 mg/mL polysorbate 80,
(c) 20 mg/mL to 80 mg/mL sucrose, and
(d) 5 mM to 50 mM histidine buffer; the reconstituted solution has a pH of 5.0 to 6.5.

In some embodiments, provided is the reconstituted solution according to any one of the above, which comprises the following components:

(a) 50 mg/mL to 200 mg/mL bispecific antibody specifically binding to HGFR and EGFR,
(b) 0.4 mg/mL to 0.8 mg/mL polysorbate 80,
(c) 60 mg/mL to 80 mg/mL sucrose, and
(d) 10 mM to 30 mM histidine-histidine hydrochloride buffer or histidine-histidine acetate buffer; the reconstituted solution has a pH of 5.5 to 6.5.

In some embodiments, provided is the reconstituted solution according to any one of the above, which comprises the following components:

(a) 90 mg/mL to 110 mg/mL bispecific antibody specifically binding to HGFR and EGFR,
(b) 0.4 mg/mL to 0.8 mg/mL polysorbate 80,
(c) 70 mg/mL to 80 mg/mL sucrose, and
(d) 15 mM to 25 mM histidine-histidine hydrochloride buffer; the reconstituted solution has a pH of 5.8 to 6.2.

In some embodiments, provided is the reconstituted solution according to any one of the above, which comprises the following components:

(a) about 100 mg/mL bispecific antibody specifically binding to HGFR and EGFR,
(b) about 0.6 mg/mL polysorbate 80,
(c) about 75 mg/mL sucrose, and
(d) about 20 mM histidine-histidine hydrochloride buffer; the reconstituted solution has a pH of about 6.0.

In some embodiments, provided is the reconstituted solution according to any one of the above, which comprises the following components:

(a) 100 mg/mL bispecific antibody specifically binding to HGFR and EGFR,
(b) 0.6 mg/mL polysorbate 80,
(c) 75 mg/mL sucrose, and
(d) 20 mM histidine-histidine hydrochloride buffer; the reconstituted solution has a pH of 6.0.

In some embodiments, provided is the reconstituted solution according to any one of the above, which comprises the following components:

(a) 1 mg/mL to 250 mg/mL said bispecific antibody specifically binding to HGFR and EGFR,
(b) 0.01 mg/mL to 1.0 mg/mL surfactant,
(c) 10 mg/mL to 100 mg/mL sugar,
(d) 1 mM to 300 mM auxiliary material,
(e) 10 U/mL to 3000 U/mL enzyme, and
(f) 5 mM to 100 mM buffer, the reconstituted solution has a pH of 4.5 to 6.5.

In some embodiments, provided is the reconstituted solution according to any one of the above, which comprises the following components:

(a) 1 mg/mL to 200 mg/mL said bispecific antibody specifically binding to HGFR and EGFR,
(b) 0.1 mg/mL to 0.8 mg/mL polysorbate 80,
(c) 20 mg/mL to 80 mg/mL sucrose,
(d) 1 mM to 200 mM methionine,
(e) 700 U/mL to 2400 U/mL hyaluronidase, and
(f) 5 mM to 50 mM histidine buffer; the pharmaceutical composition has a pH of 4.8 to 6.2.

In some embodiments, provided is the reconstituted solution according to any one of the above, which comprises the following components:

(a) 80 mg/mL to 180 mg/mL bispecific antibody specifically binding to HGFR and EGFR,
(b) 0.4 mg/mL to 0.8 mg/mL polysorbate 80,
(c) 60 mg/mL to 80 mg/mL sucrose,
(d) 1 mM to 20 mM methionine,
(e) 1600 U/mL to 2400 U/mL hyaluronidase, and
(f) 5 mM to 30 mM histidine-histidine hydrochloride buffer or histidine-histidine acetate buffer; the pharmaceutical composition has a pH of 5.0 to 6.0.

In some embodiments, provided is the reconstituted solution according to any one of the above, which comprises the following components:

(a) 50 mg/mL to 200 mg/mL said bispecific antibody specifically binding to HGFR and EGFR,
(b) 0.1 mg/mL to 0.8 mg/mL polysorbate 80,
(c) 20 mg/mL to 80 mg/mL sucrose,
(d) 1 mM to 200 mM methionine,
(e) 150 U/mL to 2200 U/mL hyaluronidase, and
(f) 5 mM to 50 mM histidine buffer; the reconstituted solution has a pH of 4.5 to 6.0.

In some embodiments, provided is the reconstituted solution according to any one of the above, which comprises the following components:

(a) 100 mg/mL to 200 mg/mL bispecific antibody specifically binding to HGFR and EGFR,

(b) 0.4 mg/mL to 0.8 mg/mL polysorbate 80,

(c) 60 mg/mL to 80 mg/mL sucrose,

(d) 4 mM to 20 mM methionine,

(e) 800 U/mL to 2200 U/mL hyaluronidase, and

(f) 10 mM to 30 mM histidine-histidine hydrochloride buffer or histidine-histidine acetate buffer; the reconstituted solution has a pH of 4.5 to 5.5.

In some embodiments, provided is the reconstituted solution according to any one of the above, which comprises the following components:

(a) 120 mg/mL to 180 mg/mL bispecific antibody specifically binding to HGFR and EGFR,

(b) 0.4 mg/mL to 0.8 mg/mL polysorbate 80,

(c) 60 mg/mL to 80 mg/mL sucrose,

(d) 1 mM to 20 mM methionine,

(e) 1600 U/mL to 2400 U/mL hyaluronidase, and

(f) 20 mM to 30 mM histidine-histidine hydrochloride buffer; the reconstituted solution has a pH of 4.8 to 5.2.

In some embodiments, provided is the reconstituted solution according to any one of the above, which comprises the following components:

(a) about 150 mg/mL bispecific antibody specifically binding to HGFR and EGFR,

(b) about 0.6 mg/mL polysorbate 80,

(c) about 70 mg/mL sucrose,

(d) about 10 mM methionine,

(e) about 2000 U/mL hyaluronidase, and

(f) about 25 mM histidine-histidine hydrochloride buffer; the reconstituted solution has a pH of about 5.0.

In some embodiments, provided is the reconstituted solution according to any one of the above, which comprises the following components:

(a) 150 mg/mL bispecific antibody specifically binding to HGFR and EGFR,

(b) 0.6 mg/mL polysorbate 80,

(c) 70 mg/mL sucrose,

(d) 10 mM methionine,

(e) 2000 U/mL hyaluronidase, and

(f) 25 mM histidine-histidine hydrochloride buffer; the reconstituted solution has a pH of 5.0.

In some embodiments, provided is the reconstituted solution according to any one of the above, which comprises the following components:

(a) 50 mg/mL to 100 mg/mL bispecific antibody specifically binding to HGFR and EGFR,

(b) 0.2 mg/mL to 0.4 mg/mL polysorbate 80,

(c) 20 mg/mL to 50 mg/mL sucrose,

(d) 1 mM to 10 mM methionine,

(e) 600 U/mL to 1000 U/mL hyaluronidase, and

(f) 5 mM to 15 mM histidine-histidine hydrochloride buffer or histidine-histidine acetate buffer; the reconstituted solution has a pH of 4.5 to 5.5.

In some embodiments, provided is the reconstituted solution according to any one of the above, which comprises the following components:

(a) 50 mg/mL to 75 mg/mL bispecific antibody specifically binding to HGFR and EGFR,

(b) 0.2 mg/mL to 0.3 mg/mL polysorbate 80,

(c) 20 mg/mL to 35 mg/mL sucrose,

(d) 1 mM to 10 mM methionine,

(e) 720 U/mL to 880 U/mL hyaluronidase, and

(f) 8 mM to 12 mM histidine-histidine hydrochloride buffer; the reconstituted solution has a pH of 4.8 to 5.2.

In some embodiments, provided is the reconstituted solution according to any one of the above, which comprises the following components:

(a) about 60 mg/mL bispecific antibody specifically binding to HGFR and EGFR,
(b) about 0.24 mg/mL polysorbate 80,
(c) about 28 mg/mL sucrose,
(d) about 4 mM methionine,
(e) about 800 U/mL hyaluronidase, and
(f) about 10 mM histidine-histidine hydrochloride buffer; the reconstituted solution has a pH of about 5.0.

In some embodiments, provided is the reconstituted solution according to any one of the above, which comprises the following components:

(a) 60 mg/mL bispecific antibody specifically binding to HGFR and EGFR,
(b) 0.24 mg/mL polysorbate 80,
(c) 28 mg/mL sucrose,
(d) 4 mM methionine,
(e) 800 U/mL hyaluronidase, and
(f) 10 mM histidine-histidine hydrochloride buffer; the reconstituted solution has a pH of 5.0.

In some embodiments, provided is the reconstituted solution according to any one of the above, which comprises the following components:

(a) 50 mg/mL to 250 mg/mL said bispecific antibody specifically binding to HGFR and EGFR,
(b) 0.1 mg/mL to 0.8 mg/mL polysorbate 80,
(c) 20 mg/mL to 80 mg/mL sucrose,
(d) 80 mM to 240 mM arginine hydrochloride, and
(e) 5 mM to 50 mM histidine buffer; the reconstituted solution has a pH of 4.5 to 6.0.

In some embodiments, provided is the reconstituted solution according to any one of the above, which comprises the following components:

(a) 160 mg/mL to 240 mg/mL bispecific antibody specifically binding to HGFR and EGFR,
(b) 0.4 mg/mL to 0.8 mg/mL polysorbate 80,
(c) 60 mg/mL to 80 mg/mL sucrose,
(d) 160 mM to 240 mM arginine hydrochloride, and
(e) 10 mM to 30 mM histidine-histidine hydrochloride buffer or histidine-histidine acetate buffer; the reconstituted solution has a pH of 4.5 to 5.5.

In some embodiments, provided is the reconstituted solution according to any one of the above, which comprises the following components:

(a) 180 mg/mL to 220 mg/mL bispecific antibody specifically binding to HGFR and EGFR,
(b) 0.4 mg/mL to 0.8 mg/mL polysorbate 80,
(c) 70 mg/mL to 80 mg/mL sucrose,
(d) 180 mM to 220 mM arginine hydrochloride, and
(e) 15 mM to 25 mM histidine-histidine hydrochloride buffer; the reconstituted solution has a pH of 4.8 to 5.2.

In some embodiments, provided is the reconstituted solution according to any one of the above, which comprises the following components:

(a) about 200 mg/mL bispecific antibody specifically binding to HGFR and EGFR,
(b) about 0.6 mg/mL polysorbate 80,
(c) about 75 mg/mL sucrose,
(d) about 200 mM arginine hydrochloride, and
(e) about 20 mM histidine-histidine hydrochloride buffer; the reconstituted solution has a pH of about 5.0.

In some embodiments, provided is the reconstituted solution according to any one of the above, which comprises the following components:

    (a) 200 mg/mL bispecific antibody specifically binding to HGFR and EGFR,
    (b) 0.6 mg/mL polysorbate 80,
    (c) 75 mg/mL sucrose,
    (d) 200 mM arginine hydrochloride, and
    (e) 20 mM histidine-histidine hydrochloride buffer; the reconstituted solution has a pH of 5.0.

In some embodiments, provided is the reconstituted solution according to any one of the above, which comprises the following components:

    (a) 50 mg/mL to 150 mg/mL bispecific antibody specifically binding to HGFR and EGFR,
    (b) 0.2 mg/mL to 0.4 mg/mL polysorbate 80,
    (c) 20 mg/mL to 50 mg/mL sucrose,
    (d) 80 mM to 120 mM arginine hydrochloride, and
    (e) 5 mM to 15 mM histidine-histidine hydrochloride buffer or histidine-histidine acetate buffer; the reconstituted solution has a pH of 4.5 to 5.5.

In some embodiments, provided is the reconstituted solution according to any one of the above, which comprises the following components:

    (a) 80 mg/mL to 120 mg/mL bispecific antibody specifically binding to HGFR and EGFR,
    (b) 0.2 mg/mL to 0.4 mg/mL polysorbate 80,
    (c) 30 mg/mL to 50 mg/mL sucrose,
    (d) 90 mM to 110 mM arginine hydrochloride, and
    (e) 15 mM to 25 mM histidine-histidine hydrochloride buffer; the reconstituted solution has a pH of 4.8 to 5.2.

In some embodiments, provided is the reconstituted solution according to any one of the above, which comprises the following components:

    (a) about 100 mg/mL bispecific antibody specifically binding to HGFR and EGFR,
    (b) about 0.3 mg/mL polysorbate 80,
    (c) about 37.5 mg/mL sucrose,
    (d) about 100 mM arginine hydrochloride, and
    (e) about 10 mM histidine-histidine hydrochloride buffer; the reconstituted solution has a pH of about 5.0.

In some embodiments, provided is the reconstituted solution according to any one of the above, which comprises the following components:

    (a) 100 mg/mL bispecific antibody specifically binding to HGFR and EGFR,
    (b) 0.3 mg/mL polysorbate 80,
    (c) 37.5 mg/mL sucrose,
    (d) 100 mM arginine hydrochloride, and
    (e) 10 mM histidine-histidine hydrochloride buffer; the reconstituted solution has a pH of 5.0.

In some embodiments, provided is the reconstituted solution according to any one of the above, which comprises the following components:

    (a) 120 mg/mL bispecific antibody specifically binding to HGFR and EGFR,
    (b) 0.6 mg/mL polysorbate 80,
    (c) 75 mg/mL sucrose,
    (d) 10 mM methionine,
    (e) 2000 U/mL PH20, and
    (f) 20 mM histidine-histidine hydrochloride buffer; the reconstituted solution has a pH of 5.0 or 6.0.

In some embodiments, provided is the reconstituted solution according to any one of the above, which comprises the following components:

(a) 120 mg/mL bispecific antibody specifically binding to HGFR and EGFR,
(b) 0.6 mg/mL polysorbate 80,
(c) 75 mg/mL sucrose,
(d) 2000 U/mL PH20, and
(e) 20 mM histidine-histidine hydrochloride buffer; the reconstituted solution has a pH of 6.0.

In some embodiments, provided is the reconstituted solution according to any one of the above, which comprises the following components:

(a) 150 mg/mL bispecific antibody specifically binding to HGFR and EGFR,
(b) 0.6 mg/mL polysorbate 80,
(c) 75 mg/mL sucrose,
(d) 10 mM methionine,
(e) 2000 U/mL PH20, and
(f) 20 mM histidine-histidine hydrochloride buffer; the reconstituted solution has a pH of 5.0.

In some embodiments, provided is the reconstituted solution according to any one of the above, which comprises the following components:

(a) 150 mg/mL bispecific antibody specifically binding to HGFR and EGFR,
(b) 0.6 mg/mL polysorbate 80,
(c) 75 mg/mL sucrose, and
(d) 20 mM histidine-histidine hydrochloride buffer; the reconstituted solution has a pH of 5.0.

In some embodiments, provided is the reconstituted solution according to any one of the above, which comprises the following components:

(a) 120 mg/mL bispecific antibody specifically binding to HGFR and EGFR,
(b) 0.6 mg/mL polysorbate 80,
(c) 75 mg/mL sucrose, and
(d) 20 mM histidine-histidine hydrochloride buffer; the reconstituted solution has a pH of 6.0.

In some embodiments, provided is the reconstituted solution according to any one of the above, which comprises the following components:

(a) 60 mg/mL bispecific antibody specifically binding to HGFR and EGFR,
(b) 0.3 mg/mL polysorbate 80,
(c) 37.5 mg/mL sucrose,
(d) 5 mM methionine,
(e) 1000 U/mL PH20, and
(f) 10 mM histidine-histidine hydrochloride buffer; the reconstituted solution has a pH of 5.0 or 6.0.

In some embodiments, provided is the reconstituted solution according to any one of the above, which comprises the following components:

(a) 60 mg/mL bispecific antibody specifically binding to HGFR and EGFR,
(b) 0.3 mg/mL polysorbate 80,
(c) 37.5 mg/mL sucrose,
(d) 1000 U/mL PH20, and
(e) 10 mM histidine-histidine hydrochloride buffer; the reconstituted solution has a pH of 6.0.

In some embodiments, provided is the reconstituted solution according to any one of the above, which comprises the following components:

(a) 75 mg/mL bispecific antibody specifically binding to HGFR and EGFR,
(b) 0.3 mg/mL polysorbate 80,
(c) 37.5 mg/mL sucrose,
(d) 5 mM methionine,

(e) 1000 U/mL PH20, and
(f) 10 mM histidine-histidine hydrochloride buffer; the reconstituted solution has a pH of 5.0.

In some embodiments, provided is the reconstituted solution according to any one of the above, which comprises the following components:

(a) 75 mg/mL bispecific antibody specifically binding to HGFR and EGFR,
(b) 0.3 mg/mL polysorbate 80,
(c) 37.5 mg/mL sucrose, and
(d) 10 mM histidine-histidine hydrochloride buffer; the reconstituted solution has a pH of 5.0.

In some embodiments, provided is the reconstituted solution according to any one of the above, which comprises the following components:

(a) 60 mg/mL bispecific antibody specifically binding to HGFR and EGFR,
(b) 0.3 mg/mL polysorbate 80,
(c) 37.5 mg/mL sucrose, and
(d) 10 mM histidine-histidine hydrochloride buffer; the reconstituted solution has a pH of 6.0.

In some embodiments, provided is the pharmaceutical composition or the reconstituted solution according to any one of the above, which is a formulation for intravenous, subcutaneous, intraperitoneal, or intramuscular injection. In some embodiments, provided is the pharmaceutical composition or the reconstituted solution according to any one of the above, which is a formulation for intravenous injection. In some embodiments, provided is the pharmaceutical composition or the reconstituted solution according to any one of the above, which is a formulation for subcutaneous injection.

In some embodiments, provided is the pharmaceutical composition or the reconstituted solution according to any one of the above, which is suitable for intravenous, subcutaneous, intraperitoneal, or intramuscular injection. In some embodiments, provided is the pharmaceutical composition or the reconstituted solution according to any one of the above, which is suitable for intravenous injection. In some embodiments, provided is the pharmaceutical composition or the reconstituted solution according to any one of the above, which is suitable for subcutaneous injection.

In some embodiments, provided is the pharmaceutical composition or the reconstituted solution or the freeze-dried formulation according to any one of the above, which is used for preparing a medicament for intravenous, subcutaneous, intraperitoneal, or intramuscular injection. In some embodiments, provided is the pharmaceutical composition or the reconstituted solution or the freeze-dried formulation according to any one of the above, which is used for preparing a medicament for intravenous injection. In some embodiments, provided is the pharmaceutical composition or the reconstituted solution or the freeze-dried formulation according to any one of the above, which is used for preparing a medicament for subcutaneous injection.

The present disclosure further provides a kit comprising at least one container, wherein each container independently contains the pharmaceutical composition according to any one of the above, the freeze-dried formulation according to any one of the above, or the reconstituted solution according to any one of the above.

In some embodiments, the present disclosure further provides a method for diagnosing, treating, and alleviating a disorder in a subject, the method comprising administering to the subject an effective amount of the pharmaceutical composition according to any one of the above, the freeze-dried formulation according to any one of the above, the reconstituted solution according to any one of the above, or the kit according to any one of the above.

In some embodiments, the present disclosure further provides a method for treating or preventing a disease, the method comprising administering to a subject a therapeutically effective amount of the pharmaceutical composition according to any one of the above, the freeze-dried formulation according to any one of the above, the reconstituted solution according to any one of the above, or the kit according to any one of the above. In some embodiments, the present disclosure further provides the pharmaceutical composition according to any one of the above, the freeze-dried formulation according to any one of the above, the reconstituted solution according to any one of the above, or the kit according to any one of the above for use in the treatment or prevention of a disease.

In one aspect, the present disclosure further provides use of the pharmaceutical composition according to any one of the above, the freeze-dried formulation according to any one of the above, the reconstituted solution according to any one of the above, or the kit according to any one of the above in the preparation of a medicament for preventing or treating a disease or disorder.

In some specific embodiments, the disease according to any one of the above is a tumor. In some embodiments, the tumor is selected from the group consisting of lung cancer (including non-small cell lung cancer and small cell lung cancer), breast cancer, pancreatic cancer, colorectal cancer (including colon cancer and rectal cancer), sarcoma, renal cell carcinoma, hepatocellular carcinoma, gastric cancer, ovarian cancer, bladder cancer, head and neck cancer, and glioblastoma. In some embodiments, the tumor is selected from the group consisting of lung cancer, breast cancer, pancreatic cancer, colon cancer, head and neck cancer, gastric cancer, and glioblastoma.

In some specific embodiments, the lung cancer is non-small cell lung cancer.

In some specific embodiments, the lung cancer is metastatic non-small cell lung cancer. In some specific embodiments, the lung cancer is small cell lung cancer.

In some specific embodiments, the lung cancer is human lung adenocarcinoma.

In some specific embodiments, the lung cancer is gastric cancer.

In some specific embodiments, the tumor is an EGFR- and/or HGFR-associated tumor.

## BRIEF DESCRIPTION OF THE DRAWINGS

FIG. 1: a structural schematic diagram of Format1.

FIG. 2: a structural schematic diagram of Format2.

FIG. 3: experimental results for the binding activity of antibodies to EGFR CHO-S cells, wherein the ordinate of the graph represents the mean fluorescence intensity (abbreviated as MFI, this applies hereinafter).

FIG. 4: experimental results for the binding activity of antibodies to HGFR CHO-S cells.

FIG. 5: experimental results for the binding activity of antibodies to H1975-HGF cells.

FIG. 6: experimental results for the binding activity of antibodies to MKN-45 cells.

FIG. 7: experimental results for the inhibition of cellular EGFR phosphorylation by antibodies.

FIG. 8: experimental results for the inhibition of cellular HGFR phosphorylation by antibodies.

FIG. 9: experimental results for the inhibition of cellular AKT phosphorylation by antibodies.

FIG. 10: experimental results for the reduction of cell surface HGFR by antibodies.

FIG. 11: the inhibition of SNU-5 cell proliferation by antibodies.

FIG. 12: experimental results for the ADCC killing of Hs746T cells by antibodies.

FIG. 13: experimental results for the ADCC killing of H292 cells by antibodies.

FIG. 14: the inhibition of the growth of HCC827 mouse tumor cells by antibodies.

## DETAILED DESCRIPTION

### Terminology

In order to facilitate the understanding of the present disclosure, certain technical and scientific terms are specifically defined below. Unless otherwise explicitly defined herein, all other technical and scientific terms used herein have the meanings generally understood by those of ordinary skill in the art to which the present disclosure pertains. As used in the present disclosure, the singular forms "a", "an", and "the" include plural referents unless the context clearly dictates otherwise.

Unless otherwise clearly stated in the context, throughout the description and the claims, the words "comprise", "have", "include", and the like, should be construed in an inclusive sense as opposed to an exclusive or exhaustive sense.

"Optional" or "optionally" means that the event or circumstance subsequently described may, but does not necessarily, occur, and this description includes instances where the event or circumstance occurs or does not occur.

It should be understood by those skilled in the art that when used with reference to numerical ranges, cutoff values, or specific values, "about" may mean within 1 or more than 1 standard deviation. Alternatively, "about" may mean a range up to 20% apart (i.e., ±20%). Since many of the numerical values used herein are determined experimentally, those skilled in the art will appreciate that such determinations can and typically do vary from experiment to experiment. Because of this inherent difference, it is believed that the values used herein should not be unduly limited. Thus, the term "about" is used to encompass variations of ±20% or less, variations of ±10% or less, variations of ±5% or less, variations of ±1% or less, variations of ±0.5% or less, or variations of 0.1% or less from a specified value.

While the present disclosure provides content ranges or values, those of ordinary skill in the art will understand that the content ranges or values encompass acceptable margins of error for the specific value determined.

The three-letter and single-letter codes for amino acids used in the present disclosure are as described in J. Biol. Chem., 243, p3558 (1968).

"HGFR" should be understood in the broadest sense and is intended to encompass various forms of molecules of HGFR in various stages in mammals, such as, but not limited to, molecules produced by the HGFR gene during amplification, replication, transcription, splicing, processing, translation, and modification, e.g., precursor HGFR, mature HGFR, HGFR expressed on the membrane, HGFR splice variants, modified HGFR, or fragments thereof; the term also encompasses HGFR artificially prepared or expressed *in vitro*.

"EGFR" should be understood in the broadest sense and is intended to encompass various forms of molecules of EGFR in various stages in mammals, such as, but not limited to, molecules produced by the EGFR gene during amplification, replication, transcription, splicing, processing, translation, and modification, e.g., precursor EGFR, mature EGFR, EGFR expressed on the membrane, EGFR splice variants, modified EGFR, or fragments thereof; the term also encompasses EGFR artificially prepared or expressed *in vitro.*

The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a structure different from the general chemical structure of amino acids, but function in a manner similar to naturally occurring amino acids.

The term "antibody" is used in the broadest sense and encompasses a variety of antibody structures, including but not limited to monoclonal antibodies, polyclonal antibodies, monospecific antibodies, multispecific antibodies (e.g., bispecific antibodies), full-length antibodies, and antibody fragments (or antigen-binding fragments, or antigen-binding moieties), as long as they exhibit the desired antigen-binding activity. "Natural antibody" refers to a naturally occurring immunoglobulin molecule. For example, a natural IgG antibody is a heterotetrameric glycoprotein of about 150,000 Daltons composed of two light chains and two heavy chains linked by a disulfide bond. From the N-terminus to the C-terminus, each heavy chain comprises one variable region (VH), also known as variable heavy domain or heavy chain variable region, followed by three constant domains (CH1, CH2, and CH3). Similarly, from the N-terminus to the C-terminus, each light chain comprises one variable region (VL), also known as variable light domain or light chain variable domain, followed by one constant light domain (light chain constant region, CL).

The term "bispecific antibody" refers to an antibody (including an antibody or an antigen-binding fragment thereof, such as a single-chain antibody) capable of specifically binding to two different antigens or two different antigenic epitopes of the same antigen. Bispecific antibodies of various structures have been disclosed in the prior art. The bispecific antibodies can be classified into IgG-like bispecific antibodies and antibody-fragment-type bispecific antibodies according to the integrity of IgG molecules. The bispecific antibodies can be classified into bivalent, trivalent, tetravalent, or higher-valent bispecific antibodies according to the number of the antigen-binding regions. The bispecific antibodies can be classified into symmetric bispecific antibodies and asymmetric bispecific antibodies according to whether their structures are symmetric or asymmetric. Among them, the fragment-type bispecific antibodies, such as Fab fragments lacking Fc fragments, are formed by combining 2 or more Fab fragments into one molecule and have relatively low immunogenicity, small molecular weight, and relatively high tumor tissue permeability. Typical antibody structures of this type include F(ab')2, scFv-Fab, (scFv)2-Fab, and the like. The IgG-like bispecific antibodies (e.g., antibodies having Fc fragments) have large relative molecular weight. The Fc fragments facilitate the purification of the antibodies and increase their solubility and stability, and the Fc portions may further bind to the receptor FcRn and increase the serum half-life of the antibodies. Typical structural models of bispecific antibodies include bispecific antibodies such as KiH, CrossMAb, Triomab quadroma, FcΔAdp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP-DART, CODV-Fab-TL, HLE-BiTE, F(ab)2-CrossMAb, IgG-(scFv)2, Bs4Ab, DVD-Ig, Tetravalent-DART-Fc, (scFv)4-Fc, CODV-Ig, mAb2, and F(ab)4-CrossMAb (see Aran F. Labrijn et al., Nature Reviews Drug Discovery, volume 18, pages 585-608 (2019); Chen S1 et al., J Immunol Res., Feb. 11, 2019; 2019:4516041).

The term "variable region" or "variable domain" refers to a domain in an antigen-binding molecule that binds to an antigen. Herein, in the antigen-binding moiety 1 specifically binding to HGFR, the heavy chain variable region is denoted by M-VH, and the light chain variable region is denoted by M-VL; in the antigen-binding moiety 2 specifically binding to EGFR, the heavy chain variable region is denoted by E-VH, and the light chain variable region is denoted by E-VL. VH and VL each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDRs: HCDR1, HCDR2, and HCDR3; a VL comprises 3 CDRs: LCDR1, LCDR2, and LCDR3. Herein, the 3 CDRs in the M-VH are denoted by M-HCDR1, M-HCDR2, and M-HCDR3, respectively; the 3 CDRs in the M-VL are denoted by M-LCDR1, M-LCDR2, and M-LCDR3, respectively; the 3 CDRs in the E-VH are denoted by E-HCDR1, E-HCDR2, and E-HCDR3, respectively; the 3 CDRs in the E-VL are denoted by E-LCDR1, E-LCDR2, and E-LCDR3, respectively. Each VH and VL is composed of three CDRs and four FRs arranged from the amino terminus to the carboxyl terminus in the following order: FR1, CDR1, FR2, CDR2, FR3, CDR3, and FR4. A single VH or VL may be sufficient to provide antigen-binding specificity.

The amino acid sequence boundaries of the CDRs can be determined by a variety of well-known schemes, for example, the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains[J]. 2001), and the ImMunoGenTics (IMGT) numbering scheme (Lefranc, M.P. et al., Dev. Comp. Immunol., 27, 55-77 (2003); Front Immunol., Oct. 16, 2018; 9:2278), and the like. The corresponding relationships between the various numbering schemes are well known to those skilled in the art. The numbering schemes of the present disclosure are shown in Table 1 below.

Table 1. Relationships between CDR numbering schemes

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the variable region and CDR sequences in examples of the present disclosure. Although the Kabat numbering scheme is employed to define amino acid residues in specific embodiments, corresponding technical solutions for other numbering schemes are to be considered as equivalent technical solutions.

The term "antibody fragment" refers to a molecule different from an intact antibody, which comprises a moiety of an intact antibody that retains the antigen-binding ability of the intact antibody. Examples of antibody fragments include, but are not limited to, Fv, Fab, Fab', Fab'-SH, F(ab')$_2$, single-domain antibodies, single-chain Fab (scFab), diabodies, linear antibodies, single-chain antibody molecules (e.g., scFv), and multispecific antibodies formed from antibody fragments.

A Fab refers to a protein consisting of VH and CH1 (Fab heavy chain) and VL and CL (Fab light chain) of an immunoglobulin.

An Fv refers to an antigen-binding domain consisting of VH and VL of an immunoglobulin.

In some embodiments in the present disclosure, the first Fab has the structure of a Fab. In a substituted Fab, the CH1 and CL are substituted with a Titin chain or an Obscurin chain.

The term "Fc region" or "fragment crystallizable region" is used to define the C-terminal region of the heavy chain of an antibody, including native Fc regions and engineered Fc regions. In some embodiments, the Fc region comprises two identical or different subunits. In some embodiments, the Fc region of the human IgG heavy chain is defined as extending from the amino acid residue at position Cys226 or from Pro230 to its carboxyl terminus. Suitable native sequence Fc regions for the antibodies described herein include human IgG1, IgG2 (IgG2A, IgG2B), IgG3, and IgG4. Unless otherwise stated, the numbering scheme for the Fc region is the EU index.

The term "Titin chain" refers to a fragment of 78-118 amino acids in length comprising a peptide fragment of a Titin Ig-like 152 domain or a functional variant thereof in a Titin protein. The Titin chain is capable of forming a dimerized complex with the Obscurin chain.

The term "Obscurin chain" refers to a fragment of 87-117 amino acids in length comprising a peptide fragment of an Obscurin Ig-like 1 domain or a functional variant thereof in an Obscurin protein, or a fragment of 78-118 amino acids in length comprising a peptide fragment of an Obscurin-like Ig-like 1 domain or a functional variant thereof in an Obscurin-like 1 protein. The Obscurin chain is capable of binding to the Titin chain to form a dimerized complex. The Titin chain and the Obscurin chain of the present disclosure can be used to substitute the CH1 and CL in the Fab to form a substituted Fab (Fab-S). This substitution does not affect the binding of an antigen-binding molecule to an antigen.

The term "antigen-binding moiety" refers to a polypeptide molecule specifically binding to a target antigen. Antigen-binding moieties include the antibodies and the fragments thereof described herein. A specific antigen-binding moiety includes an antigen-binding domain of an antibody, which comprises an antibody heavy chain variable region and an antibody light chain variable region. The term "antigen-binding moiety specifically binding to HGFR" refers to a moiety that is capable of binding to HGFR or an epitope thereof with sufficient affinity. In certain examples, the antigen-binding moiety specifically binding to HGFR has an equilibrium dissociation constant (KD) as follows: < about 1 μM, < about 100 nM, or < about 10 nM or less, as determined by the Biacore method. In certain examples, the antigen-binding moiety specifically binding to HGFR binds to a conserved epitope in HGFR from different species. The term "antigen-binding moiety specifically binding to EGFR" refers to a moiety that is capable of binding to EGFR or an epitope thereof with sufficient affinity, such that a molecule comprising the moiety can be used as a diagnostic agent and/or therapeutic agent targeting EGFR. In certain examples, the antigen-binding moiety specifically binding to EGFR has an equilibrium dissociation constant (KD) as follows: < about 1 $\mu$M, < about 100 nM, or < about 10 nM or less, as determined by the Biacore method. In certain examples, the antigen-binding moiety specifically binding to EGFR binds to a conserved epitope in EGFR from different species. Antigen-binding moieties include antibody fragments as defined herein, e.g., a Fab, a substituted Fab, or an Fv.

In this context, ordinal numerals in "antigen-binding moiety 1", "antigen-binding moiety 2", "linker 1", and "linker 2" are used for the purpose of only distinguishing different technical features and chemical entities without limiting any order, level, and quantity.

The term "linker" refers to a linker unit that links two polypeptide fragments. Herein, the linkers present in the same structural formula or different structural formulas may be identical or different. The linker may be a peptide linker comprising one or more amino acids, typically about 1-30, 2-24, or 3-15 amino acids. The linkers used herein may be identical or different. When "-" appears in a structural formula, it means that the units on both sides are directly linked by a covalent bond. When the term "bond" appears in a structural unit, it means that the units on both sides of the unit are directly linked.

Unless otherwise stated, the "Kabat" numbering scheme is applied to the variable region and CDR sequences in examples of the present disclosure.

"Pharmaceutical composition" means comprising one or more bispecific antibodies described herein, as well as other components such as physiologically/pharmaceutically acceptable carriers and excipients. The purpose of the pharmaceutical composition is to promote administration to an organism and facilitate the absorption of the active ingredient, thereby exerting biological activity. In the present disclosure, "pharmaceutical composition" and "formulation" are not mutually exclusive.

"Effective amount" comprises an amount sufficient to ameliorate or prevent a symptom or sign of a medical disorder. An effective amount also means an amount sufficient to allow or facilitate diagnosis. The effective amount for a particular subject or veterinary subject may vary depending on factors such as the disorder to be treated, the general health of the subject, the method, route, and dosage of administration, and the severity of side effects. An effective amount may be the maximum dose or administration regimen to avoid significant side effects or toxic effects.

"Pharmaceutically acceptable carrier" or "pharmaceutically acceptable excipient" includes any material that, when combined with an active ingredient, allows the ingredient to retain biological activity and is non-reactive with the immune system of a subject. Examples include, but are not limited to, any standard pharmaceutical carrier, such as phosphate-buffered saline solutions, water, emulsions such as oil/water emulsions, and various types of wetting agents. In some examples, the diluent for aerosol or parenteral administration is phosphate-buffered saline (PBS) or normal (0.9%) saline. Compositions comprising such carriers are formulated by well-known conventional methods.

"Buffer" refers to a buffer that resists changes in pH by the action of its acid-base conjugate components. Examples of buffers that maintain the pH within an appropriate range include acetate, succinate, gluconate, histidine, oxalate, lactate, phosphate, citrate, tartrate, fumarate, glycylglycine, and other organic acid buffers.

"Histidine buffer" is a buffer comprising histidine. Examples of histidine buffers include histidine-histidine hydrochloride, histidine-histidine acetate, histidine-histidine phosphate, histidine-histidine sulfate, and the like. The histidine-histidine hydrochloride buffer is preferred. The histidine-histidine hydrochloride buffer may be prepared from histidine and hydrochloric acid, or from histidine and histidine hydrochloride.

"Citrate buffer" is a buffer comprising citrate ions. Examples of citrate buffers include citric acid-sodium citrate, citric acid-potassium citrate, citric acid-calcium citrate, citric acid-magnesium citrate, and the like. The preferred citrate buffer is citric acid-sodium citrate.

"Succinate buffer" is a buffer comprising succinate ions. Examples of succinate buffers include succinic acid-sodium succinate, succinic acid-potassium succinate, succinic acid-calcium succinate, and the like. The preferred succinate buffer is succinic acid-sodium succinate. Illustratively, the succinic acid-sodium succinate may be prepared from succinic acid and sodium hydroxide, or from succinic acid and sodium succinate.

"Phosphate buffer" is a buffer comprising phosphate ions. Examples of phosphate buffers include citric acid-disodium hydrogen phosphate, disodium hydrogen phosphate-sodium dihydrogen phosphate, disodium hydrogen phosphate-potassium dihydrogen phosphate, disodium hydrogen phosphate-citric acid, and the like. The preferred phosphate buffer is citric acid-disodium hydrogen phosphate.

"Acetate buffer" is a buffer comprising acetate ions. Examples of acetate buffers include acetic acid-sodium acetate, histidine-histidine acetate, acetic acid-potassium acetate, acetic acid-calcium acetate, acetic acid-magnesium acetate, and the like. The preferred acetate buffer is acetic acid-sodium acetate.

"Poloxamer", a block copolymer of ethylene oxide and propylene oxide, is water-soluble and used as a surfactant in pharmaceutical formulations. Examples of poloxamers include poloxamer 188 (P188).

"Freeze-dried formulation" refers to a formulation or a pharmaceutical composition obtained by lyophilizing a pharmaceutical composition or a formulation in liquid or solution form *in vacuo.* Generally, lyophilization includes pre-freezing, primary drying, and secondary drying. The purpose of pre-freezing is to freeze the product to obtain a crystalline solid; in some embodiments, the pre-freezing temperature is set at -45 °C, and the pre-freezing rate is set at 1 °C/min. Primary drying is the primary stage of lyophilizing a sample. The aim is to remove ice from the product while maintaining the shape of the product and minimizing damage to the product. If the temperature and the degree of vacuum are not properly selected during primary drying, it can lead to the collapse of the product. Higher temperatures and degrees of vacuum can both increase the lyophilization efficiency, but they also increase the risk of product collapse. In some embodiments, the temperature of the primary drying may be a temperature conventional in the art, for example, -30 °C-0 °C. Secondary drying, also known as desorption drying, is the main step of removing bound water from the product by creating an ultimate vacuum (0.01 mbar) and increasing the temperature (20 °C-40 °C). As most biological products are sensitive to temperature, the secondary drying temperature is often set at a lower point of a temperature range, e.g., 25 °C. The lyophilization time is related to the freezer, the dose of the freeze-dried formulation, and the container for the freeze-dried formulation. Such time adjustments are well known to those skilled in the art.

Unless otherwise stated, the solvent in the pharmaceutical composition described herein in solution form is water. The pharmaceutical composition described herein can achieve a stable effect, that is, the antibody in the pharmaceutical composition substantially retains its physical and/or chemical stability and/or biological activity after storage; preferably, the pharmaceutical composition substantially retains its physical and chemical stability as well as its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured.

"Exchange" refers to the exchange of a solvent system that solubilizes an antibody protein. For example, a high-salt or hypertonic solvent system comprising the antibody protein is exchanged, by physical operations, with a buffer system of a stable formulation, such that the antibody protein is present in the stable formulation. The physical operations include, but are not limited to, ultrafiltration, dialysis, or centrifugation.

A stable formulation is one in which no significant change is observed under the following conditions: stored at refrigeration temperature (2-8 °C) for at least 3 months, preferably 6 months, more preferably 1 year, and even more preferably up to 2 years. In addition, stable liquid formulations include liquid formulations that exhibit the desired characteristics after storage at temperatures including 25 °C for periods including 24 h, 7 days, 1 month, 3 months, or 6 months. They also include formulations that exhibit the desired characteristics after storage at a temperature of 40 °C for periods including 4 weeks, 1 month, 3 months, or 6 months. Typical examples of stability: Generally, no more than about 20% or 10%, preferably no more than about 5%, of antibodies aggregate or are degraded as measured by SEC-HPLC. The formulation is a pale yellow, nearly colorless and clear liquid, or a colorless and clear liquid, or is clear to slightly opalescent, by visual analysis. The concentration, pH, and osmolality of the formulation have changes of no more than ±10%, preferably changes of no more than ±5%. Generally, aggregation of no more than about 10%, preferably no more than about 5% is formed in the formulation.

An antibody "retains its physical stability" in a pharmaceutical formulation if it shows no significant increase in aggregation, precipitation, and/or denaturation upon visual inspection of color and/or clarity, or as determined by UV light scattering, size exclusion chromatography (SEC), and dynamic light scattering (DLS). Changes in protein conformation can be evaluated by fluorescence spectroscopy (which determines the protein tertiary structure) and by FTIR spectroscopy (which determines the protein secondary structure).

An antibody "retains its chemical stability" in a pharmaceutical formulation if it shows no significant chemical change. Chemical stability can be evaluated by detecting and quantifying chemically changed proteins. Degradation processes that often change the chemical structure of proteins include hydrolysis or clipping (evaluated by methods such as size exclusion chromatography and CE-SDS), oxidation (evaluated by methods such as peptide mapping in conjunction with mass spectroscopy or MALDI/TOF/MS), deamidation (evaluated by methods such as ion-exchange chromatography, capillary isoelectric focusing, peptide mapping, and isoaspartic acid measurement), and isomerization (evaluated by measuring the isoaspartic acid content, peptide mapping, etc.).

An antibody "retains its biological activity" in a pharmaceutical formulation if the biological activity of the antibody at a given time is within a predetermined range of the biological activity exhibited during the preparation of the pharmaceutical formulation. "Administrating", "giving", and "treating", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids. "Administering", "giving", and "treating" can refer to, for example, therapeutic, pharmacokinetic, diagnostic, research, and experimental methods. The treatment of cells comprises contacting a reagent with cells and contacting the reagent with a fluid, where the fluid is in contact with the cells. "Administering", "giving", and "treating" also refer to treating, e.g., cells by reagents, diagnosis, binding compositions or by another cell *in vitro* and *ex vivo*. "Treating", when applied to humans, veterinary, or research subjects, refers to therapeutic treatment, preventive or prophylactic measures, and research and diagnostic applications.

"Treatment" refers to administering, either internally or externally, a therapeutic agent, for example, comprising any one of the reconstituted solutions of the present disclosure, to a patient with one or more disease symptoms on which the therapeutic agent is known to have a therapeutic effect. Generally, the therapeutic agent is administered in an amount effective to alleviate one or more disease symptoms in the patient or population being treated to induce regression of such symptoms or to inhibit the development of such symptoms to any clinically measurable degree. The amount of the therapeutic agent effective to alleviate any specific disease symptom (also known as a "therapeutically effective amount") may vary depending on a variety of factors, such as the disease state, age and body weight of the patient, and the ability of the drug to produce a desired therapeutic effect in the patient. Whether a disease symptom has been alleviated can be evaluated by any clinical testing methods commonly used by doctors or other health care professionals to evaluate the severity or progression of the symptom. Although an embodiment of the present disclosure (for example, a treatment method or a product) may not be effective in alleviating every target disease symptom, it should alleviate the target disease symptom in a statistically significant number of patients as determined by any statistical testing methods known in the art, such as Student t-test, chi-square test, Mann and Whitney's U test, Kruskal-Wallis test (H test), Jonckheere-Terpstra test, and Wilcoxon test.

The reconstituted solution of the present disclosure may be administered by any suitable means, including parenteral, intrapulmonary, and intranasal administration, and if required for local treatment, intralesional administration. Parenteral infusion includes intramuscular, intravenous, intra-arterial, intraperitoneal, or subcutaneous administration. Administration may be performed by any suitable route, e.g., by injection, such as intravenous or subcutaneous injection. Various administration schedules are contemplated herein, including but not limited to, single administration or multiple administrations at multiple time points, bolus administration, and pulse infusion. In some embodiments, the reconstituted solution of the present disclosure is administered by subcutaneous injection.

The reconstituted solution of the present disclosure will be formulated, administered, and applied in a manner consistent with Good Medical Practice. Factors considered in this context include the specific disorder being treated, the specific mammal being treated, the clinical state of the individual patient, the cause of the disorder, the site of delivery of the agent, the method of administration, the timing of administration, and other factors known to medical practitioners. Optionally, the reconstituted solution may also be formulated together with one or more additional agents used for preventing or treating the disorder. The effective amount of such additional agents depends on the amount of the antigen-binding molecule present in the reconstituted solution, the type of the disorder or treatment, and other factors. Such additional agents may be used in the same doses and with the same routes of administration as described herein, or in about 1% to 99% of the doses described herein, or in any dose and by any route that is empirically/clinically determined to be appropriate. The details of one or more embodiments of the present disclosure are set forth in the specification above. Although any methods and materials similar or identical to those described herein can be used in the practice or testing of the present disclosure, the preferred methods and materials are described below. Other features, objects, and advantages of the present disclosure will be apparent from the specification and the claims. In the specification and claims, singular forms include plural referents unless otherwise indicated clearly in the context. Unless otherwise defined, all technical and scientific terms used herein have the meanings generally understood by those of ordinary skill in the art to which the present disclosure belongs. All the patents and publications cited in the specification are incorporated by reference. The following examples are set forth in order to more fully illustrate the preferred embodiments of the present disclosure. These examples should not be construed in any way as limiting the scope of the present disclosure, which is defined by the claims.

**Examples - Preparation and Assays of Bispecific Antibodies Specifically Binding to HGFR and EGFR**

PCT/CN2022/105714 (filing date: July 14, 2022; Priority Patent Application No.: CN202110794137.9) is incorporated herein by reference in its entirety.

**Example 1: Preparation of Antigen Proteins and Antibodies**

**1.1. Antigen protein structure**

With human EGFR protein (UniProt epidermal growth factor receptor, Uniprot number: P00533) used as a template of EGFR, a His tag was fused on the basis of the EGFR protein, and a human EGFR protein extracellular domain with the His tag (abbreviated as EGFR-His) for detection in the present invention was designed. The amino acid sequence was as follows:
The amino acid sequence of EGFR-His:

LEEKKVCQGTSNKLTQLGTFEDHFLSLQRMFNNCEVVLGNLEITYVQRNYDLSF
LKTIQEVAGYVLIALNTVERIPLENLQIIRGNMYYENSYALAVLSNYDANKTGLK
ELPMRNLQEILHGAVRFSNNPALCNVESIQWRDIVSSDFLSNMSMDFQNHLGSC
QKCDPSCPNGSCWGAGEENCQKLTKIICAQQCSGRCRGKSPSDCCHNQCAAGC
TGPRESDCLVCRKFRDEATCKDTCPPLMLYNPTTYQMDVNPEGKYSFGATCVK
KCPRNYVVTDHGSCVRACGADSYEMEEDGVRKCKKCEGPCRKVCNGIGIGEF
KDSLSINATNIKHFKNCTSISGDLHILPVAFRGDSFTHTPPLDPQELDILKTVKEIT
GFLLIQAWPENRTDLHAFENLEIIRGRTKQHGQFSLAVVSLNITSLGLRSLKEISD
GDVIISGNKNLCYANTINWKKLFGTSGQKTKIISNRGENSCKATGQVCHALCSPE
GCWGPEPRDCVSCRNVSRGRECVDKCNLLEGEPREFVENSECIQCHPECLPQA
MNITCTGRGPDNCIQCAHYIDGPHCVKTCPAGVMGENNTLVWKYADAGHVCH
LCHPNCTYGCTGPGLEGCPTNGPKIPS*HHHHHH*

<div align="right">SEQ ID NO: 42</div>

Note: The EGFR protein extracellular domain is underlined; the His tag is italicized.

With human HGFR protein (UniProt hepatocyte growth factor receptor, Uniprot number: P08581) used as a template of HGFR, a His or Fc tag was fused on the basis of the HGFR protein, and an HGFR protein extracellular domain with the His tag (abbreviated as HGFR-His) or an HGFR protein extracellular domain with the Fc tag (abbreviated as HGFR-Fc) for detection in the present invention was designed. The amino acid sequence was as follows:
The amino acid sequence of HGFR-His:

ECKEALAKSEMNVNMKYQLPNFTAETPIQNVILHEHHIFLGATNYIYVLNEEDL
QKVAEYKTGPVLEHPDCFPCQDCSSKANLSGGVWKDNINMALVVDTYYDDQL
ISCGSVNRGTCQRHVFPHNHTADIQSEVHCIFSPQIEEPSQCPDCVVSALGAKVL
SSVKDRFINFFVGNTINSSYFPDHPLHSISVRRLKETKDGFMFLTDQSYIDVLPEF
RDSYPIKYVHAFESNNFIYFLTVQRETLDAQTFHTRIIRFCSINSGLHSYMEMPLE
CILTEKRKKRSTKKEVFNILQAAYVSKPGAQLARQIGASLNDDILFGVFAQSKPD
SAEPMDRSAMCAFPIKYVNDFFNKIVNKNNVRCLQHFYGPNHEHCFNRTLLRN

SSGCEARRDEYRTEFTTALQRVDLFMGQFSEVLLTSISTFIKGDLTIANLGTSEGR
FMQVVVSRSGPSTPHVNFLLDSHPVSPEVIVEHTLNQNGYTLVITGKKITKIPLN
GLGCRHFQSCSQCLSAPPFVQCGWCHDKCVRSEECLSGTWTQQICLPAIYKVFP
NSAPLEGGTRLTICGWDFGFRRNNKFDLKKTRVLLGNESCTLTLSESTMNTLKC
TVGPAMNKHFNMSIIISNGHGTTQYSTFSYVDPVITSISPKYGPMAGGTLLTLTG
NYLNSGNSRHISIGGKTCTLKSVSNSILECYTPAQTISTEFAVKLKIDLANRETSIF
SYREDPIVYEIHPTKSFISGGSTITGVGKNLNSVSVPRMVINVHEAGRNFTVACQ
HRSNSEIICCTTPSLQQLNLQLPLKTKAFFMLDGILSKYFDLIYVHNPVFKPFEKP
VMISMGNENVLEIKGNDIDPEAVKGEVLKVGNKSCENIHLHSEAVLCTVPNDLL
KLNSELNIEWKQAISSTVLGKVIVQPDQNFT*HHHHHH*

SEQ ID NO: 43

Note: The HGFR protein extracellular domain is underlined; the His tag is italicized.

With human HGF protein (UniProt hepatocyte growth factor, Uniprot number: P14210) used as a template of HGF, an HGF protein with a His tag (abbreviated as HGF-His) for detection in the present disclosure was designed. The amino acid sequence was as follows:
The amino acid sequence of HGF-His:

QRKRRNTIHEFKKSAKTTLIKIDPALKIKTKKVNTADQCANRCTRNKGLPFTCK
AFVFDKARKQCLWFPFNSMSSGVKKEFGHEFDLYENKDYIRNCIIGKGRSYKG
TVSITKSGIKCQPWSSMIPHEHSFLPSSYRGKDLQENYCRNPRGEEGGPWCFTSN
PEVRYEVCDIPQCSEVECMTCNGESYRGLMDHTESGKICQRWDHQTPHRHKFL
PERYPDKGFDDNYCRNPDGQPRPWCYTLDPHTRWEYCAIKTCADNTMNDTDV
PLETTECIQGQGEGYRGTVNTIWNGIPCQRWDSQYPHEHDMTPENFKCKDLRE
NYCRNPDGSESPWCFTTDPNIRVGYCSQIPNCDMSHGQDCYRGNGKNYMGNL
SQTRSGLTCSMWDKNMEDLHRHIFWEPDASKLNENYCRNPDDDAHGPWCYT
GNPLIPWDYCPISRCEGDTTPTIVNLDHPVISCAKTKQLRVVNGIPTRTNIGWMV
SLRYRNKHICGGSLIKESWVLTARQCFPSRDLKDYEAWLGIHDVHGRGDEKCK
QVLNVSQLVYGPEGSDLVLMKLARPAVLDDFVSTIDLPNYGCTIPEKTSCSVYG
WGYTGLINYDGLLRVAHLYIMGNEKCSQHHRGKVTLNESEICAGAEKIGSGPC
EGDYGGPLVCEQHKMRMVLGVIVPGRGCAIPNRPGIFVRVAYYAKWIHKIILTY
KVPQS*HHHHHH*

SEQ ID NO: 44

Note: The HGF protein is underlined; the His tag is italicized.

**1.2. Purification of proteins**

1.2.1. Purification steps for recombinant proteins:

The cell expression supernatant sample was centrifuged at high speed to remove impurities, and the buffer was exchanged for PBS. Imidazole was added until the final concentration was 5 mM. A nickel column was equilibrated with a PBS solution containing 5 mM imidazole and rinsed with 2-5 column volumes. The cell supernatant sample after buffer exchange was loaded on the Ni Sepharose excel column (GE, 17-3712-02). The column was rinsed with a PBS solution containing 5 mM imidazole until the A280 reading dropped to the baseline. The chromatography column was then rinsed with PBS + 10 mM imidazole to remove non-specifically bound protein impurities, and the eluate was collected. The target protein was eluted with a PBS solution containing 300 mM imidazole, and the eluted peak was collected. The collected eluate was concentrated and further purified using a gel chromatography column Superdex200 (GE, 28-9893-35) with PBS as the mobile phase. The aggregate peaks were removed, and the eluted peak was collected. After it was confirmed by electrophoresis, peptide mapping, and LC-MS that the obtained protein was the desired protein, the protein was aliquoted for later use.

1.2.2. Purification steps for antibodies:

The cell expression supernatant sample was centrifuged at high speed to remove impurities, and the supernatant was purified by MabSelect Sure (GE, 17-5438-01) affinity chromatography. The MabSelect Sure chromatography column was firstly regenerated with 0.2 M NaOH, rinsed with purified water, and then equilibrated with PBS. After the supernatant was bound, the column was washed with PBS until the A280 reading dropped to the baseline. The target protein was eluted with a 0.1 M acetic acid buffer at pH 3.5 and neutralized with 1 M Tris-HCl. The eluted sample was appropriately concentrated and further purified using a gel chromatography column Superdex200 (GE, 28-9893-35) equilibrated with PBS, and the target protein was concentrated to an appropriate concentration in the receiver tube where it was collected. This method was used to purify the antibodies involved in the present invention.

**Example 2: Preparation and Identification of Bispecific Antibodies Specifically Binding to HGFR and EGFR**

Antibodies specifically binding to HGFR and EGFR were constructed using molecules specifically binding to HGFR and molecules specifically binding to EGFR.

**2.1. Molecules specifically binding to EGFR**

The molecules specifically binding to EGFR could be derived from any suitable antibody, such as zalutumumab (abbreviated as Zal) or a variant thereof (e.g., Zal.1, which was obtained by mutating the first amino acid residue of the Zal light chain from A to D).

> The amino acid sequence of the heavy chain of Zal:

QVQLVESGGGVVQPGRSLRLSCAASGFTFSTYGMHWVRQAPGKGLEWVAVIW
DDGSYKYYGDSVKGRFTISRDNSKNTLYLQMNSLRAEDTAVYYCARDGITMVR
GVMKDYFDYWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKRVEPKSCDKTHTCPPCPAPELLGGPSVFLFPPKPKDTLMISRTPEVTC

VVVDVSHEDPEVKFNWYVDGVEVHNAKTKPREEQYNSTYRVVSVLTVLHQD
WLNGKEYKCKVSNKALPAPIEKTISKAKGQPREPQVYTLPPSREEMTKNQVSLT
CLVKGFYPSDIAVEWESNGQPENNYKTTPPVLDSDGSFFLYSKLTVDKSRWQQG
NVFSCSVMHEALHNHYTQKSLSLSPGK

SEQ ID NO: 1

> The amino acid sequence of the light chain of Zal:

AIQLTQSPSSLSASVGDRVTITCRASQDISSALVWYQQKPGKAPKLLIYDASSLES
GVPSRFSGSESGTDFTLTISSLQPEDFATYYCQQFNSYPLTFGGGTKVEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC

SEQ ID NO: 2

> The amino acid sequence of the heavy chain variable region of Zal:

QVQLVESGGGVVQPGRSLRLSCAASGFTFSTYGMHWVRQAPGKGLEWVAVIW
DDGSYKYYGDSVKGRFTISRDNSKNTLYLQMNSLRAEDTAVYYCARDGITMVR
GVMKDYFDYWGQGTLVTVSS

SEQ ID NO: 3

> The amino acid sequence of the light chain variable region of Zal:

AIQLTQSPSSLSASVGDRVTITCRASQDISSALVWYQQKPGKAPKLLIYDASSLES
GVPSRFSGSESGTDFTLTISSLQPEDFATYYCQQFNSYPLTFGGGTKVEIK

SEQ ID NO: 4

> The amino acid sequence of the heavy chain variable region of Zal.1 is set forth in
SEQ ID NO: 3
> The amino acid sequence of the light chain variable region of Zal.1:

*D*IQLTQSPSSLSASVGDRVTITCRASQDISSALVWYQQKPGKAPKLLIYDASSLES
GVPSRFSGSESGTDFTLTISSLQPEDFATYYCQQFNSYPLTFGGGTKVEIK

SEQ ID NO: 5.

The CDR sequences of Zal and Zal.1 are shown in Table 2:

Table 2. The CDRs of the molecules specifically binding to EGFR

| Antibody | Heavy chain | | Light chain | |
|---|---|---|---|---|
| | HCDR1 | TYGMH (SEQ ID NO: 6) | LCDR1 | RASQDISSALV (SEQ ID NO: 9) |
| Zal.1, Zal | HCDR2 | VIWDDGSYKYYGDSVKG (SEQ ID NO: 7) | LCDR2 | DASSLES (SEQ ID NO: 10) |
| | HCDR3 | DGITMVRGVMKDYFDY (SEQ ID NO: 8) | LCDR3 | QQFNSYPLT (SEQ ID NO: 11) |
| Note: The CDRs described above are determined according to the Kabat numbering scheme. | | | | |

### 2.2. Molecules specifically binding to HGFR

The molecules specifically binding to HGFR could be derived from any suitable antibody, including antibodies such as onartuzumab (abbreviated as Omab) described in International Publication No. WO2013003680A1 (incorporated herein by reference in its entirety), antibodies such as Ab10 described in International Publication No. WO2016165580A1 (incorporated herein by reference in its entirety), and mutant antibodies of the antibodies described above. The amino acid sequences of the variable regions of Omab and Ab 10 were as follows:

> The amino acid sequence of the heavy chain variable region of Omab:

EVQLVESGGGLVQPGGSLRLSCAASGYTFT<u>SYWLH</u>WVRQAPGKGLEWVG<u>MID
PSNSDTRFNPNFKD</u>RFTISADTSKNTAYLQMNSLRAEDTAVYYCAT<u>YRSYVTPL
DY</u>WGQGTLVTVSS

SEQ ID NO: 12

> The amino acid sequence of the light chain variable region of Omab:

DIQMTQSPSSLSASVGDRVTITC<u>KSSQSLLYTSSQKNYLA</u>WYQQKPGKAPKLLIY
<u>WASTRES</u>GVPSRFSGSGSGTDFTLTISSLQPEDFATYYC<u>QQYYAYPWT</u>FGQGTKV
EIK

SEQ ID NO: 13

> The amino acid sequence of the heavy chain variable region of Ab10:

QVQLVESGGGVVQPGRSLRLSCAASGFSLS<u>NYGVH</u>WVRQAPGKGLEWLA<u>VIW
SGGSTNYAAAFVS</u>RLTISKDNSKNTVYLQMNSLRAEDTAVYYCAR<u>NHDNPYNY
AMDY</u>WGQGTTVTVSS

SEQ ID NO: 14

> The amino acid sequence of the light chain variable region of Ab10:

DIVLTQSPDSLAVSLGERATINC**RADKSVSTSTYNYLH**WYQQKPGQPPKLLIY**LA SNLAS**GVPDRFSGSGSGTDFTLTISSLQAEDVAVYYC**QHSRDLPPT**FGQGTKLEI K

SEQ ID NO: 15

The Ab10 mutant antibody Ab10.1 was obtained by mutating the CDRs of Ab10; the constant regions of Ab10.1 were identical to those of Ab10. The amino acid sequences of the variable regions of Ab10.1 were as follows:

> The amino acid sequence of the heavy chain variable region of Ab10.1:

QVQLVESGGGVVQPGRSLRLSCAASGFSLS*D*YGVHWVRQAPGKGLEWLA**VIW SGGSTNYA*D*AFVS**RLTISKDNSKNTVYLQMNSLRAEDTAVYYCAR**NHDNPY*I*Y AMDY**WGQGTTVTVSS

SEQ ID NO: 16

> The amino acid sequence of the light chain variable region of Ab10. 1:

DIVLTQSPDSLAVSLGERATINC**RADKSVSTSTYNYLH**WYQQKPGQPPKLLIY***RG S*F*LAT***GVPDRFSGSGSGTDFTLTISSLQAEDVAVYYC**QHSRDLPPT**FGQGTKLEI K

SEQ ID NO: 17

Note: In the sequences of the molecules specifically binding to HGFR described above, the CDR parts determined according to the Kabat numbering scheme are underlined, and the mutated amino acid residues are italicized and in bold.
The CDRs of the molecules specifically binding to HGFR are shown in Table 3:

Table 3. The CDRs of the molecules specifically binding to HGFR

| Antibody | Heavy chain | | Light chain | |
|---|---|---|---|---|
| Omab | HCDR1 | SYWLH (SEQ ID NO: 18) | LCDR1 | KSSQSLLYTSSQKNYLA (SEQ ID NO: 21) |
| | HCDR2 | MIDPSNSDTRFNPNFKD (SEQ ID NO: 19) | LCDR2 | WASTRES (SEQ ID NO: 22) |
| | HCDR3 | YRSYVTPLDY (SEQ ID NO: 20) | LCDR3 | QQYYAYPWT (SEQ ID NO: 23) |
| Ab10 | HCDR1 | NYGVH (SEQ ID NO: 24) | LCDR1 | RADKSVSTSTYNYLH (SEQ ID NO: 27) |
| | HCDR2 | VIWSGGSTNYAAAFVS (SEQ ID NO: 25) | LCDR2 | LASNLAS (SEQ ID NO: 28) |
| | HCDR3 | NHDNPYNYAMDY (SEQ ID NO: 26) | LCDR3 | QHSRDLPPT (SEQ ID NO: 29) |
| Ab10.1 | HCDR1 | DYGVH (SEQ ID NO: 30) | LCDR1 | RADKSVSTSTYNYLH (SEQ ID NO: 27) |
| | HCDR2 | VIWSGGSTNYADAFVS (SEQ ID NO: 31) | LCDR2 | RGSFLAT (SEQ ID NO: 33) |
| | HCDR3 | NHDNPYIYAMDY (SEQ ID NO: 32) | LCDR3 | QHSRDLPPT (SEQ ID NO: 29) |
| Note: The CDRs described above are determined according to the Kabat numbering scheme. | | | | |

**2.3. Bispecific antibodies specifically binding to HGFR and EGFR**

A bispecific antibody specifically binding to HGFR and EGFR and having a Format1 or Format2 structure was constructed using a molecule specifically binding to HGFR and a molecule specifically binding to EGFR. The structural schematic diagram of Format1 is shown in FIG. 1, and the structural schematic diagram of Format2 is shown in FIG. 2. Among them,

Format1 comprises 4 chains of the following structures, where:
chain 1 is:

VH (anti-EGFR antibody)-linker 1-VH (anti-HGFR antibody 1)-IgG$_1$ (CH1)-IgG$_1$Fc (knob);
chain 2 is: VL (anti-EGFR antibody)-linker 1-VL (anti-HGFR antibody 1)-CL;
chain 3 is: VH (anti-HGFR antibody 2)-linker 2-Titin chain-IgG$_1$Fc (hole);
chain 4 is: VL (anti-HGFR antibody 2)-linker 2-Obscurin chain.

Format2 comprises 4 chains of the following structures, where:

chain 1 is: VH (anti-HGFR antibody)-IgG$_1$ (CH1)-IgG$_1$Fc (knob);
chain 2 is: VL (anti-HGFR antibody)-CL;
chain 3 is: VH (anti-EGFR antibody)-linker 2-Titin chain-IgG$_1$Fc (hole);
chain 4 is: VL (anti-EGFR antibody)-linker 2-Obscurin chain.

Illustratively, EM1 having the Format1 structure and EM2 having the Format2 structure were constructed using Ab10.1 (anti-HGFR antibody 1), Omab (anti-HGFR antibody 2), Zal.1 (anti-EGFR antibody), human IgG$_1$ heavy chain constant region/kappa light chain constant region, and T.16 (Titin chain)/O.28 (Obscurin chain).

The amino acid sequences of the 4 chains of EM1 were as follows:

The amino acid sequence of chain 1 of EM1:

QVQLVESGGGVVQPGRSLRLSCAASGFTFSTYGMHWVRQAPGKGLEWVAVIW
DDGSYKYYGDSVKGRFTISRDNSKNTLYLQMNSLRAEDTAVYYCARDGITMVR
GVMKDYFDYWGQGTLVTVSSGGGGSGGGGGQVQLVESGGGVVQPGRSLRLSC
AASGFSLSDYGVHWVRQAPGKGLEWLAVIWSGGSTNYADAFVSRLTISKDNSK
NTVYLQMNSLRAEDTAVYYCARNHDNPYIYAMDYWGQGTTVTVSS*ASTKGPSV*
*FPLAPSSKSTSGGTAALGCLVKDYFPEPVTVSWNSGALTSGVHTFPAVLQSSGLYSLSS*
*VVTVPSSSLGTQTYICNVNHKPSNTKVDKKVEPKSC*DKTHTCPPCPAPELLGGPSVF
LFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQ
VYTLPPCREEMTKNQVSLWCLVKGFYPSDIAVEWESNGQPENNYKTTPPVLDS
DGSFFLYSKLTVDKSRWQQGNVFSCSVMHEALHNHYTQKSLSLSPGK

SEQ ID NO: 34

The amino acid sequence of chain 2 of EM1:

DIQLTQSPSSLSASVGDRVTITCRASQDISSALVWYQQKPGKAPKLLIYDASSLES
GVPSRFSGSESGTDFTLTISSLQPEDFATYYCQQFNSYPLTFGGGTKVEIKGGGGS
GGGGDIVLTQSPDSLAVSLGERATINCRADKSVSTSTYNYLHWYQQKPGQPPKL
LIYRGSFLATGVPDRFSGSGSGTDFTLTISSLQAEDVAVYYCQHSRDLPPTFGQGT
KLEIKRTVAAPSVFIFPPSDEQLKSGTASVVCLLNNFYPREAKVQWKVDNALQS
GNSQESVTEQDSKDSTYSLSSTLTLSKADYEKHKVYACEVTHQGLSSPVTKSFN
RGEC

SEQ ID NO: 35

The amino acid sequence of chain 3 of EM1:

EVQLVESGGGLVQPGGSLRLSCAASGYTFTSYWLHWVRQAPGKGLEWVGMID
PSNSDTRFNPNFKDRFTISADTSKNTAYLQMNSLRAEDTAVYYCATYRSYVTPL
DYWGQGTLVTVSSGGGGSGIPPKIECLPIDISIDEGKVLTVASAFTGEPTPEVTWS
TGGRKIHSQEQGRFHIENTDDSTTLTIKDVQKQDGGLYTLTLRNEFGSDSATVNI

HIRSIDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVCTLPPSREEMTKNQVSLSCAVKGFYPSDIA
VEWESNGQPENNYKTTPPVLDSDGSFFLVSKLTVDKSRWQQGNVFSCSVMHEA
LHNHYTQKSLSLSPGK

SEQ ID NO: 36

The amino acid sequence of chain 4 of EM1:

DIQMTQSPSSLSASVGDRVTITCKSSQSLLYTSSQKNYLAWYQQKPGKAPKLLIY
WASTRESGVPSRFSGSGSGTDFTLTISSLQPEDFATYYCQQYYAYPWTFGQGTKV
EIKGGGGSSGAPRFLTRPKASVVSVGKDATLSCQIVGNPFPQVSWEKDKQPVTA
GVRFRLAQDGDLYRLKILDLQLSDSGQYVCRARNAHGEAFACLGLQVDAEA

SEQ ID NO: 37

The amino acid sequences of the 4 chains of EM2 were as follows:

The amino acid sequence of chain 1 of EM2:

EVQLVESGGGLVQPGGSLRLSCAASGYTFTSYWLHWVRQAPGKGLEWVGMID
PSNSDTRFNPNFKDRFTISADTSKNTAYLQMNSLRAEDTAVYYCATYRSYVTPL
DYWGQGTLVTVSS*ASTKGPSVFPLAPSSKSTSGGTAALGCLVKDYFPEPVTVSWNS*
*GALTSGVHTFPAVLQSSGLYSLSSVVTVPSSSLGTQTYICNVNHKPSNTKVDKKVEPKS*
*C*DKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSN
KALPAPIEKTISKAKGQPREPQVYTLPPCREEMTKNQVSLWCLVKGFYPSDIAVE
WESNGQPENNYKTTPPVLDSDGSFFLYSKLTVDKSRWQQGNVFSCSVMHEALH
NHYTQKSLSLSPGK

SEQ ID NO: 38

The amino acid sequence of chain 2 of EM2:

DIQMTQSPSSLSASVGDRVTITCKSSQSLLYTSSQKNYLAWYQQKPGKAPKLLIY
WASTRESGVPSRFSGSGSGTDFTLTISSLQPEDFATYYCQQYYAYPWTFGQGTKV
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC

SEQ ID NO: 39

The amino acid sequence of chain 3 of EM2:

QVQLVESGGGVVQPGRSLRLSCAASGFTFSTYGMHWVRQAPGKGLEWVAVIW
DDGSYKYYGDSVKGRFTISRDNSKNTLYLQMNSLRAEDTAVYYCARDGITMVR
GVMKDYFDYWGQGTLVTVSSGGGGSGIPPKIECLPIDISIDEGKVLTVASAFTGE
PTPEVTWSTGGRKIHSQEQGRFHIENTDDSTTLTIKDVQKDGGLYTLTLRNEFG
SDSATVNIHIRSIDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLN
GKEYKCKVSNKALPAPIEKTISKAKGQPREPQVCTLPPSREEMTKNQVSLSCAV

KGFYPSDIAVEWESNGQPENNYKTTPPVLDSDGSFFLVSKLTVDKSRWQQGNVF
SCSVMHEALHNHYTQKSLSLSPGK

SEQ ID NO: 40

The amino acid sequence of chain 4 of EM2:

DIQLTQSPSSLSASVGDRVTITCRASQDISSALVWYQQKPGKAPKLLIYDASSLES
GVPSRFSGSESGTDFTLTISSLQPEDFATYYCQQFNSYPLTFGGGTKVEIKGGGGS
SGAPRFLTRPKASVVSVGKDATLSCQIVGNPFPQVSWEKDKQPVTAGVRFRLAQ
DGDLYRLKILDLQLSDSGQYVCRARNAHGEAFACLGLQVDAEA

SEQ ID NO: 41

Note: In the sequences described above, the variable regions are single-underlined, the CL regions are dashed-underlined, the Fc regions are dotted-underlined, the CH1 regions are italicized, the linkers are double-underlined, and the Obscurin chain/Titin-T chain is underlined with a wavy line.

The amino acid sequence of linker 1: GGGGSGGGG (SEQ ID NO: 45)
The amino acid sequence of linker 2: GGGGS (SEQ ID NO: 46)
The amino acid sequence of IgG1Fc (knob):

DKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVYTLPPCREEMTKNQVSLWCLVKGFYPSDIAVEW
ESNGQPENNYKTTPPVLDSDGSFFLYSKLTVDKSRWQQGNVFSCSVMHEALHN
HYTQKSLSLSPGK

(SEQ ID NO: 47)

The amino acid sequence of IgG$_1$Fc (hole):

DKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVCTLPPSREEMTKNQVSLSCAVKGFYPSDIAVEW
ESNGQPENNYKTTPPVLDSDGSFFLVSKLTVDKSRWQQGNVFSCSVMHEALHN
HYTQKSLSLSPGK

(SEQ ID NO: 48)

CL:

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

(SEQ ID NO: 49)

T.16 (Titin chain)

GIPPKIECLPIDISIDEGKVLTVASAFTGEPTPEVTWSTGGRKIHSQEQGRFHIENT
DDSTTLTIKDVQKQDGGLYTLTLRNEFGSDSATVNIHIRSI

(SEQ ID NO: 50)

O.28 (Obscurin chain)

SGAPRFLTRPKASVVSVGKDATLSCQIVGNPFPQVSWEKDKQPVTAGVRFRLAQ

DGDLYRLKILDLQLSDSGQYVCRARNAHGEAFACLGLQVDAEA

(SEQ ID NO: 51).

**Test Examples**

**Test Example 1: *In Vitro* Binding Affinity Biacore Assay and Kinetics Experiment**

The affinities of the test molecules for human EGFR (SEQ ID NO: 42) or human HGFR protein (SEQ ID NO: 43) were determined by Biacore T200 (GE) as follows:
Antibody molecules were affinity-captured on a Protein A biosensor chip, and the antigen was then allowed to flow through the surface of the chip for 180 s, followed by 600 s of dissociation. Reaction signals were detected in real time using a Biacore T200 instrument to obtain association and dissociation curves. After the dissociation in each experimental cycle was complete, the biosensor chip was washed with 10 mM Gly-HCl (pH 1.5) for regeneration. Data were fitted using a 1:1 model, and the affinity values of the test molecules were obtained. In this example, the positive control MCLA was a bispecific antibody specifically binding to HGFR and EGFR (see PB8532 in WO2019031965A1 for specific sequences).

The experimental results are shown in Table 4. The experimental results show that the mutant antibodies and bispecific antibodies constructed in the present disclosure all exhibited very strong affinities, and the affinity of Ab10.1 was at least 4 times higher than that of the parent antibody Ab10.

Table 4. The *in vitro* affinities of the antibodies of the present invention for human EGFR or human HGFR

| Antibody | Antigen | ka (1/Ms) | kd (1/s) | KD (M) |
|---|---|---|---|---|
| EM1 | EGFR-His | 1.42E+05 | 1.03E-03 | 7.29E-09 |
| | HGFR-His | 5.48E+04 | 1.24E-04 | 2.26E-09 |
| EM2 | EGFR-His | 1.16E+05 | 9.48E-04 | 8.17E-09 |
| | HGFR-His | 5.05E+04 | 1.53E-04 | 3.04E-09 |
| MCLA | EGFR-His | 6.85E+04 | 4.73E-03 | 6.91E-08 |
| | HGFR-His | 2.95E+04 | 3.03E-04 | 1.03E-08 |
| Zal | EGFR-His | 7.16E+04 | 1.44E-03 | 2.01E-08 |
| Ab10.1 | HGFR-His | 4.08E+04 | 3.09E-04 | 7.56E-09 |
| Ab10 | HGFR-His | 2.64E+04 | 8.70E-04 | 3.30E-08 |

**Test Example 2: *In Vitro* Cell Binding Assay of Antibodies**

The cell binding activity of bispecific antibodies specifically binding to HGFR and EGFR was assessed using a flow cytometer. An EGFR CHO-S stably transfected cell strain, an HGFR CHO-S stably transfected cell strain, and an H1975-HGF or MKN-45 tumor cell strain, at $1 \times 10^6$ cells/mL, were blocked with 1% BSA PBS buffer, and diluted antibody samples at different concentrations (C25, an IgG1 antibody protein of an irrelevant target, was used as a negative control; this applied hereinafter) were then added. The cells were incubated for 1 h. After two washes, R-PE-goat anti-human (H + L) antibody (Invitrogen, CAT#H10104) was added, and the cells were incubated for 0.5 h. After two washes, fluorescence signal values were read using a flow cytometer.

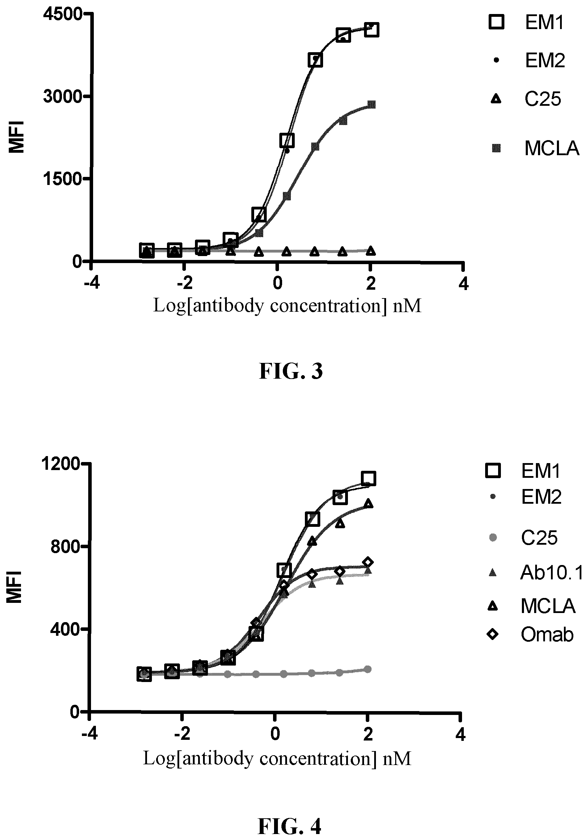

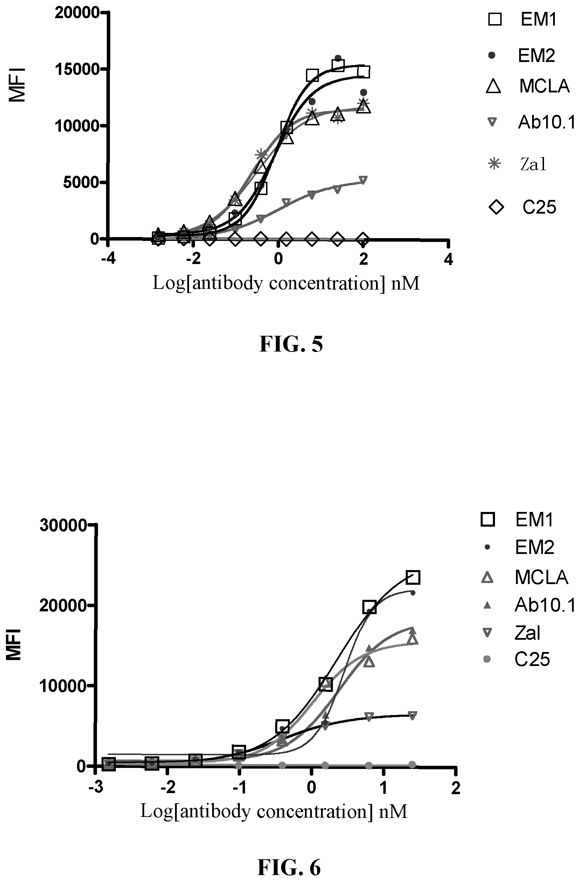

The experimental results are shown in FIGs. 3 to 6. The experimental results show that the bispecific antibodies specifically binding to HGFR and EGFR of the present disclosure exhibited stronger abilities to bind to tumor cells than the positive antibody MCLA.

**Test Example 3: Blocking Experiment of Antibodies Antagonizing Binding of EGFR to EGF**

The abilities of the antibodies to block EGF/EGFR were assessed by ELISA. A plate was coated overnight at 4 °C with 1 µg/mL rabbit anti-His antibody (GenScript, A01857) and then blocked. After the plate was washed, 2 µg/mL EGFR-His (SEQ ID NO: 42) was added, and the plate was incubated for 1 h. After the plate was washed, 2 µg/mL EGF-mFc (Acro, EGF-H525b) and diluted antibodies at different concentrations were added, and the plate was incubated for 1 h. After the plate was washed, horseradish peroxidase-goat anti-mouse IgG antibody (Jackson, 115-035-062) was added, and the plate was incubated for 1 h. After the plate was washed, a tetramethylbenzidine solution was added for color development. Finally, a stop solution was added. OD450 values were measured on a microplate reader. In this example, the positive control JNJ was a bispecific antibody specifically binding to HGFR and EGFR (JNJ is also referred to as amivantamab; see WHO Drug Information, 33 (2):237-239 for specific sequences). The experimental results are shown in Table 5. The experimental results show that the bispecific antibodies specifically binding to HGFR and EGFR of the present disclosure effectively blocked the binding of EGF to EGFR.

Table 5. Experimental results for the bispecific antigen-binding molecules blocking the binding of EGF to EGFR

| Antibody | IC$_{50}$ (µg/mL) | Max (%) |
|---|---|---|
| EM1 | 2.37 | 93.5 |
| EM2 | 2.60 | 94.5 |

(continued)

| Antibody | IC$_{50}$ ($\mu$g/mL) | Max (%) |
|----------|------------------------|---------|
| JNJ | 3.66 | 90.5 |

## Test Example 4: Blocking Experiment of Antibodies Antagonizing Binding of HGFR to HGF

The abilities of the antibodies to block HGF/HGFR were assessed by ELISA. A plate was coated overnight at 4 °C with 5 $\mu$g/mL HGFR-His (for the sequence, see SEQ ID NO: 43) and then blocked. After the plate was washed, 2 $\mu$g/mL bio-HGF-His (for the sequence, see SEQ ID NO: 44) and diluted antibodies at different concentrations were added, and the plate was incubated for 1 h. After the plate was washed, horseradish peroxidase-streptavidin (Jackson, 016-030-084) was added, and the plate was incubated for 1 h. After the plate was washed, a tetramethylbenzidine solution was added for color development. Finally, a stop solution was added. OD450 values were measured on a microplate reader.

The experimental results are shown in Table 6. The experimental results show that the bispecific antibodies specifically binding to HGFR and EGFR of the present disclosure blocked the binding of HGFR to HGF with a greater maximum blocking capacity than the positive antibody JNJ.

Table 6. Experimental results for the bispecific antigen-binding molecules blocking the binding of HGF to HGFR

| Antibody | IC50 ($\mu$g/mL) | Max (%) |
|----------|-------------------|---------|
| EM1 | 0.25 | 95.5 |
| JNJ | 0.27 | 82.3 |
| MCLA | 1.36 | 94.1 |

## Test Example 5: Inhibition of Cellular EGFR Phosphorylation by Antibodies

H292 cells (also known as NCI-H292, human lung cancer cells (lymph node metastases) from Cell Bank of Chinese Academy of Sciences; this applied hereinafter) were plated in a 96-well plate (Corning, 3599) at 12,000 cells/well, with RPMI1640 + 10% FBS as the medium, and the 96-well plate was then incubated in a 37 °C, 5% $CO_2$ incubator for 24 h. The next day, the medium was discarded, and a serum-free medium (RPMI640 + 25 mM HEPES + 0.1 mM NEAA + 1 mM sodium pyruvate) was used to starve cells for 16-20 h. On the third day, antibodies were formulated at a starting concentration of 6 $\mu$M and serially diluted 5-fold, and the starvation medium was replaced. The cells were incubated in a 37 °C, 5% $CO_2$ incubator for 1 h, during which 50 $\mu$L of 100 ng/mL rEGF (R&D, 236-EG-200) was added. The cells were incubated in the 37 °C, 5% $CO_2$ incubator for another 15 min. Finally, phosphorylated EGFR was detected according to the instructions of PHOSPHO-EGFR (TYR1068) KITS (Cisbio, 64EG1PEG).

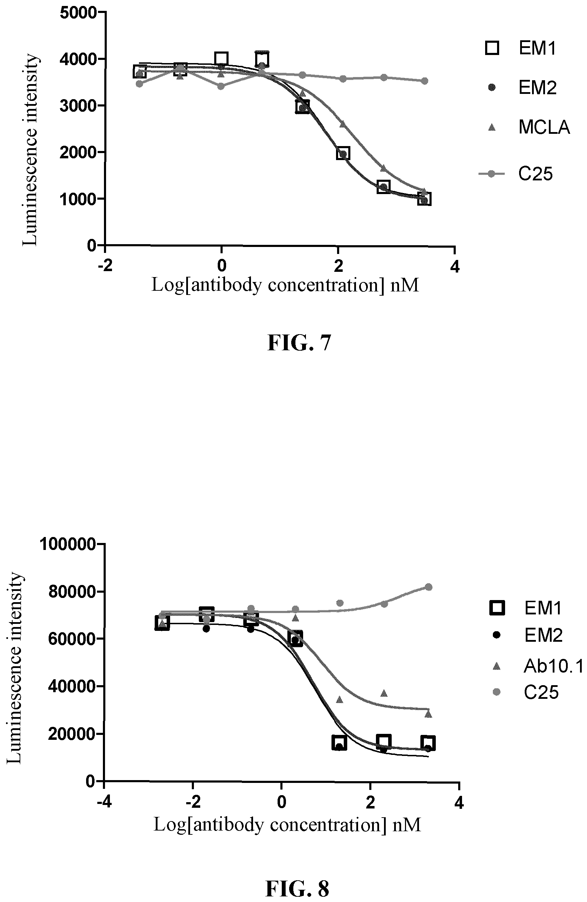

The experimental results are shown in FIG. 7. The experimental results show that the bispecific antibodies specifically binding to HGFR and EGFR of the present disclosure effectively inhibited cellular EGFR phosphorylation.

## Test Example 6: Inhibition of Cellular HGFR Phosphorylation by Antibodies

H292 cells were plated in a 96-well plate (Corning, 3599) at 12,000 cells/well, with RPMI1640 + 10% FBS as the medium, and the 96-well plate was then incubated in a 37 °C, 5% $CO_2$ incubator for 24 h. The next day, the medium was discarded, and a serum-free medium (RPMI640 + 25 mM HEPES + 0.1 mM NEAA + 1 mM sodium pyruvate) was used to starve cells for 16-20 h. On the third day, antibodies were formulated at a starting concentration of 1 $\mu$M and serially diluted 10-fold, and the starvation medium was replaced. The cells were incubated in a 37 °C, 5% $CO_2$ incubator for 1 h, during which 50 $\mu$L of 200 ng/mL rHGF (R&D, 294-HG-005) was added. The cells were incubated in the 37 °C, 5% $CO_2$ incubator for another 30 min. Finally, phosphorylated HGFR was detected according to the instructions of p-c-Met (Tyr1234/1235) Assay Kit (PerkinElmer, ALSU-PCMET-A50).

The experimental results are shown in FIG. 8. The experimental results show that the bispecific antibodies specifically binding to HGFR and EGFR of the present disclosure effectively inhibited cellular HGFR phosphorylation, and their inhibitory abilities were stronger than that of Ab10.1.

## Test Example 7: Inhibition of Cellular AKT Phosphorylation by Antibodies

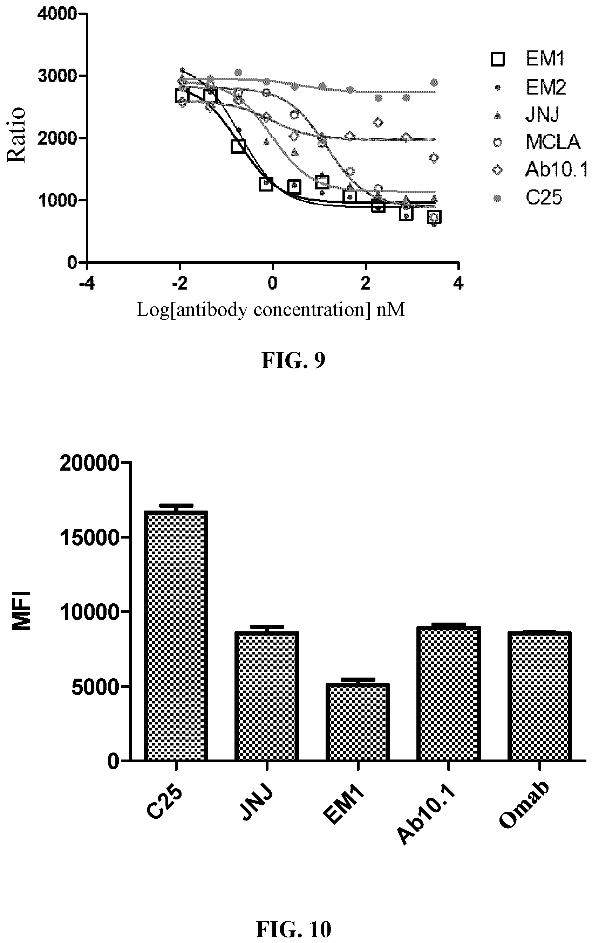

H292 cells were plated in a 96-well plate (Corning, 3599) at 12,000 cells/well, with RPMI1640 + 10% FBS as the medium, and the 96-well plate was then incubated in a 37 °C, 5% $CO_2$ incubator for 24 h. The next day, the medium was discarded, and a serum-free medium (RPMI640 + 25 mM HEPES + 0.1 mM NEAA + 1 mM sodium pyruvate) was used to starve cells for 16-20 h. On the third day, antibodies were formulated at a starting concentration of 4 $\mu$M and serially diluted 5-fold, and the starvation medium was replaced. The cells were incubated in a 37 °C, 5% $CO_2$ incubator for 1 h, during which 50 $\mu$L of 200 ng/mL rHGF (R&D, 294-HG-005) and 50 ng/mL rEGF (R&D, 236-EG-200) were added and well mixed. The cells were incubated in the 37 °C, 5% $CO_2$ incubator for another hour. Finally, phosphorylated AKT was detected according to the instructions of PHOSPHO-AKT (SER473) KITS (Cisbio, 64AKSPEG). The intracellular AKT phosphorylation level is reflected by the ratio of the fluorescence signal value at 665 nm to the fluorescence signal value at 620 nm, i.e., the ratio = the fluorescence signal value at 665 nm/the fluorescence signal value at 620 nm $\times 10^4$. The higher the ratio, the higher the intracellular AKT phosphorylation level. The experimental results are shown in FIG. 9. The experimental results show that the bispecific antibodies specifically binding to HGFR and EGFR of the present disclosure effectively inhibited cellular AKT phosphorylation, and their inhibitory abilities were stronger than that of the positive control antibody.

**Test Example 8: Reduction of Cell Surface HGFR Expression by Antibodies**

HCC827 cells (human non-small cell lung cancer cells, from ATCC) were plated in a 6-well plate at $3 \times 10^5$ cells/well, with RPMI1640 + 10% FBS as the medium, and the 6-well plate was then incubated in a 37 °C, 5% $CO_2$ incubator for 24 h. The antibodies were formulated to a concentration of 200 nM, and the cells were treated and reacted with the antibodies for 18 h. After the treatment, the cells were washed once with PBS, and 500 $\mu$L of enzyme-free cell dissociation buffer (Gibco, #13151) was added to collect the cells. The cells were isolated from the enzyme-free cell dissociation buffer by centrifugation. Immunofluorescence staining was performed using a goat anti-human HGFR antibody (R&D systems, AF276) at 4 °C for 1 h. Subsequently, the cells were washed twice with PBS containing 2% FBS. ALEXA488-donkey anti-sheep secondary antibody (Thermo Fisher, A-11055) was then added, and the reaction was performed at 4 °C for 1 h. After two washes, the cells were fixed with BD Cytofix (BD, #554655). After the cells were washed with PBS once again, fluorescence signal values were read using a flow cytometer.

The experimental results are shown in FIG. 10. The experimental results show that the bispecific antibodies specifically binding to HGFR and EGFR of the present disclosure more effectively reduced cell surface HGFR than the positive control antibody, thereby inhibiting receptor signaling pathways.

**Test Example 9: Inhibition of SNU-5 Cell Proliferation by Antibodies**

SNU-5 cells (human gastric cancer cells, from ATCC) were plated in a 96-well plate (Corning, 3903) at 4000 cells/well, with IMDM + 5% FBS as the medium, and the 96-well plate was then incubated in a 37 °C, 5% $CO_2$ incubator for 24 h. Antibodies were formulated to a concentration of 5 $\mu$M, serially diluted 5-fold, and added to the above 96-well plate. The cells were then incubated in the 37 °C, 5% $CO_2$ incubator for 5 days. Finally, CellTiter-Glo® Luminescent Cell Viability Assay (Promega, G7573) was formulated according to the instructions. 50 $\mu$L of CTG was added to each well, and the cells were incubated at room temperature for 10 min. After the plate was sealed with a sealing membrane, the luminescence intensity (abbreviated as Lum) was measured using a multi-label microplate detection system (PerkinElmer, victor3).

The calculation formulas were as follows: Lum = measurement - medium reading; %inhibition = (Lum0 - Lum)/Lum0 $\times$ 100%, where Lum0 represents the luminescence intensity of the well with an antibody concentration of 0, and Lum represents the luminescence intensity of the antibody wells with the various dilution concentrations.

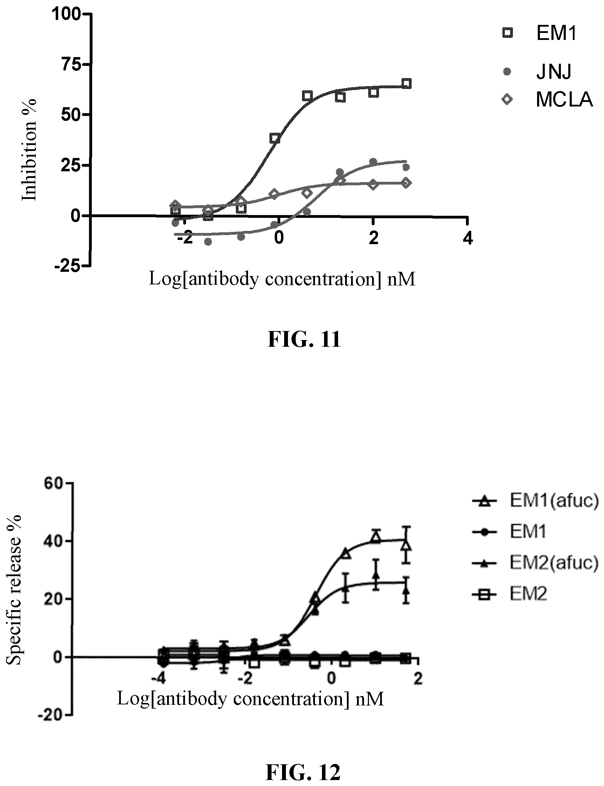

The experimental results are shown in FIG. 11. The experimental results show that the bispecific antibody specifically binding to HGFR and EGFR of the present disclosure exhibited stronger abilities to inhibit SNU-5 cell proliferation than the positive antibody.

**Test Example 10: ADCC Killing of Tumor Cells by Antibodies**

Antibodies were formulated to a concentration of 200 nM and serially diluted 5-fold. Hs746T cells (human gastric cancer cells, Nanjing Cobioer Biosciences Co., Ltd.) and H292 cells, were collected and washed once with PBS, and the cell density was then adjusted to $1 \times 10^6$ cells/mL with a buffer (PBS + 10% FBS + 20 mM HEPES). Then 3 $\mu$L of BATDA reagent (PerkinElmer, AD0116) was added to 2 mL of cells, and the cells were incubated in a 37 °C, 5% $CO_2$ incubator for 15 min. After centrifugation, the cells were washed three times with a rinsing buffer (PBS + 20 mM HEPES) and counted, and the cell density was adjusted to $1 \times 10^5$ cells/mL. NK92--FCGR3A (176V) cells (NK92 cells stably transformed with FCGR3A (176V), purchased from Nanjing Cobioer Biosciences Co., Ltd.) were collected, washed once with PBS, then resuspended in a medium, and counted, and the cell density was adjusted to $5 \times 10^5$ cells/mL. Finally, 100 $\mu$L of NK92--FCGR3A (176V) cells, 50 $\mu$L of diluted antibody, and 50 $\mu$L of labeled tumor cells were added to a 96-well plate (corning, 3788). Meanwhile, a spontaneous control release well that did not contain NK92--FCGR3A (176V) cells but contained 100 $\mu$L of medium was set; a positive control well that contained 10 $\mu$L of lysis buffer + 50 $\mu$L of labeled tumor cells + 140 $\mu$L of

medium was set; a background control well that contained 50 μL of labeled cell supernatant + 150 μL of medium was set. The 96-well plate was then centrifuged at 300 rpm for 2 min and incubated in a 37 °C, 5% $CO_2$ incubator for 2 h. 100 μL of supernatant was transferred into a new 96-well plate (corning, 3788). After the plate was centrifuged at 300 g for 5 min, 20 μL of supernatant was transferred into a detection plate (PerkinElmer, AD0116), and 200 μL of europium solution (PerkinElmer, AD0116) was then added. The plate was incubated with shaking at room temperature for 15 min, and TRF was finally detected. EM1 (afuc) was defucosylated EM1, and EM2 (afuc) was defucosylated EM2.

The calculation formula was as follows: specific release% = (experimental release - spontaneous release)/(-maximum release - spontaneous release) × 100.

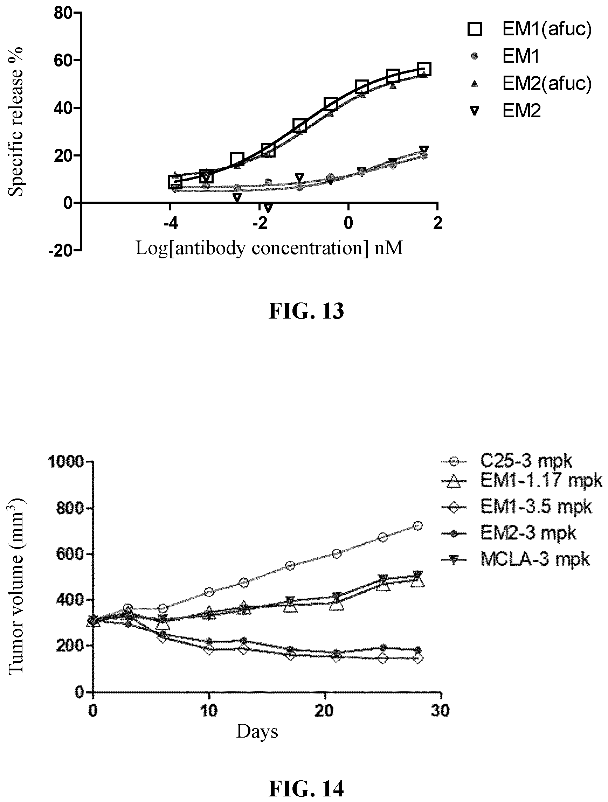

The experimental results are shown in FIGs. 12 and 13. The experimental results show that the bispecific antibodies specifically binding to HGFR and EGFR of the present disclosure exhibited significantly improved ADCC killing abilities against cells after defucosylation.

**Test Example 11: *In Vivo* Pharmacodynamic Assay of Antibodies in Mouse H1975-HGF Model**

H1975-HGF cells (H1975 cells (human lung adenocarcinoma cells, ATCC) stably transformed with human HGF) were inoculated subcutaneously into the right flank of CD1 nude female mice (Vital River) at $5 \times 10^6$ cells.

When the tumor size was 189 $mm^3$, the animals were randomized into groups of 10 according to tumor size, including a C25-3mpk group (a negative control, which was an IgG1 antibody protein of an irrelevant target, administered at a dose of 3 mg/kg), a JNJ-3mpk group (a positive control, administered at a dose of 3 mg/kg), and an EM1-3.5mpk group (an EM1 test group, administered at a dose of 3.5 mg/kg). Starting from the day when the animals were grouped (day 0), administration was performed by intraperitoneal injection twice a week for 2 to 3 consecutive weeks. The tumor volume was measured twice a week, the mice were weighed, and data were recorded. The data were statistically analyzed by T-test. C25-3mpk, JNJ-3mpk, and EM1-3.5mpk were equimolar.

The calculation formula for the tumor volume (T) was as follows: $T = 1/2 \text{ long} \times (\text{short})^2$;

$$\text{relative tumor proliferation rate } T/C\% = (T - T0)/(C - C0) \times 100\%;$$

$$\text{tumor growth inhibition rate } TGI\% = 1 - T/C\%;$$

where T and T0 represent tumor volumes of the administration group, and T0 represents the tumor volume at the beginning of the experiment; C and C0 represent tumor volumes of the negative control group, and C0 represents the tumor volume of the negative control group at the beginning of the experiment.

The experimental results are shown in Table 7. The experimental results show that the bispecific antibody specifically binding to HGFR and EGFR of the present disclosure significantly inhibited tumor cell growth, and on day 21, the mean tumor volume of the equimolar group of the bispecific antibody specifically binding to HGFR and EGFR of the present disclosure was less than half of that of the positive control, indicating that its tumor-inhibiting efficacy is stronger than that of the positive control antibody JNJ.

Table 7. The *in vivo* pharmacodynamic assay of the antibodies in the mouse H1975-HGF model

| Group | Mean tumor volume D0 | Mean tumor volume D21 | Tumor growth inhibition rate (%) D21 |
|---|---|---|---|
| C25-3mpk | 189.46 | 2355.30 | - |
| JNJ-3mpk | 190.07 | 551.37 | 83.32% |
| EM1-3.5mpk | 189.63 | 261.10 | 96.70% |

**Test Example 12: *In Vivo* Pharmacodynamic Assay of Antibodies in Mouse HCC827 Model**

HCC827 cells (human non-small cell lung cancer cells, from ATCC) ($8.555 \times 10^6$ cells) were inoculated subcutaneously into the right flank of NUNU female mice (Vital River).

When the tumor size was 200 $mm^3$, the animals were randomized into groups of 10 according to tumor size, including a C25-3mpk group (a negative control; C25 was an IgG1 antibody protein of an irrelevant target and administered at a dose of 3 mg/kg), an MCLA-3mpk group (a positive control, administered at a dose of 3 mg/kg), an EM1-3.5mpk group (an EM1 high-dose test group, administered at a dose of 3.5 mg/kg), an EM1-1.17mpk group (an EM1 low-dose test group, administered at a dose of 1.17 mg/kg), and an EM2-3mpk group (an EM2 test group, administered at a dose of 3 mg/kg). Starting from the day when the animals were grouped (day 0), administration was performed by intraperitoneal injection twice a week for 2 to 3 consecutive weeks. The tumor volume was measured twice a week, the mice were weighed, and data were recorded. C25-3mpk, MCLA-3mpk, EM1-3.5mpk, and EM2-3mpk were equimolar. The experimental results are shown in FIG. 14. The experimental results show that the bispecific antibodies specifically binding to HGFR and EGFR of the present disclosure significantly inhibited tumor cell growth, and their tumor-inhibiting effects were stronger than that of the positive control antibody.

**Preparation Example - Formulations of Bispecific Antibody Specifically Binding to HGFR and EGFR**

**SEC molecular exclusion chromatography:**

This is an analytical method that separates a solute based on the relative relationship between the pore size of the gel pores and the coil size of the polymer sample molecule. SEC% (SEC monomer content percentage) = A monomer/A total × 100% (A monomer represents the peak area of the main peak monomer in the sample, and A total represents the sum of all peak areas). ∆SEC% = SEC% of formulation after stability experiment - SEC% of formulation before stability experiment.

Instrument for SEC analysis: Agilent HPLC 1260;
Column: Waters, BioResolveTM SEC mAb 200 Å, 2.5 μm, 7.8 × 300 mm.

**NR-CE capillary gel electrophoresis:**

This is a type of electrophoresis in which a gel is moved into a capillary as a support medium and a method that achieves separation under a certain voltage based on the molecular weight of the sample.

$$\text{NR-CE\%} = \text{A main peak/A total} \times 100\%$$

(A main peak represents the peak area of the main peak in the sample, and A total represents the sum of all peak areas).
∆NR-CE% = NR-CE% of formulation after stability experiment - NR-CE% of formulation before stability experiment.
Instrument for CE analysis: Beckman capillary electrophoresis system; model: PA800 plus.

**IEC ion exchange chromatography:**

This is a chromatographic method that uses an ion exchange resin or chemically bonded ion exchanger as the stationary phase and achieves separation based on differences in ion exchange ability or selectivity coefficient among the components to be separated.
IEC% = A neutral peak area/A total area × 100% (A total area represents the sum of the areas of the acidic, neutral, and basic peaks). ∆IEC% = IEC% of formulation after stability experiment - IEC% of formulation before stability experiment.
Instrument for IEC analysis: Agilent HPLC 1260.

**Osmotic pressure determination:**

The freezing point method is used for measuring osmotic pressure. On the basis of the directly proportional relationship between the freezing point depression value and the molar concentration of a solution, the freezing point of the solution is determined using a highly sensitive temperature-sensing element and then converted into the osmotic pressure through electric quantity.
Instrument for osmotic pressure determination: Loser; model: OM815.

**Protein**

The protein used in the following examples was the HGFR-EGFR bispecific antibody EM1 (hereinafter referred to as "protein").
Instrument for protein concentration determination: ultraviolet-visible spectrophotometer; model: Nano Drop oneC; optical path length: 1 mm.

**Formulation Example 1: Screening of Different Ionic Strengths**

Formulations containing 100 mg/mL protein, 80 mg/mL sucrose, and 0.4 mg/mL polysorbate 80 (PS80) were prepared using the buffer systems shown in Table 8. Samples were subjected to a forced degradation study (standing at 40 °C for 4 weeks), and the effects of the different buffer systems on protein stability were evaluated based on SEC and NR-CE.

The results are shown in Table 8. The SEC data show no significant difference between the two. The NR-CE data show that the formulation group with an ionic strength of 20 mM was superior to the formulation group with an ionic strength of 50 mM.

Table 8. The results of the screening of different ionic strengths

| Group | Buffer system | Standing conditions | ΔSEC% | ΔNR-CE% |
|---|---|---|---|---|
| 1 | 20 mM His-HCl pH 5.5 | 40 °C W4 | -14.3 | -12.5 |
| 2 | 50 mM His-HCl pH 5.5 | 40 °C W4 | -13.6 | -16.6 |
| Note: His-HCl represents histidine-histidine hydrochloride; 40 °C W4: standing at 40 °C for 4 weeks; these apply hereinafter. | | | | |

**Formulation Example 2: pH and Buffer System Screening**

Formulations containing 100 mg/mL protein, 80 mg/mL sucrose, and 0.4 mg/mL polysorbate 80 (PS80) were prepared using the buffer systems shown in Table 9. Samples were subjected to a forced degradation study (standing at 40 °C for 4 weeks), and the effects of the different buffer systems on protein stability were evaluated based on appearance, SEC, and NR-CE.

The results are shown in Table 9. The appearance data show that the formulations containing His-HCl and His-AA were superior to the other groups in terms of appearance. The SEC and NR-CE data show that the formulations containing His-HCl (pH 6.0 or pH 6.5) were superior to the other groups in terms of monomer purity after standing at 40 °C for 4 weeks.

Table 9. The results of the pH and buffer system screening

| Group | Buffer system | Standing conditions | Appearance | ΔSEC % | ΔNR-CE % |
|---|---|---|---|---|---|
| 1 | 20 mM AA pH 5.0 | 40 °C W4 | Particles | -13.4 | -14.3 |
| 2 | 20 mM AA pH 5.5 | 40 °C W4 | Particles | -13.6 | -10.5 |
| 3 | 20 mM His-AA pH 5.5 | 40 °C W4 | Clear and transparent | -13.2 | -11.4 |
| 4 | 20 mM His-HCl pH 5.5 | 40 °C W4 | Opalescent | -14.3 | -12.5 |
| 5 | 20 mM His-HCl pH 6.0 | 40 °C W4 | Opalescent | -13.0 | -9.1 |
| 6 | 20 mM His-HCl pH 6.5 | 40 °C W4 | Opalescent | -13.5 | -8.8 |
| 7 | 20 mM SA pH 5.5 | 40 °C W4 | Significantly opalescent | -13.3 | -12.1 |
| 8 | 20 mM CA pH 5.5 | 40 °C W4 | Particles | -13.1 | -12.3 |
| 9 | 20 mM PB pH 6.5 | 40 °C W4 | Particles | -14.3 | -7.6 |
| Note: AA represents acetic acid-sodium acetate; His-AA represents histidine-histidine acetate; CA represents citric acid-sodium citrate; SA represents succinic acid-sodium succinate; PB represents disodium hydrogen phosphate-sodium dihydrogen phosphate; these apply hereinafter. | | | | | |

**Formulation Example 3: Sugar Type Screening**

Formulations containing 100 mg/mL protein, the different types of sugars shown in Table 10, and 0.6 mg/mL PS80 were prepared using 20 mM His-HCl buffer (pH 6.0). Samples were subjected to a forced degradation study (standing at 40 °C for 4 weeks), and the effects of the different sugar types on protein stability were evaluated based on appearance, SEC, and NR-CE.

The results are shown in Table 10. The data on appearance and purity show that the formulation group containing sucrose was superior to the formulation group containing trehalose after standing at 40 °C for 4 weeks.

Table 10. The results of the sugar type screening

| Group | Sugar | Standing conditions | Appearance | ΔSEC% | ΔNR-CE% |
|---|---|---|---|---|---|
| 1 | 75 mg/mL sucrose | 40 °C W4 | Clear and transparent | -16.2 | -6.0 |
| 2 | 75 mg/mL trehalose | 40 °C W4 | A small number of particles | -16.4 | -8.8 |

**Formulation Example 4: Surfactant Screening**

Formulations containing 100 mg/mL protein, 75 mg/mL sucrose, and the surfactants shown in Table 11 were prepared using 20 mM His-HCl buffer (pH 6.0). Samples were subjected to a forced degradation study (shaking at 300 rpm and 25 °C for 7 days; standing at 40 °C for 4 weeks), and the effects of the different surfactants on protein stability were evaluated based on SEC and NR-CE.

The results are shown in Table 11. The appearance data show that the formulation group containing 0.4 mg/mL PS80 was inferior to the other formulation groups in terms of appearance after 7 days of shaking and that the formulation group containing 0.6 mg/mL poloxamer 188 (P188) was inferior to the other formulation groups in terms of appearance after standing at 40 °C for 4 weeks. The NR-CE data show that after standing at 40 °C for 4 weeks, the formulation group containing 0.6 mg/mL poloxamer 188 (P188) was inferior to the other formulation groups, the formulation group containing 0.4 mg/mL PS80 was slightly superior, and the formulation group containing 0.6 mg/mL PS80 did not significantly differ from the formulation group containing 0.8 mg/mL PS80.

Table 11. The results of the surfactant screening

| Group | Surfactant | Standing conditions | Appearance | ΔSEC % | ΔNR-CE % |
|---|---|---|---|---|---|
| 1 | 0.4 mg/mL PS80 | Shaking D7 | A small number of particles | -1.9 | -0.3 |
| 1 | 0.4 mg/mL PS80 | 40 °C W4 | Clear and transparent | -16.1 | -4.5 |
| 2 | 0.6 mg/mL PS80 | Shaking D7 | Clear and transparent | -2.1 | 0.0 |
| 2 | 0.6 mg/mL PS80 | 40 °C W4 | Clear and transparent | -16.2 | -6.0 |
| 3 | 0.8 mg/mL PS80 | Shaking D7 | Clear and transparent | -2.0 | -0.1 |
| 3 | 0.8 mg/mL PS80 | 40 °C W4 | Clear and transparent | -16.6 | -6.4 |
| 4 | 0.6 mg/mL poloxamer 188 (P188) | Shaking D7 | Clear and transparent | -2.2 | 1.0 |
| 4 | 0.6 mg/mL poloxamer 188 (P188) | 40 °C W4 | A small number of particles | -16.4 | -9.8 |
| Note: Shaking D7: shaking for 7 days; 40 °C W4: standing at 40 °C for 4 weeks. | | | | | |

**Formulation Example 5: Lyophilization**

The protein samples shown in Table 12 were prepared and lyophilized. The lyophilization parameters are shown in Table 13:

Table 12. Freeze-dried formulation formula designs for the protein

| Lyophilization mode | Buffer system | Protein concentration mg/mL | Sucrose concentration mg/mL | PS80 concentration mg/mL | Filling volume mL | Reconstitution volume mL |
|---|---|---|---|---|---|---|
| High-concentration direct lyophilization | 20 mM His-HCl pH 6.0 | 100 | 70 | 0.6 | 3.5 | 3 |
| Dilution-freezing-concentration-dissolution | 10 mM His-HCl pH 6.0 | 50 | 37.5 | 0.3 | 7.0 | 3 |

Table 13. Protein lyophilization process development lyophilization parameters

| Lyophilization process parameters | Set temperature | Set time (min) | Holding time (min) | Degree of vacuum (Pa) |
|---|---|---|---|---|
| Pre-freezing | 5 °C | 10 | 60 | N/A |
| | -45 °C | 40 | 120 | N/A |
| Primary drying | -10 °C | 120 | 3000 | 10 |
| Secondary drying | 25 °C | 60 | 1 | 10 |
| | 25 °C | 1 | 600 | 1 |
| *: The primary drying time and the secondary drying time were adjusted according to specific conditions. | | | | |

Both the freeze-dried products were intact powder cakes with no significant collapse, and the powder cakes did not significantly differ from each other in appearance.

**Formulation Example 6: Lyophilization Stability 1**

Formulations containing 37.5 mg/mL sucrose, 0.3 mg/mL PS80, and 50 mg/mL protein sample were prepared using 10 mM His-HCl (pH 6.0), and lyophilization process screening was performed using 20-mL vials and 20-mm lyophilization rubber stoppers. The lyophilization parameters are shown in Table 14. The stability of the final process samples before and after lyophilization, as well as under high-temperature conditions, are shown in Table 15.

Table 14. Protein lyophilization process development lyophilization parameters

| Lyophilization process parameters | Set temperature | Set time (min) | Holding time (min) | Degree of vacuum (Pa) |
|---|---|---|---|---|
| Pre-freezing | 5 °C | 10 | 60 | N/A |
| | -45 °C | 40 | 120 | N/A |
| Primary drying | -5 °C | 120 | 3000 | 10 |
| Secondary drying | 25 °C | 60 | 1 | 10 |
| | 25 °C | 1 | 600 | 1 |
| *: The primary drying time and the secondary drying time were adjusted according to specific conditions. | | | | |

Table 15. Stability data of products after lyophilization

| Time | Appearance | ΔSEC% | ΔNR-CE% | ΔIEC% |
|---|---|---|---|---|
| Before lyophilization | Clear and transparent | N/A | N/A | N/A |
| After lyophilization | White cake with no collapse, clear and transparent upon reconstitution | -0.1 | -0.6 | -0.5 |
| 40 °C 4W | White cake with no collapse, clear and transparent upon reconstitution | -0.8 | 0.1 | -1.8 |

The results show that: before lyophilization, the formulation was clear and transparent; after lyophilization, the powder cake was intact with no significant collapse; the reconstituted solution was clear and transparent. The high-temperature (40 °C) results show that under conditions of 40 °C 4W, no significant changes were observed in SEC, NR-CE, and IEC, indicating relatively good stability.

**Formulation Example 7: Relationship Between Viscosity and pH**

Protein formulations prepared using 20 mM His-HCl (pH 5.0) and 20 mM His-HCl (pH 6.0) were concentrated to different concentrations, and their viscosities were measured at 25 °C. The results are shown in Table 16. In the His-HCl system, the viscosity at pH 5.0 was lower than that at pH 6.0.

**Table 16. Relationship between viscosity and pH**

| Protein concentration mg/mL | His-HCl pH 5.0 25 °C viscosity (cp) | His-HCl pH 6.0 25 °C viscosity (cp) |
|---|---|---|
| 100 | 4.7 | 4.3 |
| 120 | 6.9 | 7.7 |
| 150 | 14.2 | 22.0 |
| 180 | 35.9 | 64.7 |
| 200 | 84.8 | N/A |
| **Note:** N/A indicates that the viscosity of the sample was too high, so it could not be prepared. | | |

**Formulation Examples 8: Lyophilization Stability 2 (Subcutaneous Formulations)**

The protein samples shown in Table 17 were prepared and lyophilized. The lyophilization parameters are shown in Table 14:

Table 17. Freeze-dried formulation formula designs for the protein

| No. | Lyophilization mode | Buffer system | Protein concentration mg/mL | Sucrose concentration mg/mL | Arginine hydrochloride concentration/mM | PS80 concentration mg/mL |
|---|---|---|---|---|---|---|
| 1 | Dilution-freez-ing-concentra-tion-dissolution | 10 mM His-HCl pH 6.0 | 60 | 37.5 | 0 | 0.3 |
| 2 | | 10 mM | 75 | | 0 | |
| 3 | | His-HCl pH 5.0 | 100 | | 100 | |

Table 18. Protein reconstitution formulas

| No. | Buffer system | Protein concentration after reconstitution mg/mL | Sucrose concentration mg/mL | Arginine hydrochloride concentration/mM | PS80 concentration mg/mL |
|---|---|---|---|---|---|
| 1 | 20 mM His-HCl pH 6.0 | 120 | 75 | 0 | 0.6 |
| 2 | 20 mM His-HCl pH 5.0 | 150 | | 0 | |
| 3 | | 200 | | 200 | |

Table 19. Stability data of products after lyophilization

| No. | Time | Appearance | ΔSEC% | ΔNR-CE % | ΔIEC% |
|---|---|---|---|---|---|
| 1 | Before lyophilization | Clear and transparent | N/A | N/A | N/A |
| | After lyophilization | White cake with no collapse, clear and transparent upon reconstitution | -0.2 | 0.1 | -1.1 |
| | After reconstitution, 25 °C 24 h | Clear and transparent | -0.2 | -0.8 | -1.5 |
| 2 | Before lyophilization | Clear and transparent | N/A | N/A | N/A |
| | After lyophilization | White cake with no collapse, clear and transparent upon reconstitution | -0.4 | -0.5 | -0.7 |
| | After reconstitution, 25 °C 24 h | Clear and transparent | -0.4 | -0.9 | -1.2 |

(continued)

| No. | Time | Appearance | ΔSEC% | ΔNR-CE % | ΔIEC% |
|---|---|---|---|---|---|
| 3 | Before lyophilization | Clear and transparent | N/A | N/A | N/A |
| | After lyophilization | White cake with no collapse, clear and transparent upon reconstitution | -0.5 | 0.3 | -0.8 |
| | After reconstitution, 25 °C 24 h | Clear and transparent | -1.1 | 0.0 | -1.6 |

The results show that: before lyophilization, the formulations were clear and transparent; after lyophilization, the powder cakes were intact with no significant collapse; the reconstituted solutions were clear and transparent. The "after reconstitution, 25 °C 24 h" results show no significant changes in SEC, NR-CE, and IEC, indicating relatively good stability.

**Formulation Examples 9: Lyophilization Stability 3 (Subcutaneous Formulations)**

The protein samples shown in Table 20 were prepared and lyophilized. The lyophilization parameters are shown in Table 14:

Table 20. Freeze-dried formulation formula designs for the protein

| No. | Lyophilization mode | Buffer system | Protein concentration mg/mL | Sucrose concentration mg/mL | Methionine concentration mM | Hyaluronidase concentration U/mL | PS80 concentration mg/mL |
|---|---|---|---|---|---|---|---|
| 1 | Dilution-freezing-concentration-dissolution | 10 mM His-HCl pH 5.0 | 75 | 37.5 | 5 | 1000 | 0.3 |
| 2 | | 10 mM His-HCl pH 6.0 | 60 | | | | |

Table 21. Protein reconstitution formulas

| No. | Buffer system | Protein concentration after reconstitution mg/mL | Sucrose concentration mg/mL | Methionine concentration mM | Hyaluronidase concentration U/mL | PS80 concentration mg/mL |
|---|---|---|---|---|---|---|
| 1 | 20 mM His-HCl pH 5.0 | 150 | 75 | 10 | 2000 | 0.6 |
| 2 | 20 mM His-HCl pH 6.0 | 120 | | | | |

Table 22. Stability data of products after lyophilization

| No. | Time | Appearance | ΔSEC% | ΔEnzyme activity change% |
|---|---|---|---|---|
| 1 | Before lyophilization | Clear and transparent | N/A | N/A |
| | After lyophilization | White cake with no collapse, clear and transparent upon reconstitution | 0.7 | N/A |
| | 40 °C 4W | White cake with no collapse, clear and transparent upon reconstitution | -4.3 | -2.1 |

(continued)

| No. | Time | Appearance | ΔSEC% | ΔEnzyme activity change% |
|---|---|---|---|---|
| 2 | Before lyophilization | Clear and transparent | N/A | N/A |
| | After lyophilization | White cake with no collapse, clear and transparent upon reconstitution | 2.3 | N/A |
| | 40 °C 4W | White cake with no collapse, clear and transparent upon reconstitution | -4.6 | 5.3 |

The results show that: before lyophilization, the formulations were clear and transparent; after lyophilization, the powder cakes were intact with no significant collapse; the reconstituted solutions were clear and transparent. The high-temperature results show that under conditions of 40 °C 4W, no significant changes were observed in SEC and enzyme activity, indicating relatively good stability.

**Formulation Examples 10: Lyophilization Stability 4 (Subcutaneous Formulations)**

The protein samples shown in Table 23 were prepared and lyophilized. The lyophilization parameters are shown in Table 14:

Table 23. Freeze-dried formulation formula designs for the protein

| No. | Lyophilization mode | Buffer system | Protein concentration mg/mL | Sucrose concentration mg/mL | Methionine concentration mM | Hyaluronidase concentration U/mL | PS80 concentration mg/mL |
|---|---|---|---|---|---|---|---|
| 1 | Dilution-freezing-concentration-dissolution | 10 mM His-HCl pH 6.0 | 60 | 37.5 | 0 | 1000 | 0.3 |
| 2 | | | | | 5 | | |

Table 24. Protein reconstitution formulas

| No. | Buffer system | Protein concentration after reconstitution mg/mL | Sucrose concentration mg/mL | Methionine concentration mM | Hyaluronidase concentration U/mL | PS80 concentration mg/mL |
|---|---|---|---|---|---|---|
| 1 | 20 mM His-HCl pH 6.0 | 120 | 75 | 0 | 2000 | 0.6 |
| 2 | | | | 10 | | |

Table 25. Stability data of products after lyophilization

| No. | Time | Appearance | ΔSEC% | ΔNR-CE % | ΔIEC% | ΔEnzyme activity change% |
|---|---|---|---|---|---|---|
| 1 | Before lyophilization | Clear and transparent | N/A | N/A | N/A | N/A |
| | After lyophilization | White cake with no collapse, clear and transparent upon reconstitution | -0.9 | 0.0 | -1.2 | N/A |
| | 40 °C 4W | White cake with no collapse, clear and transparent upon reconstitution | -1.2 | -1.4 | -3.3 | 1.7 |
| | 2-8 °CM6 | White cake with no collapse, clear and transparent upon reconstitution | 0.6 | 0.0 | 2.7 | N/A |
| | 25 °CM6 | White cake with no collapse, clear and transparent upon reconstitution | -0.6 | -0.9 | -0.3 | N/A |
| 2 | Before lyophilization | Clear and transparent | N/A | N/A | N/A | N/A |
| | After lyophilization | White cake with no collapse, clear and transparent upon reconstitution | -0.8 | 0.1 | -0.4 | N/A |
| | 40 °C 4W | White cake with no collapse, | -1.4 | -1.7 | -2.6 | 3.1 |
| | | clear and transparent upon reconstitution | | | | |
| | 2-8 °CM6 | White cake with no collapse, clear and transparent upon reconstitution | 1.0 | 0.0 | 2.2 | N/A |
| | 25 °CM6 | White cake with no collapse, clear and transparent upon reconstitution | -0.2 | -1.1 | -0.5 | N/A |

Note: 40 °C 4W indicates standing at 40 °C for 4 weeks under these conditions, 2-8 °C M6 indicates standing at 4 °C for 6 months under these conditions, and 25 °C M6 indicates standing at 25 °C for 6 months under these conditions.

The results show that: before lyophilization, the formulations were clear and transparent; after lyophilization, the powder cakes were intact with no significant collapse; the reconstituted solutions were clear and transparent. The 40 °C 4W, 25 °C M6, and 2-8 °C M6 results show no significant changes in purity and enzyme activity, indicating relatively good stability.

**Formulation Example 11: Final Formula Lyophilization 5 (Subcutaneous Formulation)**

The protein sample shown in Table 26 was prepared and lyophilized. The lyophilization parameters are shown in Table 14:

Table 26. Freeze-dried formulation formula design for the protein

| No. | Lyophilization mode | Buffer system | Protein concentration mg/mL | Sucrose concentration mg/mL | Methionine concentration mM | Hyaluronidase concentration U/mL | PS80 concentration mg/mL |
|---|---|---|---|---|---|---|---|
| 1 | Dilution-freezing-concentration-dissolution | 10 mM His-HCl pH 5.0 | 60 | 28 | 4 | 800 | 0.24 |

The lyophilization results show that the freeze-dried product was an intact powder cake with no significant collapse. After reconstitution, the formula contained 150 mg/mL protein, 25 mM His-HCl (pH 5.0), 70 mg/mL sucrose, 10 mM methionine, 0.6 mg/mL PS80, and 2000 U/mL hyaluronidase, with a clear and transparent appearance.

**Claims**

1. A pharmaceutical composition, comprising a bispecific antibody specifically binding to HGFR and EGFR and a buffer, wherein:

   the bispecific antibody specifically binding to HGFR and EGFR comprises at least one antigen-binding moiety 1 specifically binding to HGFR and at least one antigen-binding moiety 2 specifically binding to EGFR;
   the antigen-binding moiety 1 comprises a heavy chain variable region M-VH and a light chain variable region M-VL, wherein the M-VH comprises an M-HCDR1, an M-HCDR2, and an M-HCDR3, and the M-VL comprises an M-LCDR1, an M-LCDR2, and an M-LCDR3; and
   the antigen-binding moiety 2 comprises a heavy chain variable region E-VH and a light chain variable region E-VL, wherein the E-VH comprises an E-HCDR1, an E-HCDR2, and an E-HCDR3, and the E-VL comprises an E-LCDR1, an E-LCDR2, and an E-LCDR3;
   the E-HCDR1, the E-HCDR2, the E-HCDR3, the E-LCDR1, the E-LCDR2, and the E-LCDR3 are defined according to the Kabat numbering scheme, wherein the E-HCDR1 is set forth in SEQ ID NO: 6, the E-HCDR2 is set forth in SEQ ID NO: 7, the E-HCDR3 is set forth in SEQ ID NO: 8, the E-LCDR1 is set forth in SEQ ID NO: 9, the E-LCDR2 is set forth in SEQ ID NO: 10, and the E-LCDR3 is set forth in SEQ ID NO: 11; and
   the M-HCDR1, the M-HCDR2, the M-HCDR3, the M-LCDR1, the M-LCDR2, and the M-LCDR3 are defined according to the Kabat numbering scheme, wherein

      (i) the M-HCDR1 is set forth in SEQ ID NO: 30, the M-HCDR2 is set forth in SEQ ID NO: 31, the M-HCDR3 is set forth in SEQ ID NO: 32, the M-LCDR1 is set forth in SEQ ID NO: 27, the M-LCDR2 is set forth in SEQ ID NO: 33, and the M-LCDR3 is set forth in SEQ ID NO: 29; or
      (ii) the M-HCDR1 is set forth in SEQ ID NO: 18, the M-HCDR2 is set forth in SEQ ID NO: 19, the M-HCDR3 is set forth in SEQ ID NO: 20, the M-LCDR1 is set forth in SEQ ID NO: 21, the M-LCDR2 is set forth in SEQ ID NO: 22, and the M-LCDR3 is set forth in SEQ ID NO: 23; or
      (iii) the M-HCDR1 is set forth in SEQ ID NO: 24, the M-HCDR2 is set forth in SEQ ID NO: 25, the M-HCDR3 is set forth in SEQ ID NO: 26, the M-LCDR1 is set forth in SEQ ID NO: 27, the M-LCDR2 is set forth in SEQ ID NO: 28, and the M-LCDR3 is set forth in SEQ ID NO: 29;

   the buffer is a histidine buffer, an acetate buffer, a citrate buffer, a succinate buffer, or a phosphate buffer;
   preferably, the buffer is a histidine-histidine hydrochloride buffer or a histidine-histidine acetate buffer.

2. The pharmaceutical composition according to claim 1, wherein the pharmaceutical composition has a pH of 4.5 to 6.5;

   preferably, the pharmaceutical composition has a pH of 4.8 to 6.2;
   more preferably, the pharmaceutical composition has a pH of 5.0 to 6.0.

3. The pharmaceutical composition according to claim 1 or 2, wherein the bispecific antibody specifically binding to HGFR and EGFR is at a concentration of 1 mg/mL to 250 mg/mL;

   preferably, the bispecific antibody specifically binding to HGFR and EGFR is at a concentration of 1 mg/mL to 200 mg/mL;

more preferably, the bispecific antibody specifically binding to HGFR and EGFR is at a concentration of 50 mg/mL to 200 mg/mL;

most preferably, the bispecific antibody specifically binding to HGFR and EGFR is at a concentration of 80 mg/mL to 180 mg/mL.

4. The pharmaceutical composition according to any one of claims 1 to 3, wherein the pharmaceutical composition comprises a surfactant;

preferably, the surfactant is a polysorbate or poloxamer;
more preferably, the surfactant is polysorbate 80.

5. The pharmaceutical composition according to claim 4, wherein the surfactant is at a concentration of 0.01 mg/mL to 1.0 mg/mL;

preferably, the surfactant is at a concentration of 0.1 mg/mL to 0.8 mg/mL;
more preferably, the surfactant is at a concentration of 0.4 mg/mL to 0.8 mg/mL.

6. The pharmaceutical composition according to any one of claims 1 to 5, wherein the pharmaceutical composition comprises a sugar;

preferably, the sugar is sucrose, trehalose, mannitol, or sorbitol;
more preferably, the sugar is sucrose.

7. The pharmaceutical composition according to claim 6, wherein the sugar is at a concentration of 10 mg/mL to 100 mg/mL;

preferably, the sugar is at a concentration of 20 mg/mL to 80 mg/mL;
more preferably, the sugar is at a concentration of 60 mg/mL to 80 mg/mL.

8. The pharmaceutical composition according to any one of claims 1 to 7, wherein the buffer is at a concentration of 5 mM to 100 mM;

preferably, the buffer is at a concentration of 5 mM to 50 mM;
more preferably, the buffer is at a concentration of 5 mM to 30 mM.

9. The pharmaceutical composition according to any one of claims 1 to 8, wherein the pharmaceutical composition further comprises an auxiliary material; preferably, the auxiliary material is methionine, arginine hydrochloride, glycine, proline, histidine, phenylalanine, glutamic acid, aspartic acid, sodium chloride, calcium chloride, or disodium edetate; more preferably, the auxiliary material is methionine or arginine hydrochloride; most preferably, the auxiliary material is methionine.

10. The pharmaceutical composition according to claim 9, wherein the auxiliary material is at a concentration of 1 mM to 300 mM; preferably, the auxiliary material is at a concentration of 1 mM to 200 mM; more preferably, the auxiliary material is at a concentration of 1 mM to 20 mM.

11. The pharmaceutical composition according to any one of claims 1 to 10, wherein the pharmaceutical composition further comprises an enzyme; preferably, the enzyme is hyaluronidase.

12. The pharmaceutical composition according to claim 11, wherein the enzyme is at a concentration of 10 U/mL to 3000 U/mL; preferably, the enzyme is at a concentration of 150 U/mL to 2400 U/mL; more preferably, the enzyme is at a concentration of 700 U/mL to 2400 U/mL; more preferably, the enzyme is at a concentration of 1600 U/mL to 2400 U/mL.

13. The pharmaceutical composition according to any one of claims 1 to 12, wherein in the bispecific antibody specifically binding to HGFR and EGFR,

(i) the bispecific antibody specifically binding to HGFR and EGFR comprises two antigen-binding moieties 1 specifically binding to HGFR and one antigen-binding moiety 2 specifically binding to EGFR, wherein

one of the antigen-binding moieties 1 comprises an M-VH and an M-VL, wherein the M-VH comprises: an M-HCDR1 set forth in SEQ ID NO: 30, an M-HCDR2 set forth in SEQ ID NO: 31, and an M-HCDR3 set forth in SEQ ID NO: 32, and the M-VL comprises: an M-LCDR1 set forth in SEQ ID NO: 27, an M-LCDR2 set forth in SEQ ID NO: 33, and an M-LCDR3 set forth in SEQ ID NO: 29;

the other antigen-binding moiety 1 comprises an M-VH and an M-VL, wherein the M-VH comprises: an M-HCDR1 set forth in SEQ ID NO: 18, an M-HCDR2 set forth in SEQ ID NO: 19, and an M-HCDR3 set forth in SEQ ID NO: 20, and the M-VL comprises: an M-LCDR1 set forth in SEQ ID NO: 21, an M-LCDR2 set forth in SEQ ID NO: 22, and an M-LCDR3 set forth in SEQ ID NO: 23; and

the antigen-binding moiety 2 comprises an E-VH and an E-VL, wherein the E-VH comprises: an E-HCDR1 set forth in SEQ ID NO: 6, an E-HCDR2 set forth in SEQ ID NO: 7, and an E-HCDR3 set forth in SEQ ID NO: 8, and the E-VL comprises: an E-LCDR1 set forth in SEQ ID NO: 9, an E-LCDR2 set forth in SEQ ID NO: 10, and an E-LCDR3 set forth in SEQ ID NO: 11; or

(ii) the bispecific antibody specifically binding to HGFR and EGFR comprises one antigen-binding moiety 1 specifically binding to HGFR and one antigen-binding moiety 2 specifically binding to EGFR, wherein

the antigen-binding moiety 1 comprises an M-VH and an M-VL, wherein the M-VH comprises: an M-HCDR1 set forth in SEQ ID NO: 18, an M-HCDR2 set forth in SEQ ID NO: 19, and an M-HCDR3 set forth in SEQ ID NO: 20, and the M-VL comprises: an M-LCDR1 set forth in SEQ ID NO: 21, an M-LCDR2 set forth in SEQ ID NO: 22, and an M-LCDR3 set forth in SEQ ID NO: 23; and

the antigen-binding moiety 2 comprises an E-VH and an E-VL, wherein the E-VH comprises: an E-HCDR1 set forth in SEQ ID NO: 6, an E-HCDR2 set forth in SEQ ID NO: 7, and an E-HCDR3 set forth in SEQ ID NO: 8, and the E-VL comprises: an E-LCDR1 set forth in SEQ ID NO: 9, an E-LCDR2 set forth in SEQ ID NO: 10, and an E-LCDR3 set forth in SEQ ID NO: 11.

14. The pharmaceutical composition according to claim 13, wherein in the bispecific antibody specifically binding to HGFR and EGFR:

(i) the M-VH comprises an amino acid sequence having at least 90% sequence identity to SEQ ID NO: 16, and the M-VL comprises an amino acid sequence having at least 90% sequence identity to SEQ ID NO: 17; or

the M-VH comprises an amino acid sequence having at least 90% sequence identity to SEQ ID NO: 12, and the M-VL comprises an amino acid sequence having at least 90% sequence identity to SEQ ID NO: 13; or
the M-VH comprises an amino acid sequence having at least 90% sequence identity to SEQ ID NO: 14, and the M-VL comprises an amino acid sequence having at least 90% sequence identity to SEQ ID NO: 15; or

(ii) the E-VH comprises an amino acid sequence having at least 90% sequence identity to SEQ ID NO: 3, and the E-VL comprises an amino acid sequence having at least 90% sequence identity to SEQ ID NO: 5 or SEQ ID NO: 4; preferably,

(i) the bispecific antibody specifically binding to HGFR and EGFR comprises two antigen-binding moieties 1 specifically binding to HGFR and one antigen-binding moiety 2 specifically binding to EGFR, wherein

one of the antigen-binding moieties 1 comprises an M-VH set forth in SEQ ID NO: 16 and an M-VL set forth in SEQ ID NO: 17;
the other antigen-binding moiety 1 comprises an M-VH set forth in SEQ ID NO: 12 and an M-VL set forth in SEQ ID NO: 13; and
the antigen-binding moiety 2 comprises an E-VH set forth in SEQ ID NO: 3 and an E-VL set forth in SEQ ID NO: 5; or

(ii) the bispecific antibody specifically binding to HGFR and EGFR comprises one antigen-binding moiety 1 specifically binding to HGFR and one antigen-binding moiety 2 specifically binding to EGFR, wherein the antigen-binding moiety 1 comprises an M-VH set forth in SEQ ID NO: 12 and an M-VL set forth in SEQ ID NO: 13, and the antigen-binding moiety 2 comprises an E-VH set forth in SEQ ID NO: 3 and an E-VL set forth in SEQ ID NO: 5.

15. The pharmaceutical composition according to claim 13 or 14, wherein the bispecific antibody specifically binding to HGFR and EGFR comprises one first chain set forth in SEQ ID NO: 34, one second chain set forth in SEQ ID NO: 35, one third chain set forth in SEQ ID NO: 36, and one fourth chain set forth in SEQ ID NO: 37; or
comprises one first chain set forth in SEQ ID NO: 38, one second chain set forth in SEQ ID NO: 39, one third chain set
forth in SEQ ID NO: 40, and one fourth chain set forth in SEQ ID NO: 41.

16. The pharmaceutical composition according to any one of claims 1 to 8 and 13 to 15, wherein the pharmaceutical
composition comprises the following components:

(a) 1 mg/mL to 250 mg/mL said bispecific antibody specifically binding to HGFR and EGFR,
(b) 0.01 mg/mL to 1.0 mg/mL surfactant,
(c) 10 mg/mL to 100 mg/mL sugar, and
(d) 5 mM to 100 mM buffer; the pharmaceutical composition has a pH of 4.5 to 6.5;

preferably, the pharmaceutical composition comprises the following components:

(a) 1 mg/mL to 200 mg/mL said bispecific antibody specifically binding to HGFR and EGFR,
(b) 0.1 mg/mL to 0.8 mg/mL polysorbate 80,
(c) 20 mg/mL to 80 mg/mL sucrose, and
(d) 5 mM to 50 mM histidine buffer; the pharmaceutical composition has a pH of 4.8 to 6.2;

more preferably, the pharmaceutical composition comprises the following components:

(a) 80 mg/mL to 180 mg/mL bispecific antibody specifically binding to HGFR and EGFR,
(b) 0.4 mg/mL to 0.8 mg/mL polysorbate 80,
(c) 60 mg/mL to 80 mg/mL sucrose, and
(d) 5 mM to 30 mM histidine-histidine hydrochloride buffer or histidine-histidine acetate buffer; the pharmaceutical
composition has a pH of 5.0 to 6.0.

17. The pharmaceutical composition according to any one of claims 1 to 15, wherein the pharmaceutical composition
comprises the following components:

(a) 1 mg/mL to 250 mg/mL said bispecific antibody specifically binding to HGFR and EGFR,
(b) 0.01 mg/mL to 1.0 mg/mL surfactant,
(c) 10 mg/mL to 100 mg/mL sugar,
(d) 1 mM to 300 mM auxiliary material,
(e) 10 U/mL to 3000 U/mL enzyme, and
(f) 5 mM to 100 mM buffer; the pharmaceutical composition has a pH of 4.5 to 6.5;

preferably, the pharmaceutical composition comprises the following components:

(a) 1 mg/mL to 200 mg/mL said bispecific antibody specifically binding to HGFR and EGFR,
(b) 0.1 mg/mL to 0.8 mg/mL polysorbate 80,
(c) 20 mg/mL to 80 mg/mL sucrose,
(d) 1 mM to 200 mM methionine,
(e) 700 U/mL to 2400 U/mL hyaluronidase, and
(f) 5 mM to 50 mM histidine buffer; the pharmaceutical composition has a pH of 4.8 to 6.2;

more preferably, the pharmaceutical composition comprises the following components:

(a) 80 mg/mL to 180 mg/mL bispecific antibody specifically binding to HGFR and EGFR,
(b) 0.4 mg/mL to 0.8 mg/mL polysorbate 80,
(c) 60 mg/mL to 80 mg/mL sucrose,
(d) 1 mM to 20 mM methionine,
(e) 1600 U/mL to 2400 U/mL hyaluronidase, and
(f) 5 mM to 30 mM histidine-histidine hydrochloride buffer or histidine-histidine acetate buffer; the pharmaceutical
composition has a pH of 5.0 to 6.0.

18. A freeze-dried formulation, wherein the freeze-dried formulation is capable of being reconstituted to form the
pharmaceutical composition according to any one of claims 1 to 17.

19. A method for preparing a freeze-dried formulation, comprising a step of lyophilizing the pharmaceutical composition according to any one of claims 1 to 17.

20. A freeze-dried formulation, wherein the formulation is obtained by the method according to claim 19.

21. A reconstituted solution, wherein the reconstituted solution is prepared by reconstituting the freeze-dried formulation according to claim 18 or 20.

22. The pharmaceutical composition according to any one of claims 1 to 17 or the reconstituted solution according to claim 21, being a formulation for intravenous, subcutaneous, intraperitoneal, or intramuscular injection, preferably a formulation for intravenous or subcutaneous injection.

23. A method for treating or preventing a disease, comprising administering to a subject a therapeutically effective amount of the pharmaceutical composition according to any one of claims 1 to 17, or the freeze-dried formulation according to claim 18 or 20, or the reconstituted solution according to claim 21, wherein
preferably, the disease is a tumor; more preferably, the tumor is selected from the group consisting of lung cancer, breast cancer, pancreatic cancer, colorectal cancer, sarcoma, renal cell carcinoma, hepatocellular carcinoma, gastric cancer, ovarian cancer, bladder cancer, head and neck cancer, and glioblastoma.

**FIG. 1**

**FIG. 2**

**FIG. 3**

**FIG. 4**

**FIG. 5**

**FIG. 6**

**FIG. 7**

**FIG. 8**

**FIG. 9**

**FIG. 10**

**FIG. 11**

**FIG. 12**

**FIG. 13**

**FIG. 14**

## INTERNATIONAL SEARCH REPORT

| | |
|---|---|
| International application No. | |
| | **PCT/CN2023/133895** |

| | |
|---|---|
| **A.   CLASSIFICATION OF SUBJECT MATTER** | |
| C07K 16/28(2006.01)i;   A61K 39/395(2006.01)i;   C12N 15/13(2006.01)i | |
| According to International Patent Classification (IPC) or to both national classification and IPC | |

| **B.   FIELDS SEARCHED** |
|---|
| Minimum documentation searched (classification system followed by classification symbols) |
| IPC: C07K, A61K, C12N |
| Documentation searched other than minimum documentation to the extent that such documents are included in the fields searched |
| Electronic data base consulted during the international search (name of data base and, where practicable, search terms used) |
| CNABS, SIPOABS, DWPI, CNTXT, WOTXT, USTXT, EPTXT, JPTXT, KRTXT, CNKI, Web of Science, Elsevier Science, STN, GenBank, EMBL, 中国专利生物序列检索系统, China Patent Biological Sequence Search System, 必应搜索, Bing Search, 超星读秀, CHAOXING DUXIU: 表皮生长因子受体, Epidermal growth factor receptor, EGFR, ErbB-1, HER-1, 肝细胞生长因子受体, Hepatocyte growth factor receptor, HGFR, c-Met, MET, 双特异性抗体, bispecific antibody, 扎芦木, Zalutumumab, Zal, Zal.1, 2F8, 奥纳珠, Onartuzumab, Omab, 5D5, Ab10, Ab-10, 对SEQ ID NOs: 3-5, 12-17进行序列检索, search for SEQ ID NOs: 3-5, 12-17 |

| **C.   DOCUMENTS CONSIDERED TO BE RELEVANT** | | |
|---|---|---|
| Category* | Citation of document, with indication, where appropriate, of the relevant passages | Relevant to claim No. |
| PX | WO 2023284829 A1 (JIANGSU HENGRUI PHARMACEUTICALS CO., LTD. et al.) 19 January 2023 (2023-01-19) see embodiment 3, and claims 1-5 | 1-23 |
| X | CN 104955838 A (JANSSEN BIOTECH, INC.) 30 September 2015 (2015-09-30) see description, paragraphs 8, 396-399 and 518 | 1-23 |
| Y | CN 106188293 A (JIANGSU HENGRUI MEDICINE CO., LTD. et al.) 07 December 2016 (2016-12-07) see claims 1-16, and embodiment 6 | 1-23 |
| Y | CN 110028584 A (BEIJING KEXIN BIOTECHNOLOGY CO., LTD.) 19 July 2019 (2019-07-19) see SEQ ID NOs: 7-8 | 1-23 |

| | | |
|---|---|---|
| ☑ Further documents are listed in the continuation of Box C. | ☑ See patent family annex. | |

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **02 February 2024** | **15 February 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| | International application No. |
|---|---|
| | **PCT/CN2023/133895** |

**C. DOCUMENTS CONSIDERED TO BE RELEVANT**

| Category* | Citation of document, with indication, where appropriate, of the relevant passages | Relevant to claim No. |
|---|---|---|
| Y | CN 102361883 A (ROCHE GLYCART AG) 22 February 2012 (2012-02-22)<br>see SEQ ID NOs: 5-10 | 1-23 |
| Y | CN 112513074 A (GENMAB AS) 16 March 2021 (2021-03-16)<br>see SEQ ID NOs: 39-41 | 1-23 |

Form PCT/ISA/210 (second sheet) (July 2022)

## INTERNATIONAL SEARCH REPORT

International application No.

**PCT/CN2023/133895**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
|---|
| **PCT/CN2023/133895** |

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **23**
   because they relate to subject matter not required to be searched by this Authority, namely:

   The subject matter of claim 23 is a method for treating a human or animal body, which belongs to a subject matter as defined in PCT Rule 39.1(iv) for which no search is required. The subject matter of claim 23 can be reasonably expected to be amended to: the use of the pharmaceutical composition of any one of claims 1 to 17, the freeze-dried preparation of claim 18 or 20, or the reconstitution solution of claim 21 in the preparation of a drug for treating or preventing tumors. The international search is conducted on the basis of this reasonable expectation.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
| --- |
| **PCT/CN2023/133895** |

| Patent document cited in search report | | | Publication date (day/month/year) | Patent family member(s) | | | Publication date (day/month/year) |
| --- | --- | --- | --- | --- | --- | --- | --- |
| WO | 2023284829 | A1 | 19 January 2023 | WO | 2023284829 | A8 | 21 December 2023 |
| | | | | TW | 202317635 | A | 01 May 2023 |
| CN | 104955838 | A | 30 September 2015 | HUS | 2200016 | I1 | 28 May 2022 |
| | | | | HUE | 041499 | T2 | 28 May 2019 |
| | | | | US | 2014255408 | A1 | 11 September 2014 |
| | | | | US | 9580508 | B2 | 28 February 2017 |
| | | | | SG | 11201503938 | VA | 29 June 2015 |
| | | | | US | 2014141000 | A1 | 22 May 2014 |
| | | | | US | 9593164 | B2 | 14 March 2017 |
| | | | | UA | 117121 | C2 | 25 June 2018 |
| | | | | CA | 2893505 | A1 | 30 May 2014 |
| | | | | CA | 2893505 | C | 21 February 2023 |
| | | | | PT | 3447069 | T | 13 November 2020 |
| | | | | ES | 2831374 | T3 | 08 June 2021 |
| | | | | LT | 2922872 | T | 27 December 2018 |
| | | | | CY | 1121270 | T1 | 29 May 2020 |
| | | | | JP | 2021006561 | A | 21 January 2021 |
| | | | | JP | 7019771 | B2 | 15 February 2022 |
| | | | | EP | 3808767 | A1 | 21 April 2021 |
| | | | | EP | 3808767 | B1 | 06 December 2023 |
| | | | | IL | 238796 | A0 | 30 June 2015 |
| | | | | IL | 238796 | B | 30 June 2019 |
| | | | | FIC | 20220016 | I1 | 31 May 2022 |
| | | | | EA | 201590985 | A1 | 30 November 2015 |
| | | | | EA | 031184 | B1 | 30 November 2018 |
| | | | | HRP | 20182128 | T1 | 08 February 2019 |
| | | | | CA | 3182876 | A1 | 30 May 2014 |
| | | | | CL | 2015001356 | A1 | 06 November 2015 |
| | | | | DK | 3447069 | T3 | 16 November 2020 |
| | | | | SI | 2922872 | T1 | 31 January 2019 |
| | | | | WO | 2014081954 | A1 | 30 May 2014 |
| | | | | JP | 2022062155 | A | 19 April 2022 |
| | | | | JP | 7397105 | B2 | 12 December 2023 |
| | | | | AU | 2021202394 | A1 | 20 May 2021 |
| | | | | JP | 2016505537 | A | 25 February 2016 |
| | | | | JP | 6423357 | B2 | 14 November 2018 |
| | | | | US | 2017101475 | A1 | 13 April 2017 |
| | | | | US | 9695242 | B2 | 04 July 2017 |
| | | | | MX | 2015006387 | A | 03 December 2015 |
| | | | | MX | 361088 | B | 26 November 2018 |
| | | | | NL | 301173 | I1 | 04 May 2022 |
| | | | | NL | 301173 | I2 | 15 June 2022 |
| | | | | DK | 2922872 | T3 | 02 January 2019 |
| | | | | PL | 2922872 | T3 | 29 March 2019 |
| | | | | NI | 201500069 | A | 19 October 2015 |
| | | | | PH | 12015501118 | A1 | 01 February 2016 |
| | | | | KR | 20150087365 | A | 29 July 2015 |
| | | | | KR | 102373193 | B1 | 10 March 2022 |
| | | | | NZ | 708352 | A | 25 October 2019 |
| | | | | PE | 20151181 | A1 | 19 August 2015 |

Form PCT/ISA/210 (patent family annex) (July 2022)

**INTERNATIONAL SEARCH REPORT**
Information on patent family members

International application No.

**PCT/CN2023/133895**

| Patent document cited in search report | | | Publication date (day/month/year) | Patent family member(s) | | | Publication date (day/month/year) |
|---|---|---|---|---|---|---|---|
| | | | | SI | 3447069 | T1 | 26 February 2021 |
| | | | | AU | 2019200441 | A1 | 07 February 2019 |
| | | | | AU | 2019200441 | B2 | 28 January 2021 |
| | | | | KR | 20220032654 | A | 15 March 2022 |
| | | | | CY | 1123550 | T1 | 24 March 2022 |
| | | | | EP | 3447069 | A1 | 27 February 2019 |
| | | | | EP | 3447069 | B1 | 23 September 2020 |
| | | | | JP | 2019048817 | A | 28 March 2019 |
| | | | | JP | 6773746 | B2 | 21 October 2020 |
| | | | | BR | 112015011717 | A2 | 15 August 2017 |
| | | | | BR | 112015011717 | B1 | 12 December 2023 |
| | | | | RS | 58192 | B1 | 29 March 2019 |
| | | | | AU | 2013347962 | A1 | 28 May 2015 |
| | | | | AU | 2013347962 | B2 | 25 October 2018 |
| | | | | EP | 2922872 | A1 | 30 September 2015 |
| | | | | EP | 2922872 | A4 | 06 July 2016 |
| | | | | EP | 2922872 | B1 | 10 October 2018 |
| | | | | HRP | 20201848 | T1 | 08 January 2021 |
| | | | | FR | 2210181I1 | | 17 June 2022 |
| | | | | FR | 2210181I2 | | 05 May 2023 |
| | | | | RS | 61057 | B1 | 31 December 2020 |
| | | | | LTPA | 2022507 | I1 | 27 June 2022 |
| | | | | ES | 2700231 | T3 | 14 February 2019 |
| | | | | LT | 3447069 | T | 10 December 2020 |
| | | | | PT | 2922872 | T | 18 January 2019 |
| | | | | HUE | 052548 | T2 | 28 May 2021 |
| CN | 106188293 | A | 07 December 2016 | BR | 112017021245 | A2 | 26 June 2018 |
| | | | | JP | 2018516539 | A | 28 June 2018 |
| | | | | CA | 2982777 | A1 | 20 October 2016 |
| | | | | KR | 20170138451 | A | 15 December 2017 |
| | | | | MX | 2017012965 | A | 06 June 2018 |
| | | | | RU | 2017135257 | A | 17 May 2019 |
| | | | | RU | 2017135257 | A3 | 24 September 2019 |
| | | | | US | 2018110875 | A1 | 26 April 2018 |
| | | | | US | 10543284 | B2 | 28 January 2020 |
| | | | | EP | 3284751 | A1 | 21 February 2018 |
| | | | | EP | 3284751 | A4 | 05 December 2018 |
| | | | | TW | 201638108 | A | 01 November 2016 |
| | | | | TWI | 731856 | B | 01 July 2021 |
| | | | | AU | 2016248357 | A1 | 26 October 2017 |
| CN | 110028584 | A | 19 July 2019 | None | | | |
| CN | 102361883 | A | 22 February 2012 | KR | 20110126748 | A | 23 November 2011 |
| | | | | KR | 20110124368 | A | 16 November 2011 |
| | | | | IL | 214847 | A0 | 30 November 2011 |
| | | | | US | 2013156772 | A1 | 20 June 2013 |
| | | | | WO | 2010115553 | A1 | 14 October 2010 |
| | | | | US | 2013273054 | A1 | 17 October 2013 |
| | | | | SG | 175080 | A1 | 28 November 2011 |
| | | | | AR | 076194 | A1 | 26 May 2011 |
| | | | | JP | 2012522525 | A | 27 September 2012 |

Form PCT/ISA/210 (patent family annex) (July 2022)

**INTERNATIONAL SEARCH REPORT**
Information on patent family members

International application No.

**PCT/CN2023/133895**

| Patent document cited in search report | | | Publication date (day/month/year) | Patent family member(s) | | | Publication date (day/month/year) |
|---|---|---|---|---|---|---|---|
| | | | | JP | 5497887 | B2 | 21 May 2014 |
| | | | | WO | 2010115551 | A1 | 14 October 2010 |
| | | | | JP | 2012522523 | A | 27 September 2012 |
| | | | | JP | 5612663 | B2 | 22 October 2014 |
| | | | | BRPI | 1014474 | A2 | 27 June 2017 |
| | | | | EP | 2417160 | A1 | 15 February 2012 |
| | | | | BRPI | 1014449 | A2 | 27 June 2017 |
| | | | | US | 2010254988 | A1 | 07 October 2010 |
| | | | | TW | 201039849 | A | 16 November 2010 |
| | | | | US | 2010254989 | A1 | 07 October 2010 |
| | | | | TW | 201039848 | A | 16 November 2010 |
| | | | | AU | 2010233993 | A1 | 08 September 2011 |
| | | | | IL | 214885 | A0 | 30 November 2011 |
| | | | | MX | 2011010169 | A | 11 October 2011 |
| | | | | AU | 2010233995 | A1 | 08 September 2011 |
| | | | | CA | 2757426 | A1 | 14 October 2010 |
| | | | | AR | 076195 | A1 | 26 May 2011 |
| | | | | EP | 2417164 | A1 | 15 February 2012 |
| | | | | SG | 175078 | A1 | 28 November 2011 |
| | | | | MX | 2011010158 | A | 17 October 2011 |
| | | | | CA | 2757669 | A1 | 14 October 2010 |
| CN | 112513074 | A | 16 March 2021 | US | 2021269509 | A1 | 02 September 2021 |
| | | | | WO | 2019243626 | A1 | 26 December 2019 |
| | | | | IL | 279528 | A | 31 January 2021 |
| | | | | JP | 2021528435 | A | 21 October 2021 |
| | | | | CA | 3104390 | A1 | 26 December 2019 |
| | | | | SG | 11202012713 | RA | 28 January 2021 |
| | | | | EP | 3810649 | A1 | 28 April 2021 |
| | | | | MA | 52945 | A | 28 April 2021 |

Form PCT/ISA/210 (patent family annex) (July 2022)

**REFERENCES CITED IN THE DESCRIPTION**

**Patent documents cited in the description**

- CN 2022105714 W
- CN 202110794137
- WO 2013003680 A1
- WO 2016165580 A1
- WO 2019031965 A1

**Non-patent literature cited in the description**

- *J. Biol. Chem.*, 1968, vol. 243, 3558
- **ARAN F. LABRIJN et al.** *Nature Reviews Drug Discovery*, 2019, vol. 18, 585-608
- **CHEN S1 et al.** *J Immunol Res*, 11 February 2019, vol. 2019, 4516041
- **LEFRANC, M.P et al.** *Dev. Comp. Immunol*, 2003, vol. 27, 55-77
- *Front Immunol.*, 16 October 2018, vol. 9, 2278
- *WHO Drug Information*, vol. 33 (2), 237-239